# EUROPEAN PATENT APPLICATION

(11) **EP 4 831 171 A1**
(43) Date of publication of application: **28.10.2026**
(21) Application number: 24906450.2
(22) Date of filing: 19.12.2024
(51) Int. Cl.: C07D 471/04, A61K 31/519, A61P 35/00

(54) **FUSED RING COMPOUND, PHARMACEUTICAL COMPOSITION COMPRISING SAME, AND USE THEREOF**

(30) Priority: 21.12.2023 CN 202311774491
(71) Applicant: Suzhou Genhouse Bio Co., Ltd., Suzhou,Jiangsu 215123 (CN)
(72) Inventor: ZHENG, Jiyue, Suzhou, Jiangsu 215123 (CN); ZHANG, Guiping, Suzhou, Jiangsu 215123 (CN); LI, Jiapeng, Suzhou, Jiangsu 215123 (CN); WANG, Kuifeng, Suzhou, Jiangsu 215123 (CN); TONG, Shuilong, Suzhou, Jiangsu 215123 (CN); LIU, Tao, Suzhou, Jiangsu 215123 (CN); JI, Feihong, Suzhou, Jiangsu 215123 (CN)
(74) Representative: Turner, Craig Robert
(86) International application number: PCT/CN2024/140567
(87) International publication number: WO 2025/130971

(57) **Abstract**

The present invention relates to a compound of formula (I), a pharmaceutical composition comprising same, and a use of the compound in the prevention or treatment of diseases.

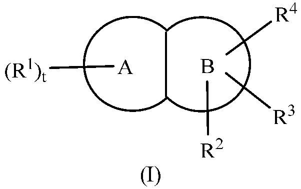

## Description

### TECHNICAL FIELD

The present disclosure relates to a fused ring compound, a pharmaceutical composition comprising same, and use thereof in preventing or treating a disease.

### BACKGROUND

Microsatellite instability (MSI) is a type of genomic damage caused by mismatch repair deficiency (dMMR). Although progress has been made in the treatment of microsatellite instability-high (MSI-H) cancers, tumor evolution and drug resistance remain major causes of treatment failure and death in cancer patients. For example, among dMMR colorectal cancer patients receiving PD-1 and PD-L1 checkpoint inhibitor therapies, about half of the patients experience primary resistance. See, e.g., Overman MJ et al., J Clin Oncol. 2018 Mar 10;36(8):773-779. There remains an unmet clinical need for new treatment options for patients who are refractory to currently available therapies.

Werner helicase (WRN) is considered to be a synthetic lethal target for MSI-H cancers (see, e.g., Chan EM et al., Nature. 2019 Apr;568(7753):551-556). WRN is a member of the RecQ family of DNA helicases and plays an important role in maintaining genomic stability, DNA repair, replication, transcription, and telomere maintenance. Studies on WRN-dependent mechanisms have found that dinucleotide TA repeat sequences undergo large-scale expansion in MSI cells, and these expanded TA repeat sequences form secondary DNA structures that require unwinding by WRN helicase (see, e.g., van Wietmarschen N et al., Nature. 2020 Oct;586(7828):292-298). In the absence of WRN (or under conditions in which WRN helicase is inhibited), the expanded TA repeat sequences in MSI cells are cleaved by nucleases, ultimately leading to chromosomal breakage. Therefore, inhibition of WRN helicase is an effective strategy for treating cancers characterized by microsatellite instability-high (MSI-H) or mismatch repair deficiency (dMMR), including colorectal cancer, gastric cancer, or endometrial cancer.

### BRIEF SUMMARY

The present application provides a compound as a WRN inhibitor, which can be used for preventing or treating a cancer characterized by microsatellite instability-high (MSI-H) or mismatch repair deficiency (dMMR). In addition, the compound of the present disclosure also has good physicochemical properties (e.g., solubility and physical and/or chemical stability), good pharmacokinetic properties (e.g., improved bioavailability, good metabolic stability, and suitable half-life and duration of action), good safety (less toxicity (e.g., reduced cardiotoxicity), and/or fewer side effects), lower propensity to induce drug resistance, and other excellent properties.

One aspect of the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound has a structure of formula (I): 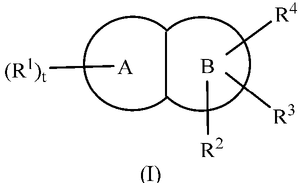 wherein: 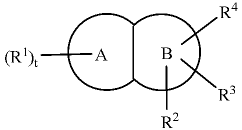 is selected from 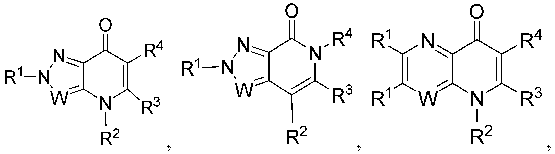 and 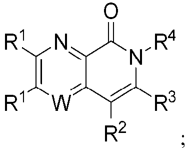
W is C-R¹ or N;
R¹, at each occurrence, is each independently selected from H, halogen, -OH, -NH₂, -CN, -NO₂, -SF₅, C₁₋₆ alkyl, deuterated C₁₋₆ alkyl, C₁₋₆ haloalkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, - C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a_}C(=O)R^{b}, -NR^{a}-C(=O)OR^{b}, -NR^{a}-S(=O)₂-R^{b}, -NR^{a}-C(=O)-NR^{a}R^{b}, -P(=O)R^{a}R^{b}, -C₁₋₆ alkylene-R^{a}, -C₁₋₆ alkylene-OR^{a}, -C₁₋₆ alkylene-NR^{a}R^{b}, -O-C₁₋₆ alkylene-NR^{a}R^{b}, (-C₃₋₆ cyclohydrocarbylene)-CN, and (-C₃₋₆ cyclohydrocarbylene)-C₁₋₆ haloalkyl;
or, two R¹ at adjacent positions, together with the group to which they are attached, optionally form a C₃₋₆ hydrocarbon ring, a 3- to 10-membered heterocyclic ring, a C₆₋₁₀ aromatic ring, or a 5- to 14-membered heteroaromatic ring;
R² is 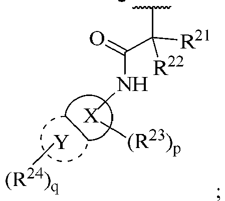
R³, R²¹, R²², R²³, and R²⁴, at each occurrence, are each independently selected from H, halogen, -OH, -NH₂, -CN, -NO₂, -SF₅, C₁₋₆ alkyl, deuterated C₁₋₆ alkyl, C₁₋₆ haloalkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, - C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -S(=O)(-NR^{a})R^{b}, - NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -P(=O)R^{a}R^{b}, -C₁₋₆ alkylene-R^{a}, -C₁₋₆ alkylene-OR^{a}, -C₁₋₆ alkylene-NR^{a}R^{b}, -O-C₁₋₆ alkylene-NR^{a}R^{b}, (-C₃₋₆ cyclohydrocarbylene)-CN, and (-C₃₋₆ cyclohydrocarbylene)-C₁₋₆ haloalkyl;
or, R³ and R²¹ or R²², together with the groups to which they are attached, optionally form a C₃₋₆ hydrocarbon ring, a 3- to 10-membered heterocyclic ring, a C₆₋₁₀ aromatic ring, or a 5- to 14-membered heteroaromatic ring;
R⁴ is 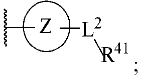
R⁴¹ is selected from a C₃₋₆ hydrocarbon ring, a 3- to 10-membered heterocyclic ring, a C₆₋₁₀ aromatic ring, and a 5- to 14-membered heteroaromatic ring;
ring X and ring Z are each independently selected from a C₃₋₆ hydrocarbon ring, a 3- to 10-membered heterocyclic ring, a C₆₋₁₀ aromatic ring, and a 5- to 14-membered heteroaromatic ring;
ring Y is absent or selected from a C₃₋₆ hydrocarbon ring, a 3- to 10-membered heterocyclic ring, a C₆₋₁₀ aromatic ring, and a 5- to 14-membered heteroaromatic ring; when ring Y is absent, R²⁴ is also absent;
L² is selected from -O-, -C(=O)-, -NRC(=O)-, -S-, -S(=O)-, -S(=O)₂-, C₁₋₆ alkylene, and -O-(C₁₋₆ alkylene)-; R, R^{a}, and R^{b}, at each occurrence, are each independently selected from H, C₁₋₆ alkyl, C₃₋₁₀ cyclohydrocarbyl, 3- to 10-membered heterocyclyl, C₆₋₁₀ aryl, 5- to 14-membered heteroaryl, and C₆₋₁₂ aralkyl;
the alkylene, the alkyl, the alkenyl, the alkynyl, the cyclohydrocarbylene, the cyclohydrocarbyl, the hydrocarbon ring, the heterocyclyl, the heterocyclic ring, the aryl, the aromatic ring, the heteroaryl, the heteroaromatic ring, and the aralkyl described above, at each occurrence, are each optionally substituted with one or more substituents independently selected from the following: a deuterium atom, halogen, -OH, =O, - NH₂, -CN, -NO₂, =CH₂, C₁₋₆ alkyl, deuterated C₁₋₆ alkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, -C(=O)R^{c}, - OC(=O)R^{c}, -C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{c}, -C₁₋₆ alkylene-NR^{c}R^{d}, and -O-C₁₋₆ alkylene-NR^{c}R^{d}, and when the same ring atom or adjacent ring atoms of the cyclohydrocarbylene, the cyclohydrocarbyl, the hydrocarbon ring, the heterocyclyl, the heterocyclic ring, the aryl, the aromatic ring, the heteroaryl, and the heteroaromatic ring are substituted with two substituents, the two substituents, together with groups to which they are attached, optionally form a C₃₋₆ hydrocarbon ring, a 3- to 10-membered heterocyclic ring, a C₆₋₁₀ aromatic ring, or a 5- to 14-membered heteroaromatic ring; the alkylene, the alkyl, the alkenyl, =CH₂, the alkynyl, the cyclohydrocarbyl, the hydrocarbon ring, the heterocyclyl, the heterocyclic ring, the aryl, the aromatic ring, the heteroaryl, the heteroaromatic ring, and the aralkyl are each further optionally substituted with one or more substituents independently selected from the following: halogen, -OH, =O, -C(=O)O-tert-butyl, -NH₂, -CN, -NO₂, C₁₋₆ alkyl, C₁₋₆ haloalkyl, C₃₋₆ cyclohydrocarbyl, 3- to 10-membered heterocyclyl, C₆₋₁₀ aryl, 5- to 14-membered heteroaryl, C₆₋₁₂ aralkyl, -C₁₋₆ alkylene-C₃₋₆ cyclohydrocarbyl, -O-C₁₋₆ alkyl, and -C₁₋₆ alkylene-O-C₁₋₆ alkyl;
R^{c} and R^{d}, at each occurrence, are each independently selected from: H, C₁₋₆ alkyl, C₃₋₁₀ cyclohydrocarbyl, 3- to 10-membered heterocyclyl, C₆₋₁₀ aryl, 5- to 14-membered heteroaryl, and C₆₋₁₂ aralkyl, wherein the alkyl, the cyclohydrocarbyl, the heterocyclyl, the aryl, the heteroaryl, and the aralkyl are further optionally substituted with one or more substituents independently selected from the following: halogen, -OH, =O, - C(=O)O-tert-butyl, -NH₂, -CN, -NO₂, C₁₋₆ alkyl, C₁₋₆ haloalkyl, C₃₋₆ cyclohydrocarbyl, 3- to 10-membered heterocyclyl, C₆₋₁₀ aryl, 5- to 14-membered heteroaryl, C₆₋₁₂ aralkyl, and -C₁₋₆ alkylene-O-C₁₋₆ alkyl; and
p, q, and t are each independently an integer selected from 1, 2, or 3.

Another aspect of the present disclosure provides a pharmaceutical composition comprising a prophylactically or therapeutically effective amount of the compound, or the pharmaceutically acceptable salt, the ester, the stereoisomer, the tautomer, the polymorph, the solvate, the metabolite, the isotopically labeled compound, or the prodrug thereof of the present disclosure, and one or more pharmaceutically acceptable carriers.

Another aspect of the present disclosure provides use of the compound, or the pharmaceutically acceptable salt, the ester, the stereoisomer, the tautomer, the polymorph, the solvate, the metabolite, the isotopically labeled compound, or the prodrug thereof of the present disclosure, or of the pharmaceutical composition of the present disclosure in preparing a medicament as a WRN inhibitor.

Another aspect of the present disclosure provides the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure, for use as a WRN inhibitor.

Another aspect of the present disclosure provides a method for preventing or treating a cancer (preferably a cancer characterized by microsatellite instability-high (MSI-H) or mismatch repair deficiency (dMMR)), comprising administering to a subject in need thereof an effective amount of the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure.

### DETAILED DESCRIPTION OF THE INVENTION

### Definitions

Unless otherwise defined below, all technical and scientific terms used herein are intended to have the same meaning as commonly understood by those skilled in the art. Reference to the techniques used herein is intended to refer to techniques commonly understood in the art, including those variations of or equivalent alternatives to the techniques that are apparent to those skilled in the art. Although the following terms are believed to be well understood by those skilled in the art, the following definitions are set forth to better explain the present disclosure.

The terms "include", "comprise", "have", "contain", or "involve", and other variant forms thereof herein, are inclusive or open-ended and do not exclude other unrecited elements or method steps.

As used herein, the term "alkylene" refers to saturated divalent hydrocarbyl, preferably saturated divalent hydrocarbyl having 1, 2, 3, 4, 5, or 6 carbon atoms, e.g., methylene, ethylene, propylene, or butylene.

As used herein, the term "alkyl" is defined as a linear or branched saturated aliphatic hydrocarbon. In some embodiments, the alkyl has 1 to 12, e.g., 1 to 6, carbon atoms. For example, as used herein, the term "C₁₋₆ alkyl" refers to a linear or branched group of 1 to 6 carbon atoms (e.g., methyl, ethyl, n-propyl, isopropyl, n-butyl, isobutyl, sec-butyl, tert-butyl, n-pentyl, or n-hexyl) that is optionally substituted with 1 or more (such as 1 to 3) suitable substituents such as halogen (in this case the group is referred to as "haloalkyl") (e.g., CF₃, C₂F₅, CHF₂, CH₂F, CH₂CF₃, CH₂Cl, or -CH₂CH₂CF₃). The term "C₁₋₄ alkyl" refers to a linear or branched aliphatic hydrocarbon chain of 1 to 4 carbon atoms (i.e., methyl, ethyl, n-propyl, isopropyl, n-butyl, isobutyl, sec-butyl, or tert-butyl).

As used herein, the term "alkenyl" refers to linear or branched monovalent hydrocarbyl containing one or more double bonds and having 2 to 6 carbon atoms ("C₂₋₆ alkenyl"). The alkenyl is, for example, -CH=CH₂, -CH₂CH=CH₂, -C(CH₃)=CH₂, -CH₂-CH=CH-CH₃, 2-pentenyl, 3-pentenyl, 4-pentenyl, 2-hexenyl, 3-hexenyl, 4-hexenyl, 5-hexenyl, 2-methyl-2-propenyl, or 4-methyl-3-pentenyl. When the compound of the present disclosure contains the alkenyl, the compound may be present in a pure E (entgegen) form, a pure Z (zusammen) form, or any mixture thereof. The term "alkenylene" is a corresponding divalent group, including, for example, "C₂₋₆ alkenylene", "C₂₋₄ alkenylene", and the like, specific examples of which include, but are not limited to: -CH=CH-, -CH₂CH=CH-, -C(CH₃)=CH-, butenylene, pentenylene, hexenylene, and the like.

As used herein, the term "alkynyl" refers to monovalent hydrocarbyl containing one or more triple bonds and preferably having 2, 3, 4, 5, or 6 carbon atoms, e.g., ethynyl, 2-propynyl, 2-butynyl, or 1,3-butadiynyl. The alkynyl is optionally substituted with one or more (such as 1 to 3) identical or different substituents. The term "alkynylene" is a corresponding divalent group, including, for example, "C₂₋₈ alkynylene", "C₂₋₆ alkynylene", "C₂₋₄ alkynylene", and the like, examples of which include, but are not limited to, 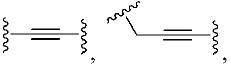 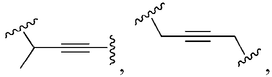 and the like. The alkynylene is optionally substituted with one or more (such as 1 to 3) identical or different substituents.

As used herein, the term "fused ring" refers to a ring system formed by two or more cyclic structures that share two adjacent atoms.

As used herein, the term "spiro ring" refers to a ring system formed by two or more cyclic structures that share one ring atom.

As used herein, the term "bridged ring" refers to a ring system formed by two or more cyclic structures that share two atoms not directly connected.

As used herein, the terms "cyclohydrocarbylene", "cyclohydrocarbyl", and "hydrocarbon ring" refer to a saturated (i.e., "cycloalkylene" and "cycloalkyl") or partially unsaturated (i.e., having one or more double and/or triple bonds within the ring) monocyclic or polycyclic hydrocarbon ring (including a spiro ring, fused ring, or bridged ring system) having, for example, 3 to 10 (suitably 3 to 8, and more suitably 3 to 6) ring carbon atoms, including, but not limited to, cyclopropyl(ene) (cyclopropane ring), cyclobutyl(ene) (cyclobutane ring), cyclopentyl(ene) (cyclopentane ring), cyclohexyl(ene) (cyclohexane ring), cycloheptyl(ene) (cycloheptane ring), cyclooctyl(ene) (cyclooctane ring), cyclononyl(ene) (cyclononane ring), cyclohexenyl(ene) (cyclohexene ring), and the like.

As used herein, the term "cycloalkyl" refers to a saturated monocyclic or polycyclic (such as bicyclic) hydrocarbon ring (e.g., a monocyclic ring, such as cyclopropyl, cyclobutyl, cyclopentyl, cyclohexyl, cycloheptyl, cyclooctyl, or cyclononyl, or a bicyclic ring, including a spiro ring, fused ring, or bridged ring system (such as bicyclo[1.1.1]pentyl, bicyclo[2.2.1]heptyl, bicyclo[3.2.1]octyl, bicyclo[5.2.0]nonyl, or decahydronaphthyl)) optionally substituted with 1 or more (such as 1 to 3) suitable substituents. The cycloalkyl has 3 to 15 carbon atoms. For example, the term "C₃₋₆ cycloalkyl" refers to a saturated monocyclic or polycyclic (such as bicyclic) hydrocarbon ring of 3 to 6 ring-forming carbon atoms (e.g., cyclopropyl, cyclobutyl, cyclopentyl, or cyclohexyl) optionally substituted with 1 or more (such as 1 to 3) suitable substituents, for example, cyclopropyl substituted with methyl.

As used herein, the term "heterocyclyl" (or "heterocyclic ring") refers to a saturated or partially unsaturated monocyclic or bicyclic group having 2, 3, 4, 5, 6, 7, 8, 9, or 10 carbon atoms and one or more (e.g., one, two, three, or four) heteroatoms selected from O, S, N, and P in the ring, and the "heterocyclyl" (or "heterocyclic ring") may contain -C(=O)- as a ring member. The heterocyclyl may be connected to the rest of the molecule by the carbon atom and/or the heteroatom (if present). In particular, 3- to 10-membered heterocyclyl is a group having 3 to 10 carbon atoms and heteroatoms in the ring, for example, but not limited to, oxiranyl, aziridinyl, azetidinyl, oxetanyl, tetrahydrofuranyl, dioxolinyl, pyrrolidinyl, pyrrolidinonyl, imidazolidinyl, pyrazolidinyl, pyrrolinyl, tetrahydropyranyl, piperidinyl, morpholinyl, dithianyl, thiomorpholinyl, piperazinyl, or trithianyl.

As used herein, the term "heterocyclyl" (or "heterocyclic ring") encompasses fused ring structures, the point of attachment of which to other groups may be on any ring of the fused ring structures. Thus, the heterocyclyl of the present disclosure further includes, but is not limited to, heterocyclyl fused with heterocyclyl, heterocyclyl fused with cycloalkyl, monoheterocyclyl fused with monoheterocyclyl, monoheterocyclyl fused with monocycloalkyl, aryl fused with heterocyclyl, and heteroaryl fused with heterocyclyl, e.g., 3- to 7-membered (mono)heterocyclyl fused with 3- to 7-membered (mono)heterocyclyl, 3- to 7-membered (mono)heterocyclyl fused with (mono)cycloalkyl, 3- to 7-membered (mono)heterocyclyl fused with C₄₋₆ (mono)cycloalkyl, C₆₋₁₀ aryl fused with 3- to 7-membered heterocyclyl, and 5- to 6-membered heteroaryl fused with 3- to 7-membered heterocyclyl, examples of which include, but are not limited to, pyrrolidinyl fused with cyclopropyl, cyclopentyl fused with aziridinyl, pyrrolidinyl fused with cyclobutyl, pyrrolidinyl fused with pyrrolidinyl, pyrrolidinyl fused with piperidinyl, pyrrolidinyl fused with piperazinyl, piperidinyl fused with morpholinyl,

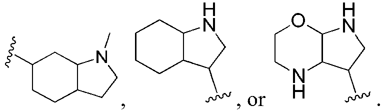

As used herein, the term "heterocyclyl" (or "heterocyclic ring") encompasses bridged heterocyclyl (bridged heterocyclic ring) and spiro heterocyclyl (spiro heterocyclic ring).

As used herein, the term "bridged heterocyclic ring" refers to a cyclic structure containing one or more (e.g., 1, 2, 3, or 4) heteroatoms (e.g., oxygen atoms, nitrogen atoms, and/or sulfur atoms) formed by two rings that share two ring atoms not directly connected, including, but not limited to, a 7- to 10-membered bridged heterocyclic ring, a 8- to 10-membered bridged heterocyclic ring, a 7- to 10-membered nitrogen-containing bridged heterocyclic ring, a 7- to 10-membered oxygen-containing bridged heterocyclic ring, a 7- to 10-membered sulfur-containing bridged heterocyclic ring, and the like, e.g., 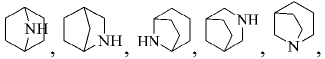 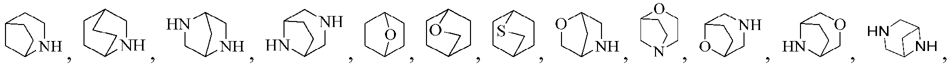 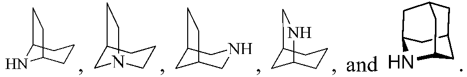 The "nitrogen-containing bridged heterocyclic ring", "oxygen-containing bridged heterocyclic ring", or "sulfur-containing bridged heterocyclic ring" optionally further contains one or more other heteroatoms selected from oxygen, nitrogen, and sulfur.

As used herein, the term "spiro heterocyclic ring" refers to a cyclic structure containing one or more (e.g., 1, 2, 3, or 4) heteroatoms (e.g., oxygen atoms, nitrogen atoms, sulfur atoms) formed by two or more rings that share one ring atom, including, but not limited to, a 5- to 10-membered spiro heterocyclic ring, a 6- to 10-membered spiro heterocyclic ring, a 6- to 10-membered nitrogen-containing spiro heterocyclic ring, a 6- to 10-membered oxygen-containing spiro heterocyclic ring, a 6- to 10-membered sulfur-containing spiro heterocyclic ring, and the like, e.g., 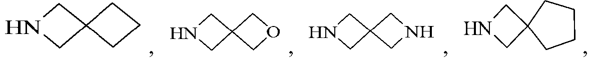 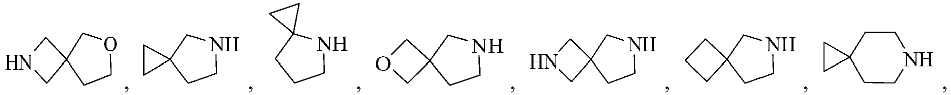 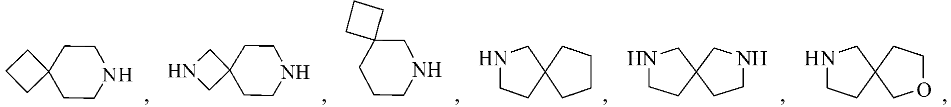 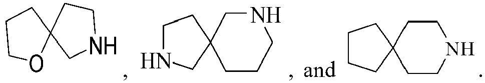 The "nitrogen-containing spiro heterocyclic ring", "oxygen-containing spiro heterocyclic ring", or "sulfur-containing spiro heterocyclic ring" optionally further contains one or more other heteroatoms selected from oxygen, nitrogen, and sulfur. The term "6- to 10-membered nitrogen-containing spiro heterocyclyl" refers to spiro heterocyclyl containing a total of 6 to 10 ring atoms, and at least one of which is a nitrogen atom.

As used herein, the terms "aryl(ene)" and "aromatic ring" refer to an all-carbon monocyclic or fused polycyclic aromatic group having a conjugated π-electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). The aryl(ene) and aromatic ring are optionally substituted with 1 or more (such as 1 to 3) suitable substituents (e.g., halogen, -OH, -CN, -NO₂, and C₁₋₆ alkyl).

The term "aralkyl" refers to alkyl substituted with aryl, wherein the aryl and the alkyl are as defined herein. Generally, the aryl may have 6 to 14 carbon atoms, and the alkyl may have 1 to 6 carbon atoms. Exemplary aralkyl includes, but is not limited to, benzyl, phenylethyl, phenylpropyl, and phenylbutyl.

As used herein, the terms "heteroaryl(ene)" and "heteroaromatic ring" refer to a monocyclic, bicyclic, or tricyclic aromatic ring system, which has 5, 6, 7, 8, 9, 10, 11, 12, 13, or 14 ring atoms, in particular 1 or 2 or 3 or 4 or 5 or 6 or 9 or 10 carbon atoms, and contains at least one heteroatom which may be identical or different (the heteroatom is, for example, oxygen, nitrogen, or sulfur), and may be benzo-fused in each case. In particular, "heteroaryl(ene)" or "heteroaromatic ring" is selected from thienyl(ene) (thiophene ring), furyl(ene) (furan ring), pyrrolyl(ene) (pyrrole ring), oxazolyl(ene) (oxazole ring), thiazolyl(ene) (thiazole ring), imidazolyl(ene) (imidazole ring), pyrazolyl(ene) (pyrazole ring), isoxazolyl(ene) (isoxazole ring), isothiazolyl(ene) (isothiazole ring), oxadiazolyl(ene) (oxadiazole ring), triazolyl(ene) (triazole ring), thiadiazolyl(ene) (thiadiazole ring), and the like, as well as benzo derivatives thereof; or pyridinyl(ene) (pyridine ring), pyridazinyl(ene) (pyridazine ring), pyrimidinyl(ene) (pyrimidine ring), pyrazinyl(ene) (pyrazine ring), triazinyl(ene) (triazine ring), and the like, as well as benzo derivatives thereof.

As used herein, the term "halogenated" or "halogen" group is defined to include F, Cl, Br, or I.

As used herein, the term "alkylthio" refers to alkyl as defined above that is attached to a parent molecular moiety via a sulfur atom. Representative examples of C₁₋₆ alkylthio include, but are not limited to, methylthio, ethylthio, tert-butylthio, and hexylthio.

As used herein, the term "nitrogen-containing heterocyclic ring" refers to a saturated or partially unsaturated monocyclic or bicyclic group, which has 2, 3, 4, 5, 6, 7, 8, 9, 10, 11, 12, or 13 carbon atoms and at least one nitrogen atom in the ring, and may also optionally contain one or more (e.g., one, two, three, or four) ring members selected from N, O, S, S=O, and S(=O)₂. The nitrogen-containing heterocyclic ring is attached to the rest of the molecule via any one of the ring members. The nitrogen-containing heterocyclic ring is preferably a saturated nitrogen-containing monocyclic ring. In particular, a 3- to 14-membered nitrogen-containing heterocyclic ring is a group having 3 to 14 carbon atoms and heteroatoms (at least one of which is a nitrogen atom) in the ring, including, but not limited to, a three-membered nitrogen-containing heterocyclic ring (e.g., aziridinyl), a four-membered nitrogen-containing heterocyclic ring (e.g., azetidinyl), a five-membered nitrogen-containing heterocyclic ring (e.g., pyrrolyl, pyrrolidinyl (pyrrolidine ring), pyrrolinyl, pyrrolidinonyl, imidazolyl, imidazolidinyl, imidazolinyl, pyrazolyl, or pyrazolinyl), a six-membered nitrogen-containing heterocyclic ring (e.g., piperidinyl (piperidine ring), morpholinyl, thiomorpholinyl, or piperazinyl), a seven-membered nitrogen-containing heterocyclic ring, and the like.

The term "substitution" means that one or more (e.g., one, two, three, or four) hydrogen atoms on a specified atom are replaced with a selection from the designated group, provided that the normal valency of the atom specified under the present circumstances is not exceeded and that the replacement results in a stable compound. A combination of substituents and/or variables is permissible only if the combination results in a stable compound.

If a substituent is described as "optionally substituted with...", the substituent may be (1) unsubstituted or (2) substituted. If a carbon of a substituent is described as being optionally substituted with one or more of a list of substituents, then one or more hydrogen atoms on the carbon (to the extent of any hydrogen atoms present) may be replaced individually and/or together with an independently selected optional substituent. If a nitrogen of a substituent is described as being optionally substituted with one or more of a list of substituents, then one or more hydrogen atoms on the nitrogen (to the extent of any hydrogen atoms present) may each be replaced with an independently selected optional substituent.

If a substituent is described as being "independently selected from" one group, each substituent is selected independently of the other. Thus, each substituent may be identical to or different from another (other) substituent(s).

As used herein, the term "one or more" refers to 1 or more than 1, such as 2, 3, 4, 5, or 10, under reasonable conditions.

Unless otherwise indicated, as used herein, the point of attachment of a substituent may be from any suitable position of the substituent.

When a bond of a substituent is shown to pass through a bond connecting two atoms in the ring, the substituent may be bonded to any one of the ring-forming atoms in the substitutable ring.

The present disclosure also includes all pharmaceutically acceptable isotopically labeled compounds, which are identical to the compounds of the present disclosure, except that one or more atoms are replaced by an atom having the same atomic number, but an atomic mass or mass number different from the atomic mass or mass number predominant in nature. Examples of isotopes suitable for incorporation into the compounds of the present disclosure include, but are not limited to, isotopes of hydrogen (e.g., deuterium (D, ²H) and tritium (T, ³H)); isotopes of carbon (e.g., ¹¹C, ¹³C, and ¹⁴C); isotopes of chlorine (e.g., ³⁶Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S). Certain isotopically-labeled compounds of the present disclosure (e.g., those into which a radioactive isotope is incorporated) can be used in drug and/or substrate tissue distribution studies (e.g., assays). The radioactive isotopes tritium (i.e., ³H) and carbon-14 (i.e., ¹⁴C) are particularly useful for this purpose because of their ease of incorporation and detection. Substitution with positron emitting isotopes (e.g., ¹¹C, ¹⁸F, ¹⁵O, and ¹³N) can be used to examine substrate receptor occupancy in positron emission tomography (PET) studies. The isotopically labeled compounds of the present disclosure can be prepared by processes analogous to those described in the accompanying routes and/or in the examples and preparations by using an appropriate isotopically labeled reagent in place of the non-labeled reagent previously employed. The pharmaceutically acceptable solvates of the present disclosure include those in which the crystallization solvent may be isotopically substituted, e.g., D₂O, acetone-d₆, or DMSO-d₆.

The term "stereoisomer" refers to an isomer formed due to at least one asymmetric center. In compounds having one or more (e.g., one, two, three, or four) asymmetric centers, racemic mixtures, single enantiomers, mixtures of diastereomers, and individual diastereomers may be generated. Certain individual molecules may also present as geometric isomers (cis/trans). Similarly, the compound of the present disclosure may present as a mixture of two or more structurally different forms in rapid equilibrium (commonly referred to as tautomers). Representative examples of tautomers include keto-enol tautomers, phenol-keto tautomers, nitroso-oxime tautomers, imine-enamine tautomers, and the like. It should be understood that the scope of the present application encompasses all such isomers or mixtures thereof in any proportion (e.g., 60%, 65%, 70%, 75%, 80%, 85%, 90%, 95%, 96%, 97%, 98%, or 99%).

A solid line ( 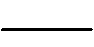 ), a solid wedge ( 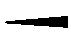 ), or a dashed wedge ( 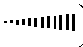 ) may be used herein to depict carbon-carbon bonds of the compounds of the present disclosure. Depiction of a bond bonded to an asymmetric carbon atom using a solid line is intended to indicate that all possible stereoisomers (e.g., specific enantiomers and racemic mixtures) at the carbon atom are included. Depiction of a bond bonded to an asymmetric carbon atom using a solid or dashed wedge is intended to indicate that the shown stereoisomer is present. When a bond is present in a racemic mixture, solid and dashed wedges are used to define relative stereochemistry, rather than absolute stereochemistry. Unless otherwise indicated, the compounds of the present disclosure are intended to be present in the forms of stereoisomers (including cis and trans isomers, optical isomers (e.g., R and S enantiomers), diastereomers, geometric isomers, rotamers, conformers, atropisomers, and mixtures thereof). The compounds of the present disclosure may exhibit one or more types of isomerism, and consist of mixtures thereof (e.g., racemic mixtures and diastereomeric pairs).

Atropisomers refer to compounds that can be separated into rotationally restricted isomers.

It should be understood that certain compounds of the present disclosure may be present in free form for use in therapy or, where appropriate, in the form of a pharmaceutically acceptable derivative thereof. In the present disclosure, the pharmaceutically acceptable derivatives include, but are not limited to, pharmaceutically acceptable salts, esters, solvates, metabolites, or prodrugs, which upon administration to a patient in need thereof are capable of providing, directly or indirectly, the compound of the present disclosure or a metabolite or residue thereof. Thus, when reference is made herein to "the compound of the present disclosure", it is also intended to encompass the various derivative forms of the compound described above. The pharmaceutically acceptable salts of the compounds of the present disclosure include acid addition salts and base addition salts thereof.

A review of suitable salts is found in Stahl and Wermuth, "Handbook of Pharmaceutical Salts: Properties, Selection, and Use" (Wiley-VCH, 2002). Methods for preparing the pharmaceutically acceptable salts of the compounds of the present disclosure are known to those skilled in the art.

As used herein, the term "ester" refers to an ester derived from each formula compound in the present application, including physiologically hydrolyzable esters (which can be hydrolyzed under physiological conditions to release the compounds of the present disclosure in the form of free acids or alcohols). The compounds of the present disclosure *per se* may also be esters.

The compounds of the present disclosure may be present in the form of solvates (preferably hydrates), and the compounds of the present disclosure contain a polar solvent as a structural element of the crystal lattice of the compound, particularly, for example, water, methanol, or ethanol. The amount of the polar solvent, particularly water, may be present in a stoichiometric or non-stoichiometric ratio.

Also included within the scope of the present disclosure are metabolites of the compounds of the present disclosure, i.e., substances formed *in vivo* upon administration of the compounds of the present disclosure. Such products may result, for example, from oxidation, reduction, hydrolysis, amidation, deamidation, esterification, deesterification, enzymatic digestion, and the like of the administered compound. Thus, the present disclosure includes metabolites of the compounds of the present disclosure, including compounds prepared by the process of contacting the compounds of the present disclosure with a mammal for a time sufficient to produce metabolites thereof.

Further included within the scope of the present disclosure are prodrugs of the compounds of the present disclosure, which are certain derivatives of the compounds of the present disclosure that may themselves have relatively weak or no pharmacological activity and, upon administration into or onto the body, are converted to the compounds of the present disclosure having the desired activity by, for example, hydrolysis.

Generally, such prodrugs will be functional group derivatives of the compounds, which are readily converted into desired therapeutically active compounds *in vivo.* Additional information on the use of prodrugs can be found in "Pro-drugs as Novel Delivery Systems", volume 14, ACS Symposium Series (T. Higuchi and V. Stella) and "Bioreversible Carriers in Drug Design", Pergamon Press, 1987 (E. B. Roche Ed., American Pharmaceutical Association). The prodrugs of the present disclosure may be prepared by, for example, replacing appropriate functional groups present in the compounds of the present disclosure with certain moieties known to those skilled in the art as "pro-moieties", for example, as described in "Design of Prodrugs", H. Bundgaard (Elsevier, 1985).

The present disclosure further encompasses the compounds of the present disclosure containing a protective group. In any process of preparing the compounds of the present disclosure, it may be necessary and/or desirable to protect sensitive or reactive groups on any of the molecules concerned, thereby forming a chemically protected form of the compounds of the present disclosure. This can be achieved by conventional protective groups, as described, for example, in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which are incorporated herein by reference. The protective groups may be removed at an appropriate subsequent stage by methods known in the art.

As used herein, the term "about" means within + 10%, preferably within + 5%, and more preferably within ± 2% of the stated numerical value.

### Compound

In some embodiments, the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound has a structure of formula (I): 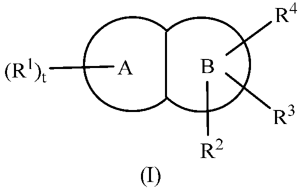 wherein: 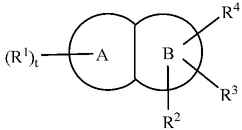 is selected from 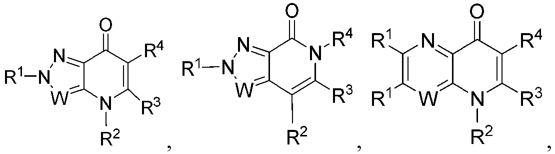 and 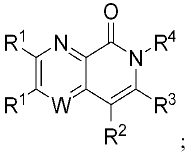
W is C-R¹ or N;
R¹, at each occurrence, is each independently selected from H, halogen, -OH, -NH₂, -CN, -NO₂, -SF₅, C₁₋₆ alkyl, deuterated C₁₋₆ alkyl, C₁₋₆ haloalkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, - C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a_}C(=O)R^{b}, -NR^{a}-C(=O)OR^{b}, -NR^{a}-S(=O)₂-R^{b}, -NR^{a}-C(=O)-NR^{a}R^{b}, -P(=O)R^{a}R^{b}, -C₁₋₆ alkylene-R^{a}, -C₁₋₆ alkylene-OR^{a}, -C₁₋₆ alkylene-NR^{a}R^{b}, -O-C₁₋₆ alkylene-NR^{a}R^{b}, (-C₃₋₆ cyclohydrocarbylene)-CN, and (-C₃₋₆ cyclohydrocarbylene)-C₁₋₆ haloalkyl;
or, two R¹ at adjacent positions, together with the group to which they are attached, optionally form a C₃₋₆ hydrocarbon ring, a 3- to 10-membered heterocyclic ring, a C₆₋₁₀ aromatic ring, or a 5- to 14-membered heteroaromatic ring;
R² is 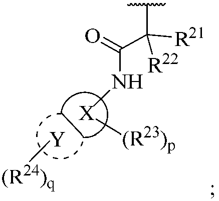
R³, R²¹, R²², R²³, and R²⁴, at each occurrence, are each independently selected from H, halogen, -OH, -NH₂, -CN, -NO₂, -SF₅, C₁₋₆ alkyl, deuterated C₁₋₆ alkyl, C₁₋₆ haloalkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, - C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -S(=O)(-NR^{a})R^{b}, - NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -P(=O)R^{a}R^{b}, -C₁₋₆ alkylene-R^{a}, -C₁₋₆ alkylene-OR^{a}, -C₁₋₆ alkylene-NR^{a}R^{b}, -O-C₁₋₆ alkylene-NR^{a}R^{b}, (-C₃₋₆ cyclohydrocarbylene)-CN, and (-C₃₋₆ cyclohydrocarbylene)-C₁₋₆ haloalkyl;
or, R³ and R²¹ or R²², together with the groups to which they are attached, optionally form a C₃₋₆ hydrocarbon ring, a 3- to 10-membered heterocyclic ring, a C₆₋₁₀ aromatic ring, or a 5- to 14-membered heteroaromatic ring;
R⁴ is 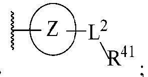
R⁴¹ is selected from a C₃₋₆ hydrocarbon ring, a 3- to 10-membered heterocyclic ring, a C₆₋₁₀ aromatic ring, and a 5- to 14-membered heteroaromatic ring;
ring X and ring Z are each independently selected from a C₃₋₆ hydrocarbon ring, a 3- to 10-membered heterocyclic ring, a C₆₋₁₀ aromatic ring, and a 5- to 14-membered heteroaromatic ring;
ring Y is absent or selected from a C₃₋₆ hydrocarbon ring, a 3- to 10-membered heterocyclic ring, a C₆₋₁₀ aromatic ring, and a 5- to 14-membered heteroaromatic ring; when ring Y is absent, R²⁴ is also absent;
L² is selected from -O-, -C(=O)-, -NRC(=O)-, -S-, -S(=O)-, -S(=O)₂-, C₁₋₆ alkylene, and -O-(C₁₋₆ alkylene)-; R, R^{a}, and R^{b}, at each occurrence, are each independently selected from H, C₁₋₆ alkyl, C₃₋₁₀ cyclohydrocarbyl, 3- to 10-membered heterocyclyl, C₆₋₁₀ aryl, 5- to 14-membered heteroaryl, and C₆₋₁₂ aralkyl;
the alkylene, the alkyl, the alkenyl, the alkynyl, the cyclohydrocarbylene, the cyclohydrocarbyl, the hydrocarbon ring, the heterocyclyl, the heterocyclic ring, the aryl, the aromatic ring, the heteroaryl, the heteroaromatic ring, and the aralkyl described above, at each occurrence, are each optionally substituted with one or more substituents independently selected from the following: a deuterium atom, halogen, -OH, =O, - NH₂, -CN, -NO₂, =CH₂, C₁₋₆ alkyl, deuterated C₁₋₆ alkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, -C(=O)R^{c}, - OC(=O)R^{c}, -C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{c}, -C₁₋₆ alkylene-NR^{c}R^{d}, and -O-C₁₋₆ alkylene-NR^{c}R^{d}, and when the same ring atom or adjacent ring atoms of the cyclohydrocarbylene, the cyclohydrocarbyl, the hydrocarbon ring, the heterocyclyl, the heterocyclic ring, the aryl, the aromatic ring, the heteroaryl, and the heteroaromatic ring are substituted with two substituents, the two substituents, together with groups to which they are attached, optionally form a C₃₋₆ hydrocarbon ring, a 3- to 10-membered heterocyclic ring, a C₆₋₁₀ aromatic ring, or a 5- to 14-membered heteroaromatic ring; the alkylene, the alkyl, the alkenyl, =CH₂, the alkynyl, the cyclohydrocarbyl, the hydrocarbon ring, the heterocyclyl, the heterocyclic ring, the aryl, the aromatic ring, the heteroaryl, the heteroaromatic ring, and the aralkyl are each further optionally substituted with one or more substituents independently selected from the following: halogen, -OH, =O, -C(=O)O-tert-butyl, -NH₂, -CN, -NO₂, C₁₋₆ alkyl, C₁₋₆ haloalkyl, C₃₋₆ cyclohydrocarbyl, 3- to 10-membered heterocyclyl, C₆₋₁₀ aryl, 5- to 14-membered heteroaryl, C₆₋₁₂ aralkyl, -C₁₋₆ alkylene-C₃₋₆ cyclohydrocarbyl, -O-C₁₋₆ alkyl, and -C₁₋₆ alkylene-O-C₁₋₆ alkyl;
R^{c} and R^{d}, at each occurrence, are each independently selected from: H, C₁₋₆ alkyl, C₃₋₁₀ cyclohydrocarbyl, 3- to 10-membered heterocyclyl, C₆₋₁₀ aryl, 5- to 14-membered heteroaryl, and C₆₋₁₂ aralkyl, wherein the alkyl, the cyclohydrocarbyl, the heterocyclyl, the aryl, the heteroaryl, and the aralkyl are further optionally substituted with one or more substituents independently selected from the following: halogen, -OH, =O, - C(=O)O-tert-butyl, -NH₂, -CN, -NO₂, C₁₋₆ alkyl, C₁₋₆ haloalkyl, C₃₋₆ cyclohydrocarbyl, 3- to 10-membered heterocyclyl, C₆₋₁₀ aryl, 5- to 14-membered heteroaryl, C₆₋₁₂ aralkyl, and -C₁₋₆ alkylene-O-C₁₋₆ alkyl; and
p, q, and t are each independently an integer selected from 1, 2, or 3.

In some embodiments, R¹, at each occurrence, is each independently selected from H, halogen, C₁₋₆ alkyl, C₃₋₆ cyclohydrocarbyl, 3- to 10-membered heterocyclyl, C₆₋₁₀ aryl, 5- to 14-membered heteroaryl, -OR^{a}, and -NR^{a}R^{b}, and preferably, R¹, at each occurrence, is each independently selected from H, halogen, C₁₋₆ alkyl, C₃₋₆ cyclohydrocarbyl, and -OR^{a}, wherein the alkyl, the cyclohydrocarbyl, the heterocyclyl, the aryl, and the heteroaryl are each optionally substituted with one or more substituents independently selected from the following: halogen, C₁₋₆ alkyl, C₂₋₆ alkenyl, =CH₂, C₃₋₆ cyclohydrocarbyl, 3- to 10-membered heterocyclyl, C₆₋₁₀ aryl, and 5- to 14-membered heteroaryl; the alkyl, the alkenyl, =CH₂, the cyclohydrocarbyl, the heterocyclyl, the aryl, and the heteroaryl are each further optionally substituted with one or more substituents independently selected from the following: halogen, C₁₋₆ alkyl, C₃₋₆ cyclohydrocarbyl, 3- to 10-membered heterocyclyl, and -C₁₋₆ alkylene-C₃₋₆ cyclohydrocarbyl.

In a preferred embodiment, R¹ is H, methyl, halogen, methoxy,

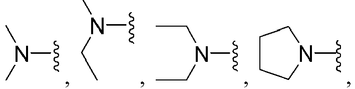

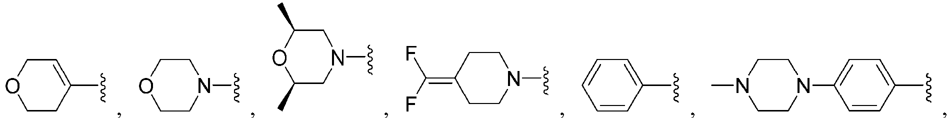

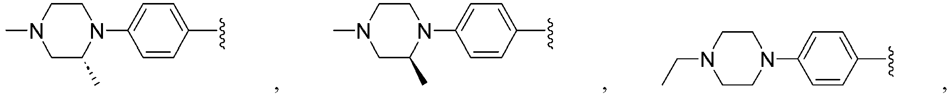

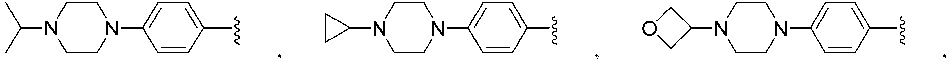

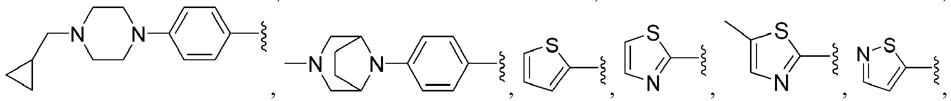

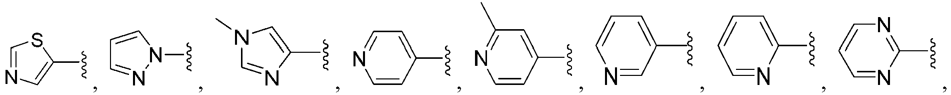

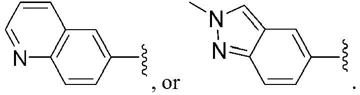

In some embodiments, two R¹ at adjacent positions, together with the group to which they are attached, optionally form a C₃₋₆ hydrocarbon ring, a 3- to 10-membered heterocyclic ring, a C₆₋₁₀ aromatic ring, or a 5-to 14-membered heteroaromatic ring, and the hydrocarbon ring, the heterocyclic ring, the aromatic ring, and the heteroaromatic ring are each optionally substituted with one or more substituents independently selected from the following: halogen, C₁₋₆ alkyl, and C₁₋₆ haloalkyl.

In a preferred embodiment, two R¹ at adjacent positions, together with the group to which they are attached, optionally form 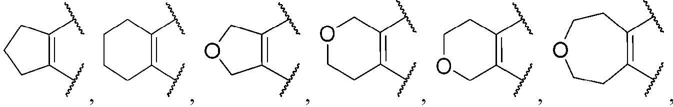 a benzene ring, or a pyridine ring, each being optionally substituted with one or more substituents independently selected from the following: halogen, C₁₋₆ alkyl, and C₁₋₆ haloalkyl.

In some embodiments, ring X is a benzene ring, a 5- to 6-membered heterocyclic ring, or a 5- to 6-membered heteroaromatic ring (preferably a thiophene ring, a thiazole ring, or a pyridine ring), and ring Y is absent. In some embodiments, ring X is a benzene ring or a 5- to 6-membered heteroaromatic ring, and ring Y is a C₃₋₆ hydrocarbon ring, a 5- to 6-membered heterocyclic ring, or a 5- to 6-membered heteroaromatic ring.

In a preferred embodiment, 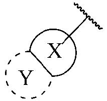 is 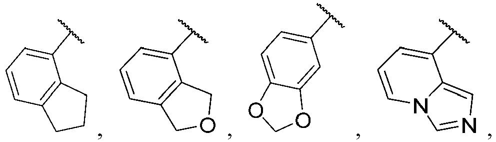 or 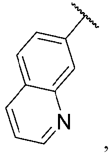 more preferably

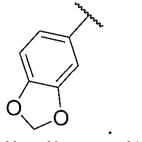

In some embodiments, R²¹, R²², R²³, and R²⁴, at each occurrence, are each independently selected from H, halogen, -SF₅, C₁₋₆ alkyl, C₃₋₆ cyclohydrocarbyl, 3- to 10-membered heterocyclyl, -O-(C₁₋₆ alkyl), -S(=O)₂-(C₁₋₆ alkyl), -S(=O)₂-(C₃₋₆ cyclohydrocarbyl), -P(=O)(C₁₋₆ alkyl)₂, (-C₃₋₆ cyclohydrocarbylene)-CN, and (-C₃₋₆ cyclohydrocarbylene)-C₁₋₆ alkyl, and the alkyl, the cyclohydrocarbylene, the cyclohydrocarbyl, and the heterocyclyl are each optionally substituted with one or more substituents independently selected from the following: halogen, C₁₋₆ alkyl, and C₁₋₆ haloalkyl.

In a preferred embodiment, R²¹, R²², R²³, and R²⁴, at each occurrence, are each independently selected from H, halogen, -SF₅, C₁₋₆ alkyl, C₃₋₆ cyclohydrocarbyl, 3- to 10-membered heterocyclyl, and -O-(C₁₋₆ alkyl), and the alkyl, the cyclohydrocarbyl, and the heterocyclyl are each optionally substituted with one or more substituents independently selected from the following: halogen, C₁₋₆ alkyl, and C₁₋₆ haloalkyl.

In some embodiments, 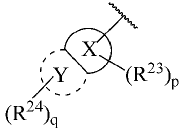 is selected from: 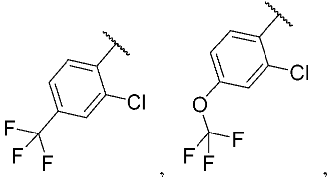 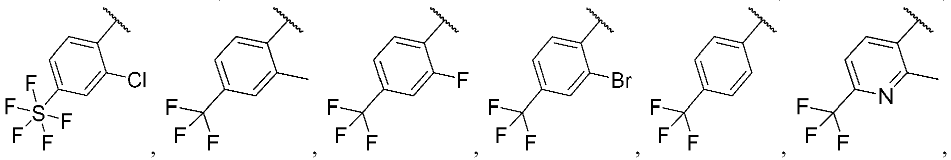 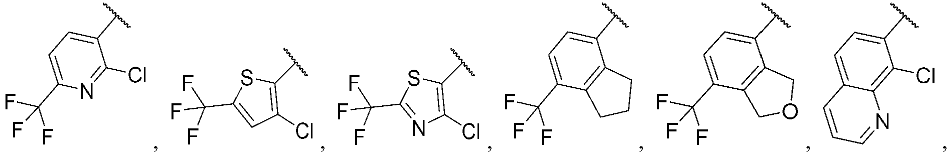 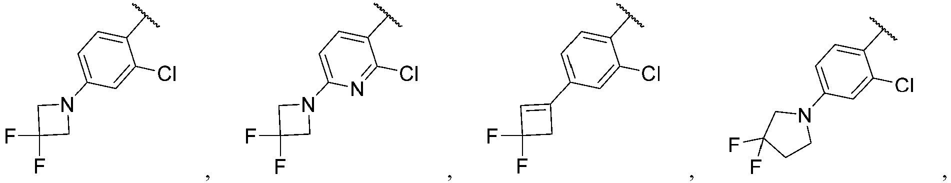 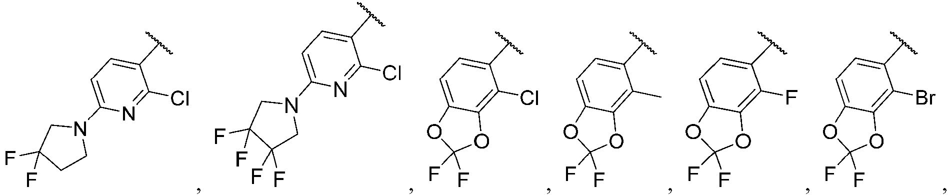 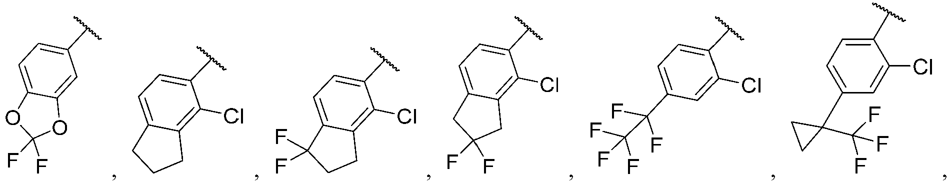 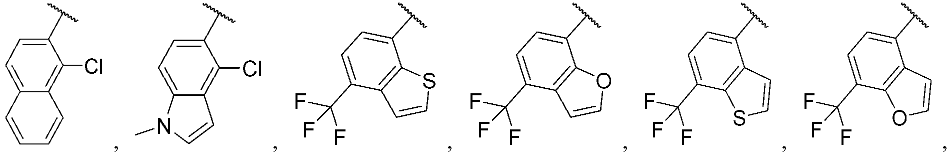 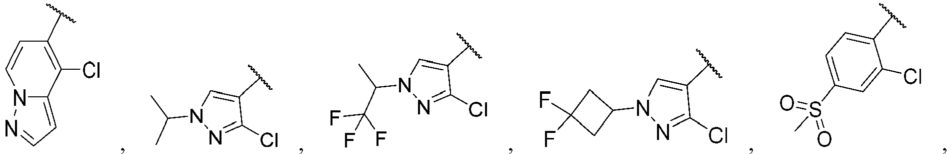 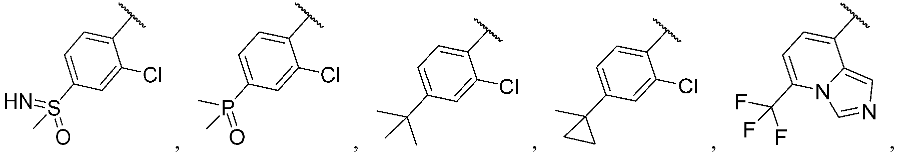 and

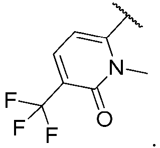

In some embodiments, 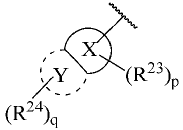 is selected from:

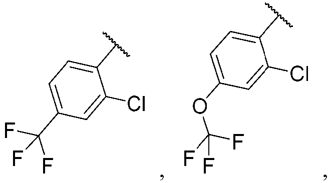

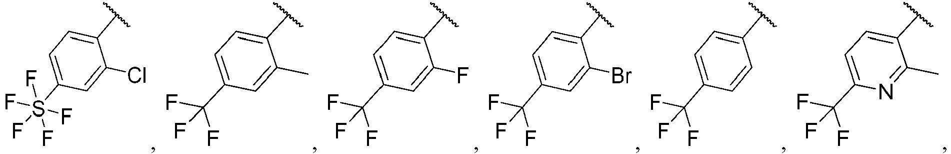

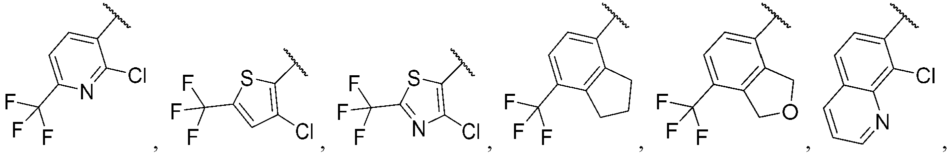

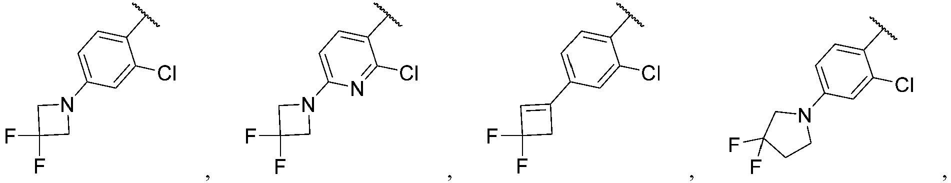

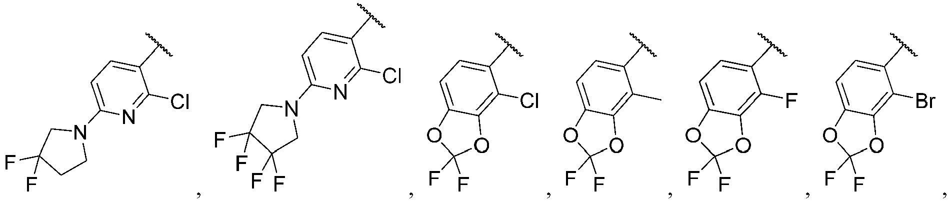

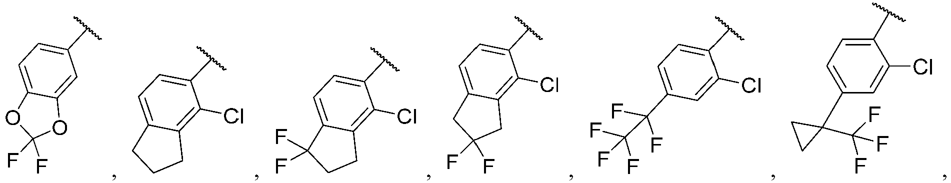

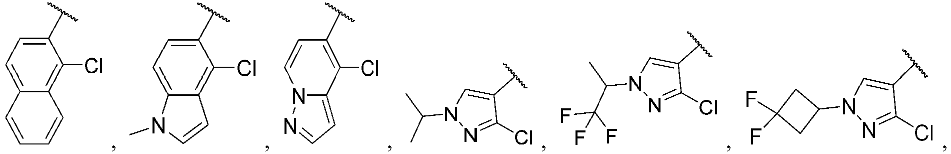

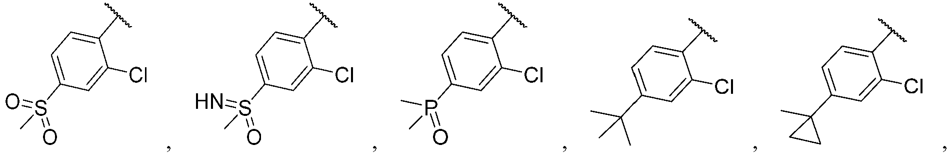

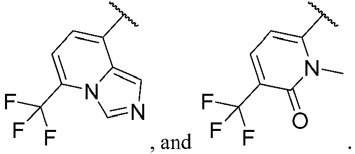

In some embodiments, R³ is H, C₁₋₆ alkyl, -OR^{a}, or -SR^{a}; preferably, R³ is H, ethyl, -O-CH₃, or -S-CH₃.

In some embodiments, R³ and R²¹ or R²², together with the groups to which they are attached, optionally form a C₃₋₆ hydrocarbon ring or a 3- to 10-membered heterocyclic ring, wherein the hydrocarbon ring and the heterocyclic ring are optionally substituted with one or more substituents selected from a deuterium atom, halogen, -OH, C₁₋₆ alkyl, C₁₋₆ haloalkyl, and -O-C₁₋₆ alkyl, and when the same ring atom or adjacent ring atoms of the hydrocarbon ring and the heterocyclic ring are substituted with two substituents, the two substituents, together with groups to which they are attached, optionally form a C₃₋₆ hydrocarbon ring or a 3-to 10-membered heterocyclic ring.

In a preferred embodiment, R³ and R²¹ or R²², together with the groups to which they are attached, optionally form a cyclopentene ring, a cyclohexene ring, a pyrrolidine ring, a piperidine ring, or a morpholine ring, wherein the cyclopentene ring, the cyclohexene ring, the pyrrolidine ring, the piperidine ring, and the morpholine ring are optionally substituted with one or more substituents selected from a deuterium atom, halogen, -OH, C₁₋₆ alkyl, C₁₋₆ haloalkyl, and -O-C₁₋₆ alkyl, and when the same ring atom of the cyclopentene ring, the cyclohexene ring, the pyrrolidine ring, the piperidine ring, and the morpholine ring is substituted with two substituents, the two substituents, together with the groups to which they are attached, optionally form a C₃₋₆ hydrocarbon ring.

In some embodiments, ring Z is a 3- to 10-membered heterocyclic ring or a benzene ring, preferably a 5- to 10-membered heterocyclic ring, and more preferably a 5- to 6-membered heterocyclic ring; and
the heterocyclic ring and the benzene ring, at each occurrence, are each optionally substituted with one or more substituents independently selected from the following: halogen, C₁₋₆ alkyl, and C₁₋₆ haloalkyl.

In a preferred embodiment, ring Z is

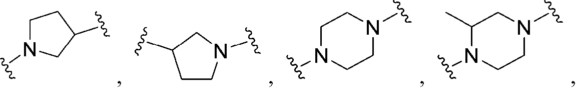

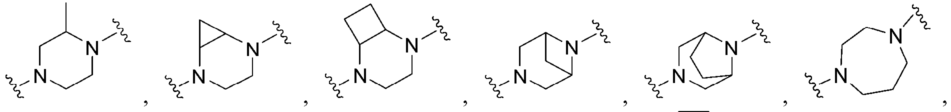

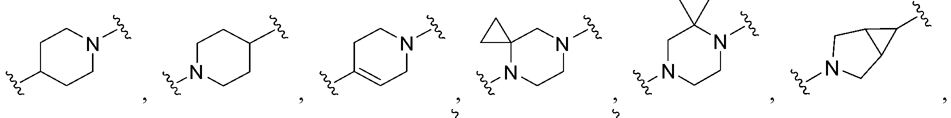

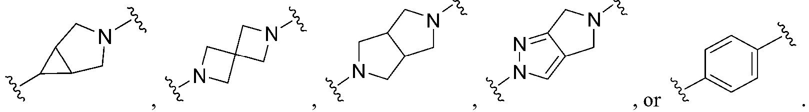

In some embodiments, L² is -C(=O)- or -NRC(=O)-, and R is H or C₁₋₆ alkyl.

In a preferred embodiment, L² is -C(=O)-.

In some embodiments, R⁴¹ is selected from a 3- to 10-membered heterocyclic ring, a C₆₋₁₀ aromatic ring, and a 5- to 14-membered heteroaromatic ring, and the heterocyclic ring, the aromatic ring, and the heteroaromatic ring are each optionally substituted with one or more substituents independently selected from the following: halogen, -OH, C₁₋₆ alkyl, -O-C₁₋₆ alkyl, and -S-C₁₋₆ alkyl, preferably, the heterocyclic ring, the aromatic ring, and the heteroaromatic ring at least being substituted with -OH or -O-C₁₋₆ alkyl.

In some embodiments, -L²-R⁴¹ is

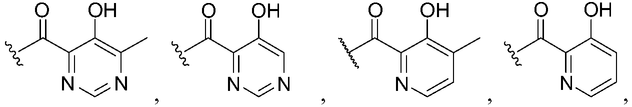

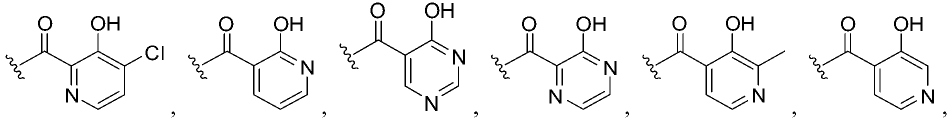

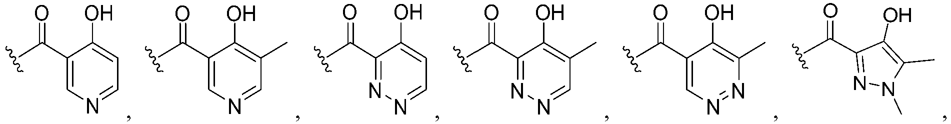

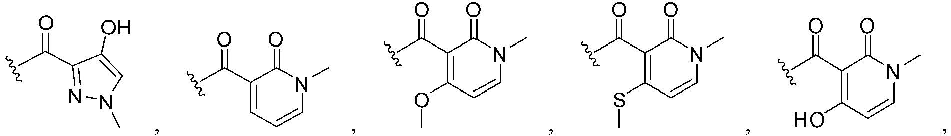

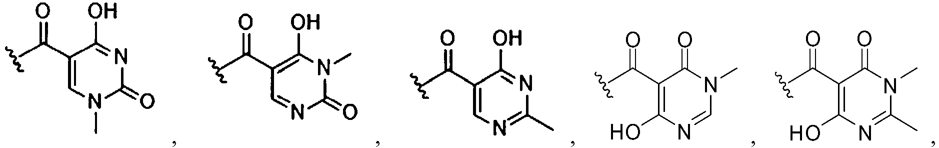

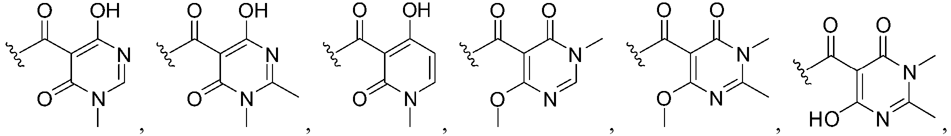

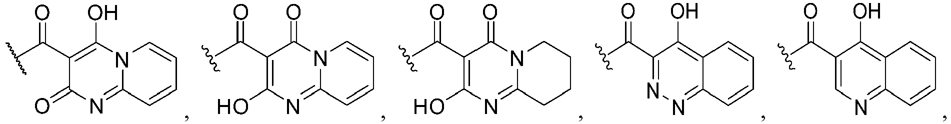

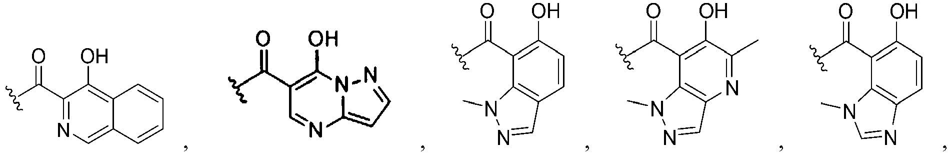

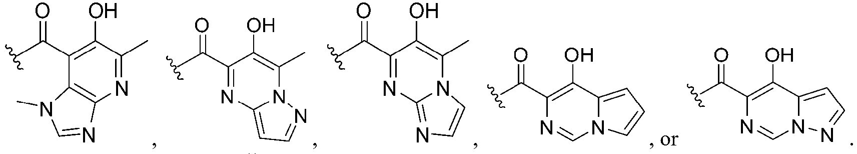

In some embodiments, R⁴¹ is a 5- to 6-membered heteroaromatic ring, preferably a 6-membered heteroaromatic ring, and more preferably a pyridine ring or a pyrimidine ring, which is at least substituted with one -OH.

In a preferred embodiment, -L²-R⁴¹ is

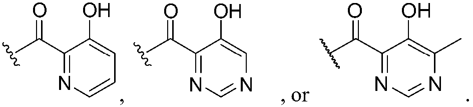

In some embodiments, the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound has structures of the following formulas: 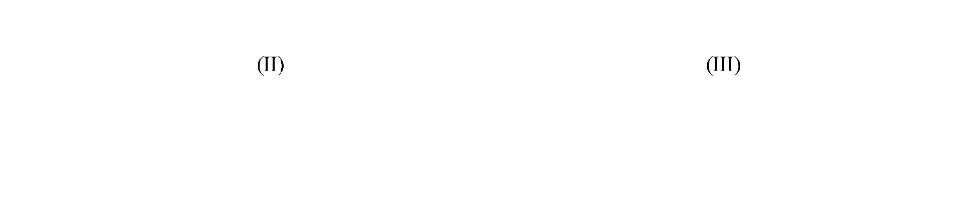 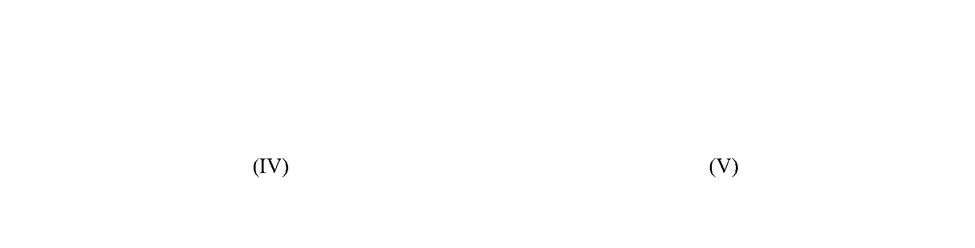 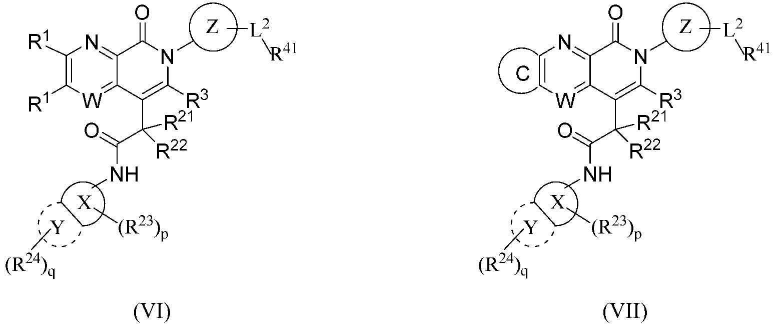 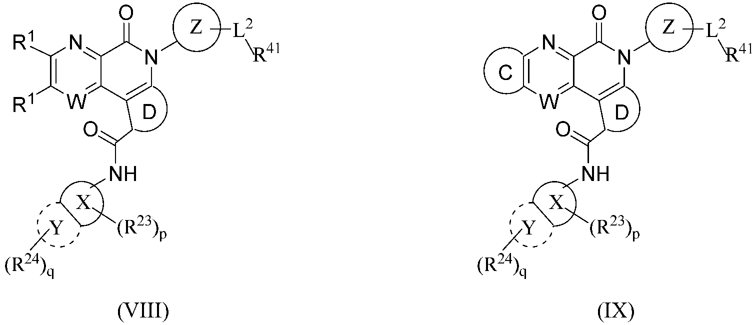 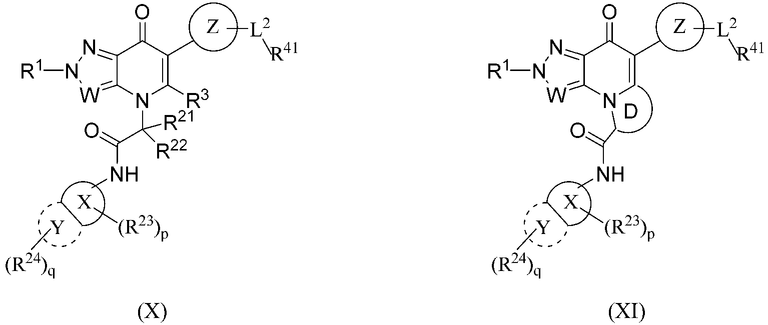 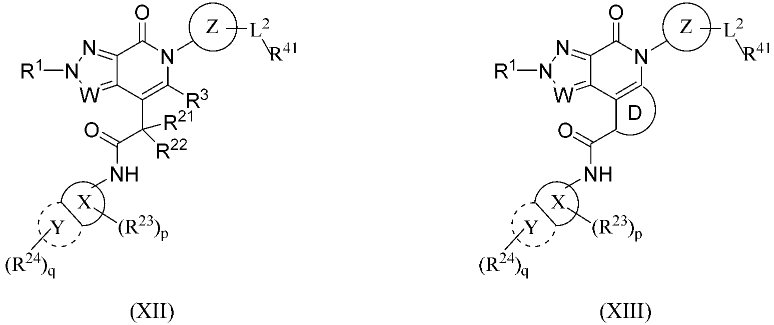 wherein:
ring C and ring D are each independently a C₃₋₆ hydrocarbon ring, a 3- to 10-membered heterocyclic ring, a C₆₋₁₀ aromatic ring, or a 5- to 14-membered heteroaromatic ring, wherein the hydrocarbon ring, the heterocyclic ring, the aromatic ring, and the heteroaromatic ring are each optionally substituted with one or more substituents independently selected from the following: a deuterium atom, halogen, -OH, C₁₋₆ alkyl, C₁₋₆ haloalkyl, and -O-C₁₋₆ alkyl, and when the same ring atom or adjacent ring atoms of the hydrocarbon ring, the heterocyclic ring, the aromatic ring, and the heteroaromatic ring are substituted with two substituents, the two substituents, together with groups to which they are attached, optionally form a C₃₋₆ hydrocarbon ring, a 3- to 10-membered heterocyclic ring, a C₆₋₁₀ aromatic ring, or a 5- to 14-membered heteroaromatic ring;
preferably, ring C is 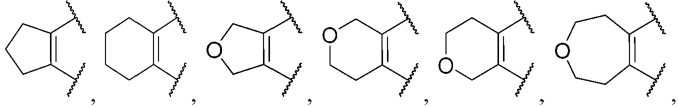 a benzene ring, or a pyridine ring, each being optionally substituted with one or more substituents independently selected from the following: halogen, C₁₋₆ alkyl, and C₁₋₆ haloalkyl; and/or
ring D is a cyclopentene ring, a cyclohexene ring, a pyrrolidine ring, a piperidine ring, or a morpholine ring, each being optionally substituted with one or more substituents independently selected from the following: a deuterium atom, halogen, -OH, C₁₋₆ alkyl, C₁₋₆ haloalkyl, and -O-C₁₋₆ alkyl; when the same ring atom or adjacent ring atoms of ring D are substituted with two substituents, the two substituents, together with groups to which they are attached, optionally form a C₃₋₆ hydrocarbon ring or a 3- to 10-membered heterocyclic ring; the other groups are as defined above.

The present disclosure encompasses technical solutions/compounds resulting from any combination of the various embodiments.

In a preferred embodiment, the present disclosure provides a compound, or a pharmaceutically acceptable salt, an ester, a stereoisomer, an atropisomer, a tautomer, a polymorph, a solvate, a metabolite, an isotopically labeled compound, or a prodrug thereof, wherein the compound is selected from:

| **Compound No.** | **Structure** | **Compound No.** | **Structure** |
|---|---|---|---|
| **B1** | 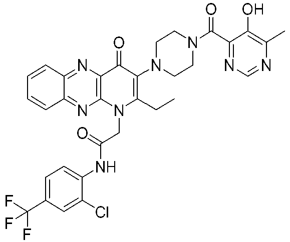 | **B2** | 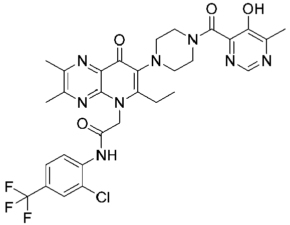 |
| **B3** | 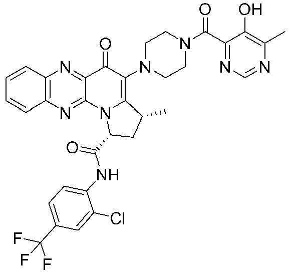 | **B4** | 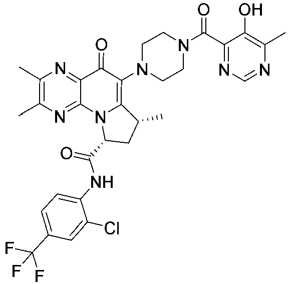 |
| **B5** | 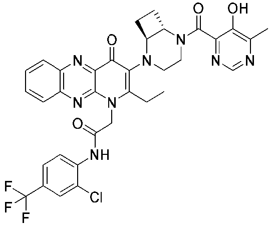 | **B6** | 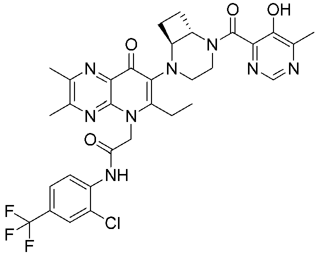 |
| **B7** | 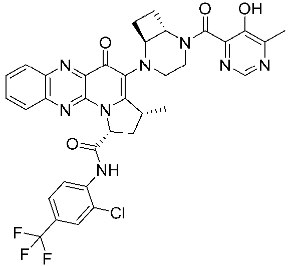 | **B8** | 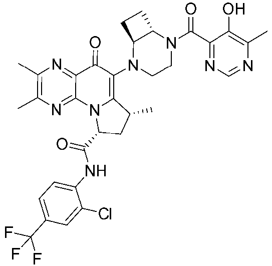 |
| **B9** | 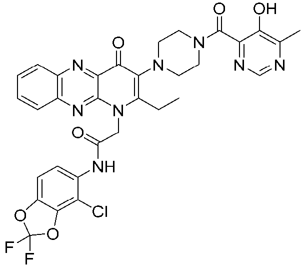 | **B10** | 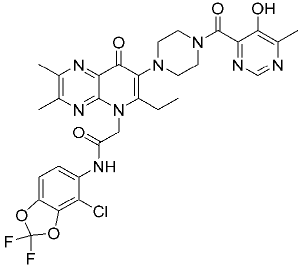 |
| **B11** | 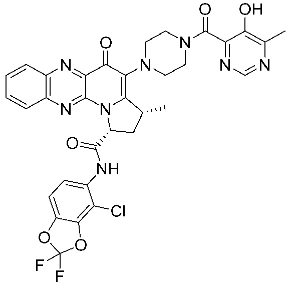 | **B12** | 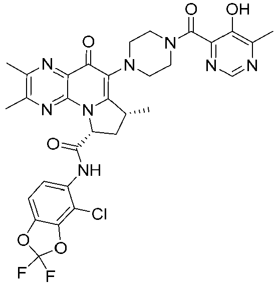 |
| **B17** | 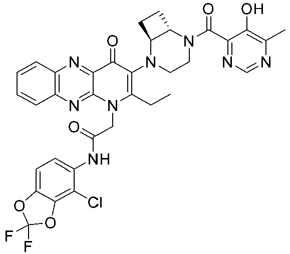 | **B18** | 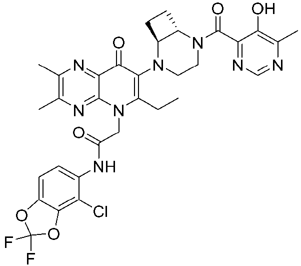 |
| **B19** | 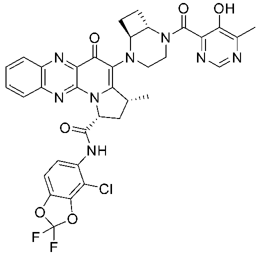 | **B20** | 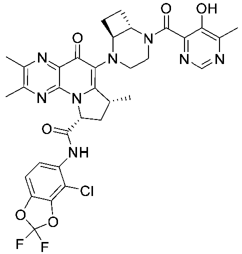 |
| **B21** | 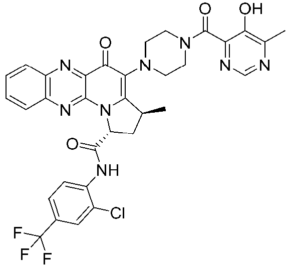 | **B22** | 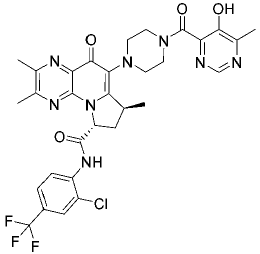 |
| **B23** | 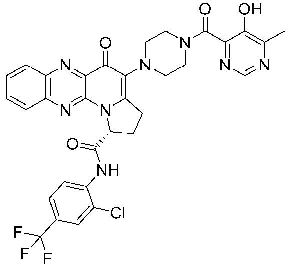 | **B24** | 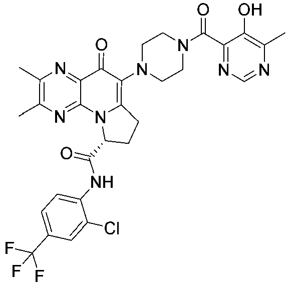 |
| **B25** | 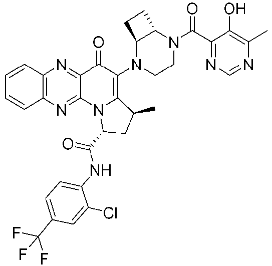 | **B26** | 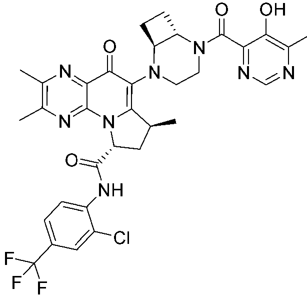 |
| **B27** | 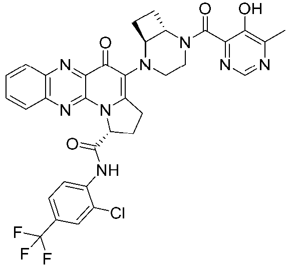 | **B28** | 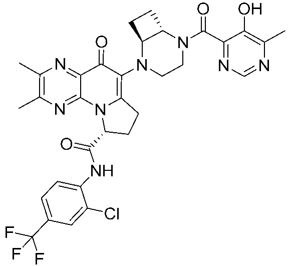 |
| **B29** | 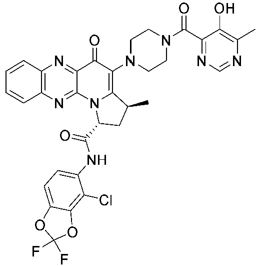 | **B30** | 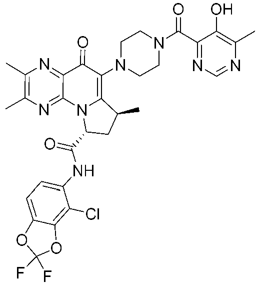 |
| **B31** | 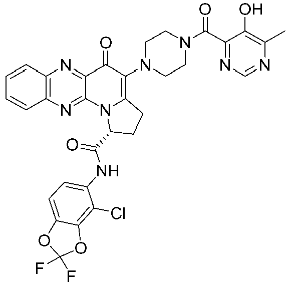 | **B32** | 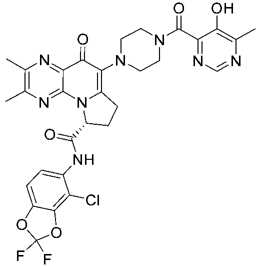 |
| **B33** | 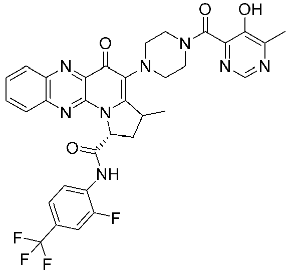 | **B34** | 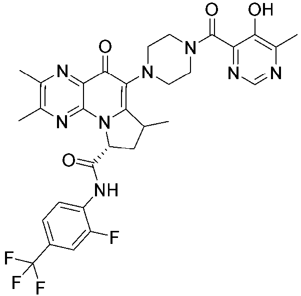 |
| **B35** | 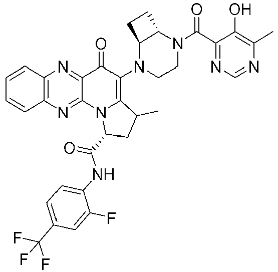 | **B36** | 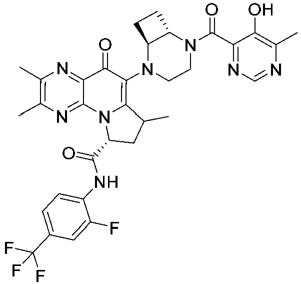 |
| **B37** | 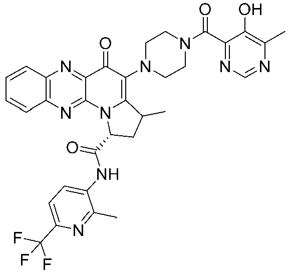 | **B38** | 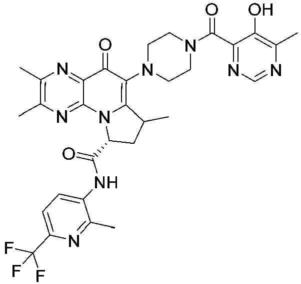 |
| **B39** | 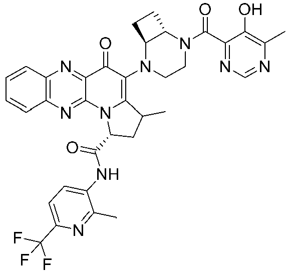 | **B40** | 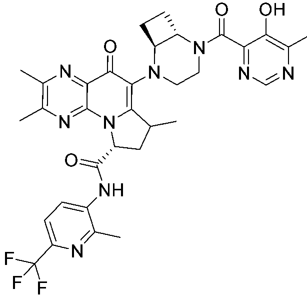 |
| **B41** | 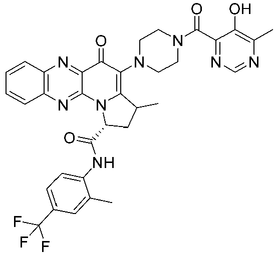 | **B42** | 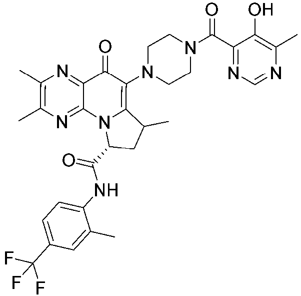 |
| **B43** | 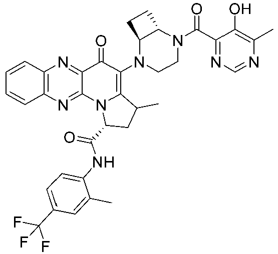 | **B44** | 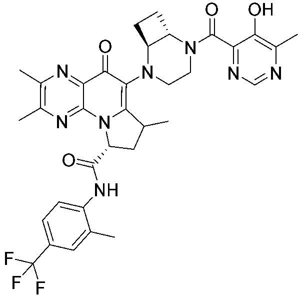 |
| **B45** | 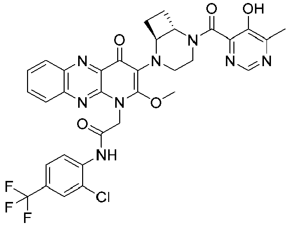 | **B46** | 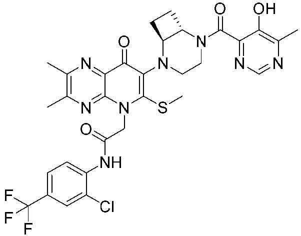 |
| **B47** | 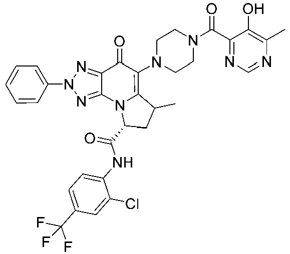 | **B48** | 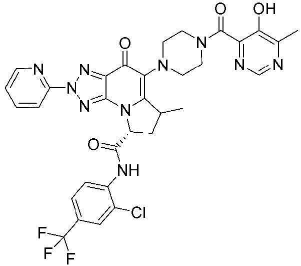 |
| **B49** | 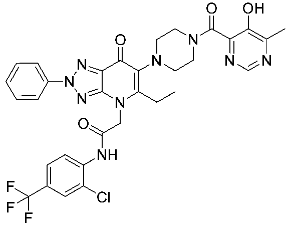 | **B50** | 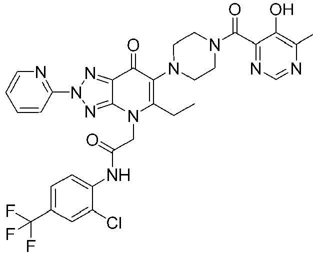 |
| **B51** | 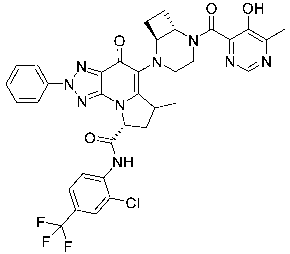 | **B52** | 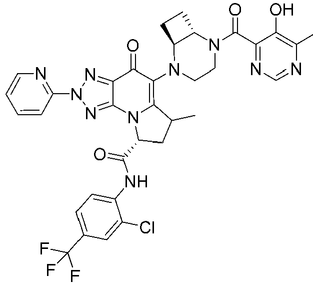 |
| **B53** | 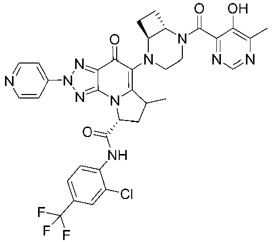 | **B54** | 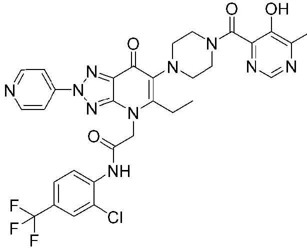 |
| **B55** | 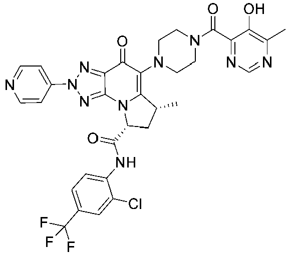 | **B56** | 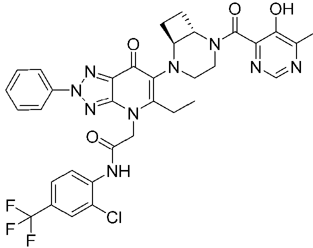 |
| **B57** | 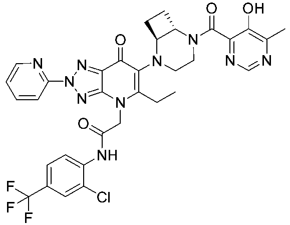 | **B58** | 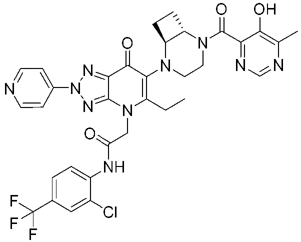 |
| **B59** | 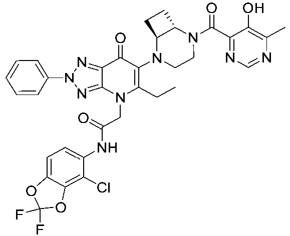 | **B60** | 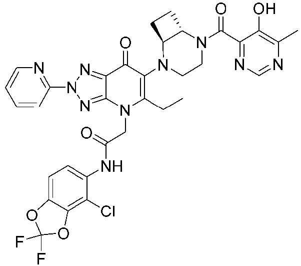 |
| **B61** | 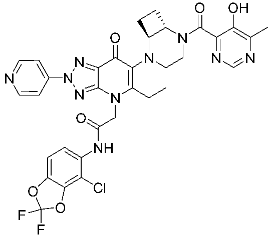 | **B62** | 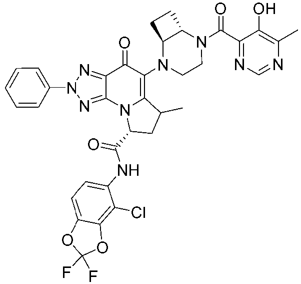 |
| **B63** | 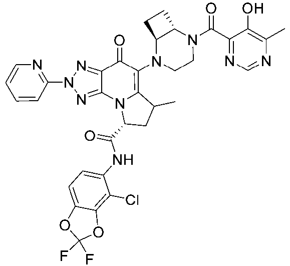 | **B64** | 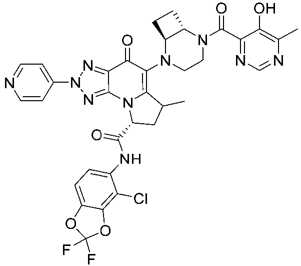 |
| **B65** | 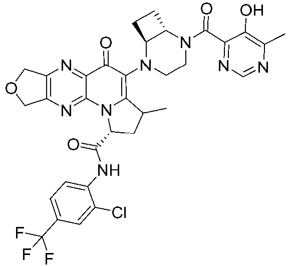 | **B66** | 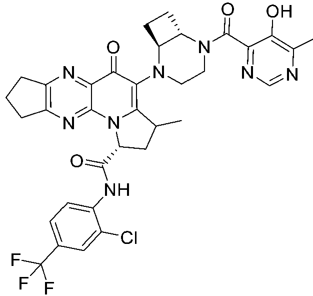 |
| **B67** | 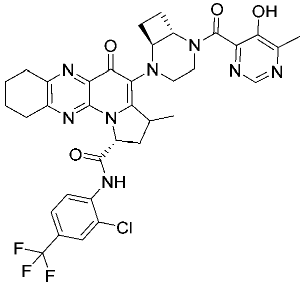 | **B68** | 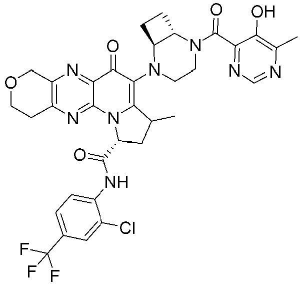 |
| **B69** | 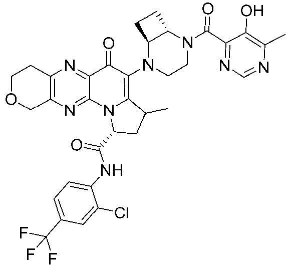 | **B70** | 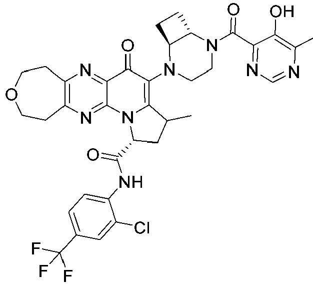 |
| **B71** | 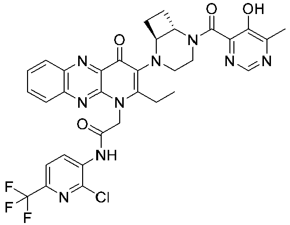 | **B72** | 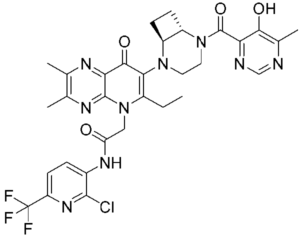 |
| **B73** | 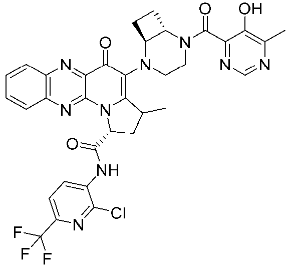 | **B74** | 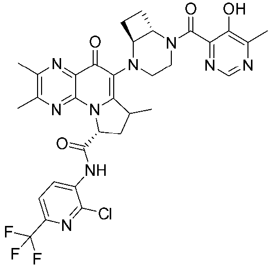 |
| **B75** | 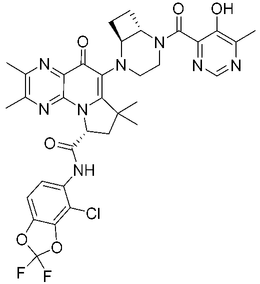 | **B76** | 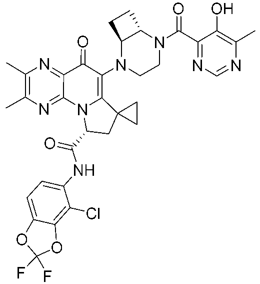 |
| **B77** | 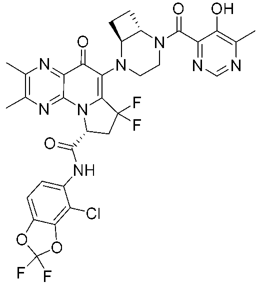 | **B78** | 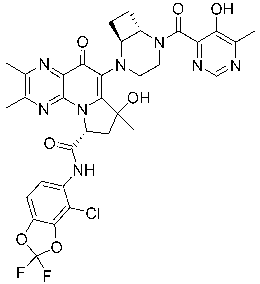 |
| **B79** | 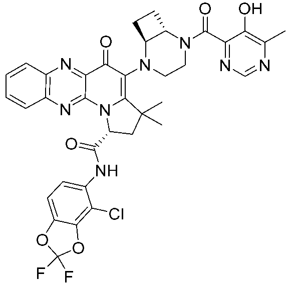 | **B80** | 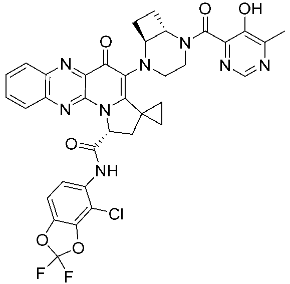 |
| **B81** | 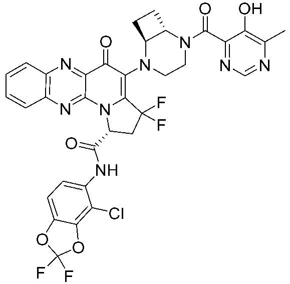 | **B82** | 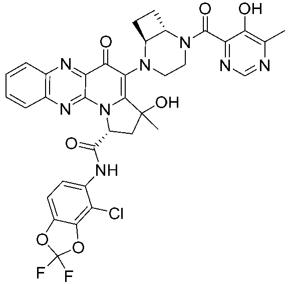 |
| **B83** | 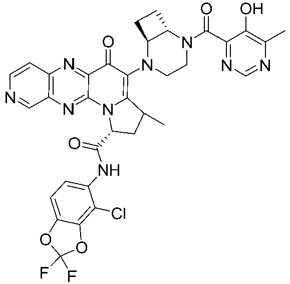 | **B84** | 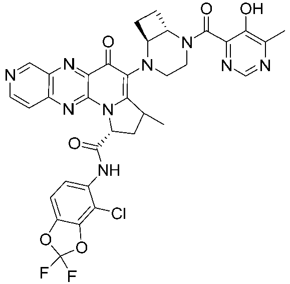 |
| **B85** | 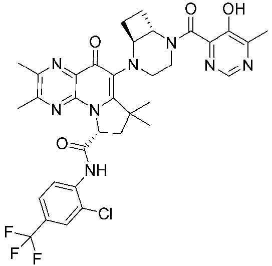 | **B86** | 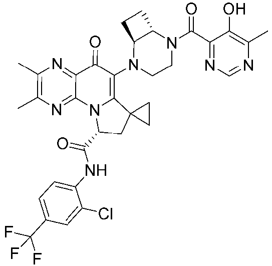 |
| **B87** | 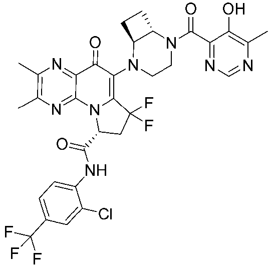 | **B88** | 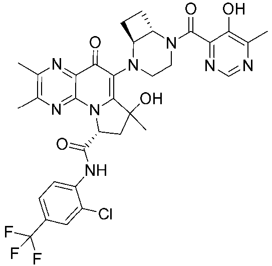 |
| **B89** | 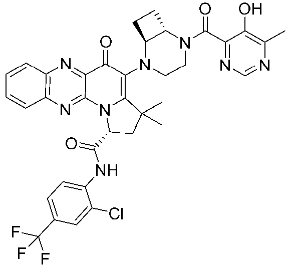 | **B90** | 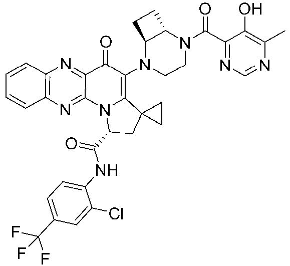 |
| **B91** | 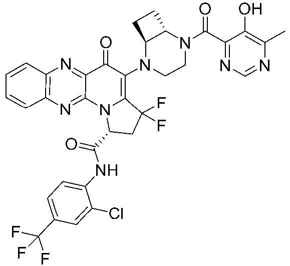 | **B92** | 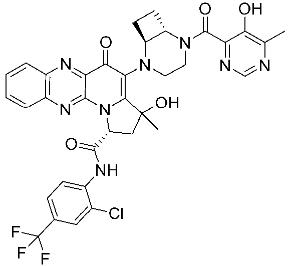 |
| **B93** | 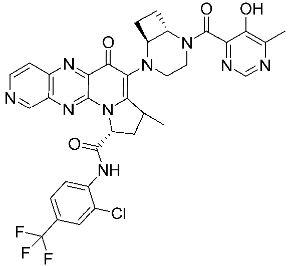 | **B94** | 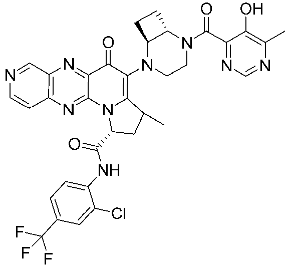 |
| **B95** | 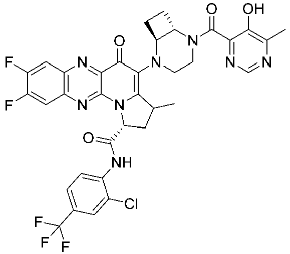 | **B96** | 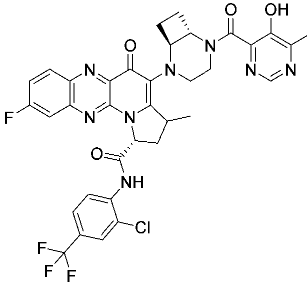 |
| **B97** | 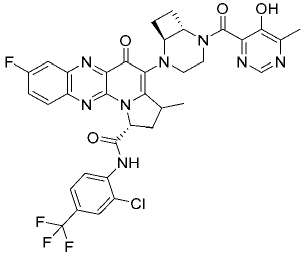 | **B98** | 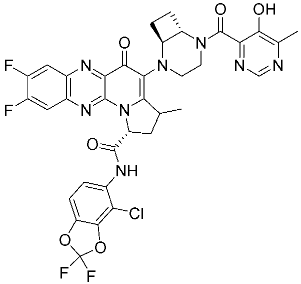 |
| **B99** | 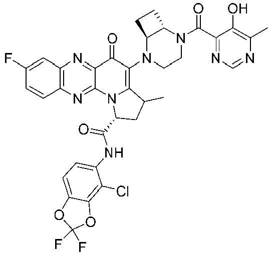 | **B100** | 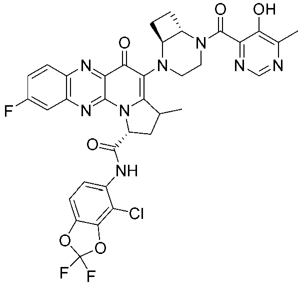 |
| **B101** | 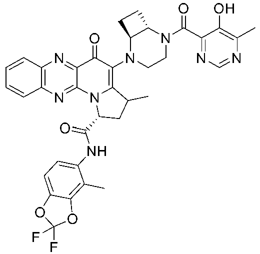 | **B102** | 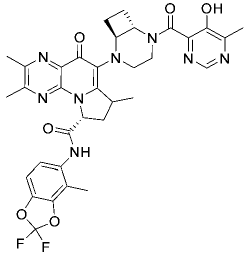 |
| **B103** | 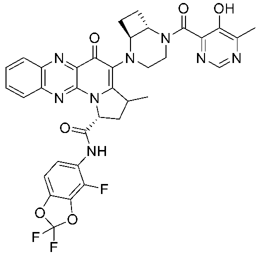 | **B104** | 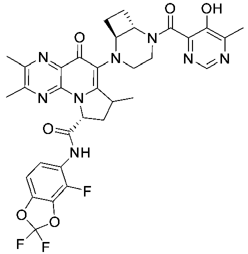 |
| **B105** | 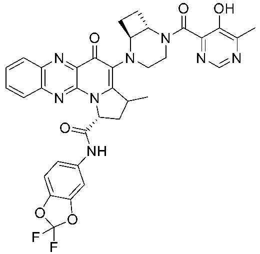 | **B106** | 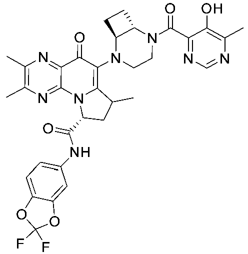 |
| **B107** | 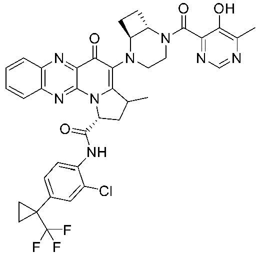 | **B108** | 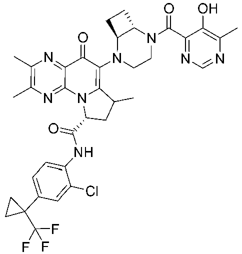 |
| **B109** | 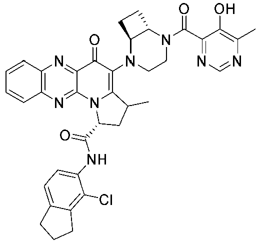 | **B110** | 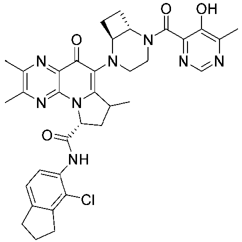 |
| **B111** | 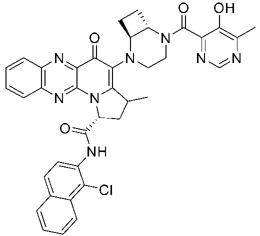 | **B112** | 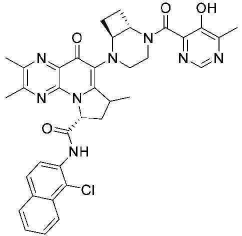 |
| **B113** | 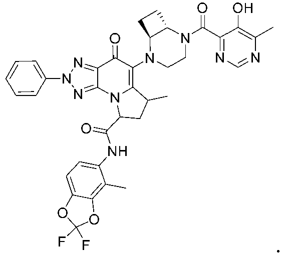 | | |

### Pharmaceutical Composition and Treatment Method

In some embodiments, the present disclosure provides a pharmaceutical composition comprising a prophylactically or therapeutically effective amount of the compound, or the pharmaceutically acceptable salt, the ester, the stereoisomer, the tautomer, the polymorph, the solvate, the metabolite, the isotopically labeled compound, or the prodrug thereof of the present disclosure, and one or more pharmaceutically acceptable carriers. The pharmaceutical composition is preferably a solid formulation, a semisolid formulation, a liquid formulation, or a gaseous formulation. In some embodiments, the pharmaceutical composition may further comprise one or more additional therapeutic agents.

In some embodiments, the present disclosure provides use of the compound, or the pharmaceutically acceptable salt, the ester, the stereoisomer, the tautomer, the polymorph, the solvate, the metabolite, the isotopically labeled compound, or the prodrug thereof of the present disclosure, or of the pharmaceutical composition of the present disclosure in preparing a medicament as a WRN inhibitor.

In some embodiments, the present disclosure provides the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure, for use as a WRN inhibitor.

In some embodiments, the present disclosure provides a method for preventing or treating a cancer (preferably a cancer characterized by microsatellite instability-high (MSI-H) or mismatch repair deficiency (dMMR)), comprising administering to a subject in need thereof an effective amount of the compound, or the pharmaceutically acceptable salt, the ester, the stereoisomer, the tautomer, the polymorph, the solvate, the metabolite, the isotopically labeled compound, or the prodrug thereof of the present disclosure, or the pharmaceutical composition of the present disclosure.

In some embodiments, the cancer comprises colorectal cancer, gastric cancer, endometrial cancer, uterine cancer, adrenocortical carcinoma, cervical cancer, esophageal cancer, breast cancer, renal cancer, prostate cancer, and ovarian cancer.

The term "pharmaceutically acceptable carrier" in the present disclosure refers to a diluent, an adjuvant, an excipient, or a vehicle administered together with a therapeutic agent, which is suitable, within the scope of sound medical judgment, for contact with the tissues of humans and/or other animals without undue toxicity, irritation, allergic response, or other problems or complications relative to a reasonable benefit/risk ratio.

Unless otherwise stated, the term "treat", "treating", or "treatment" as used herein refers to reversing, alleviating, or inhibiting the progression of a disorder or condition to which such term applies or one or more symptoms of such a disorder or condition, or preventing such a disorder or condition or one or more symptoms of such a disorder or condition.

As used herein, an "individual" includes a human or a non-human animal. Exemplary human individuals include human individuals suffering from a disease (e.g., a disease described herein), which are referred to as patients, or normal individuals. In the present disclosure, "non-human animals" include all vertebrates, e.g., non-mammals (e.g., birds, amphibians, and reptiles) and mammals, e.g., non-human primates, livestock, and/or domesticated animals (e.g., sheep, dogs, cats, cows, and pigs).

In another type of embodiment, the pharmaceutical composition of the present disclosure may further comprise one or more additional therapeutic or prophylactic agents.

### Examples

The present disclosure is further described below with reference to examples, but these provided examples are not intended to limit the scope of the present disclosure.

The abbreviations in the present disclosure have the following meanings:

| **Abbreviation** | **Full version** |
|---|---|
| ACN | Acetonitrile |
| AgBF₄ | Silver tetrafluoroborate |
| Boc | tert-Butyloxycarbonyl |
| CMPI | 2-Chloro-1-methylpyridinium iodide |
| DCM | Dichloromethane |
| DIEA/DIPEA | N,N-diisopropylethylamine |
| dioxane | 1,4-Dioxane |
| DMF | N,N-dimethylformamide |
| DMSO | Dimethyl sulfoxide |
| DPPA | Diphenylphosphoryl azide |
| EDCI | 1-Ethyl-(3-dimethylaminopropyl)carbodiimide |
| Et | Ethyl |
| Et₃N/NEt₃ | Triethylamine |
| EtOAc/EA | Ethyl acetate |
| EtOH | Ethanol |
| h/hrs | Hour |
| HATU | O-(7-azabenzotriazol-1-yl)-N,N,N',N'-tetramethyluronium hexafluorophosphate |
| HCl | Hydrochloric acid |
| HOAT | N-hydroxy-7-azabenzotriazole |
| HOBT | 1-Hydroxybenzotriazole |
| HPLC | High-performance liquid chromatography |
| KOBu^{t} | Potassium tert-butoxide |
| LCMS | Liquid chromatography-mass spectrometry |
| MeOH | Methanol |
| min | Minute |
| NaOH | Sodium hydroxide |
| NBS | N-bromosuccinimide |
| NCS | N-chlorosuccinimide |
| n-BuLi | n-Butyllithium |
| O.N | Overnight |
| PE | Petroleum ether |
| t-BuOH | tert-Butanol |
| t-BuOK | Potassium tert-butoxide |
| TEA | Triethylamine |
| TFA | Trifluoroacetic acid |
| THF | Tetrahydrofuran |
| TLC | Thin-layer chromatography |
| TEMPO | 2,2,6,6-Tetramethylpiperidin-1-oxyl |
| TOMAC | Methyltrioctylammonium chloride |
| r.t./rt | Room temperature |

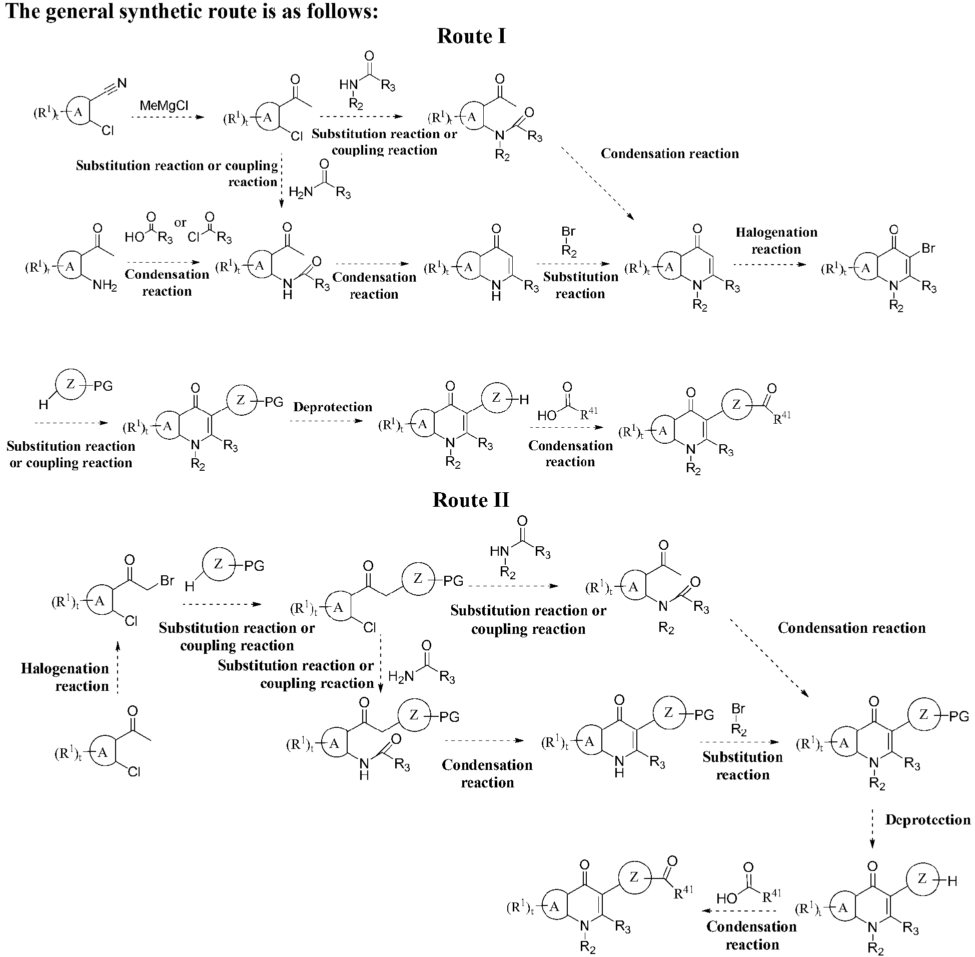

where in route I and route II, PG is a protecting group, and the other groups are as defined herein.

### Example 1: Synthesis of Compound B1

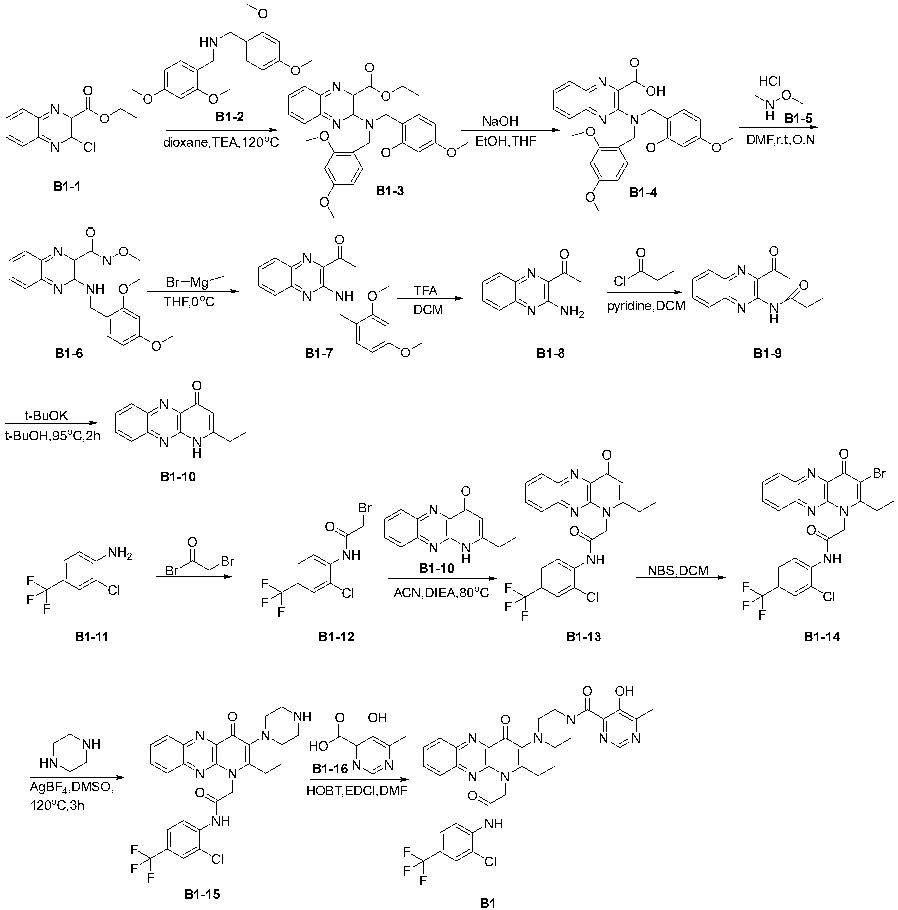

### 1) Synthesis of intermediate B1-3:

Compound **B1-1** (9.0 g, 38.03 mmol), compound **B1-2** (21.72 g, 68.45 mmol), and TEA (11.52 g, 114.09 mmol) were added to 1,4-dioxane (100 mL). The mixture was purged with nitrogen three times, then warmed to 120 °C, and stirred overnight under nitrogen atmosphere. After the starting materials were consumed completely as detected by TLC, the reaction liquid was concentrated under reduced pressure, and the residue was purified by column chromatography (PE/EA = 3/1) to obtain compound **B1-3** (15.0 g, yield: 76%) as a yellow solid. LCMS (ESI) m/z = 518.2 [M+H]⁺

### 2) Synthesis of intermediate B1-4:

Compound **B1-3** (12 g, 23.2 mmol) was added to a mixed solution of ethanol and tetrahydrofuran (2:1) (450 mL), and the mixture was cooled to 0 °C. NaOH (1.85 g, 46.4 mmol) was dissolved in 10 mL of water, and the resulting solution was slowly added dropwise to the reaction liquid. The mixture was stirred at room temperature overnight. After the starting materials were consumed completely as detected by TLC, HCl (1 M) was added dropwise to the reaction liquid to adjust the pH to 5, and the mixture was washed with water and extracted with ethyl acetate. The organic phase was then washed with saturated brine, dried over anhydrous sodium sulfate, and filtered, and the filtrate was concentrated under reduced pressure. Diethyl ether was added to the residue, and the resulting mixture was filtered to obtain compound **B1-4** (9.0 g, yield: 79%) as a yellow solid. LCMS (ESI) m/z = 490.3 [M+H]⁺

### 3) Synthesis of intermediate B1-6:

Compound **B1-4** (9.6 g, 19.6 mmol), compound **B1-5** (3.78 g, 39.26 mmol), TEA (7.92 g, 78.4 mmol), and HATU (8.94 g, 23.52 mmol) were added to DMF (100 mL). The mixture was purged with nitrogen three times and stirred overnight at room temperature under nitrogen atmosphere. After the starting materials were consumed completely as detected by TLC, the reaction mixture was washed with water and extracted with ethyl acetate. The organic phase was then washed with saturated brine, dried over anhydrous sodium sulfate, and filtered. The filtrate was concentrated under reduced pressure, and the residue was purified by column chromatography (PE/EA = 4/1) to obtain compound **B1-6** (4.5 g, yield: 60%) as a yellow solid. LCMS (ESI) m/z = 383.2 [M+H]⁺

### 4) Synthesis of intermediate B1-7:

Compound **B1-6** (5.0 g, 13.09 mmol) was added to a tetrahydrofuran solution (100 mL), and the mixture was purged with nitrogen three times and cooled to 0 °C. Methyl Grignard reagent (1 M, 52.4 mL, 52.4 mmol) was slowly added dropwise, and the resulting mixture was stirred at 0 °C for 1 h. After the starting materials were consumed completely as detected by LC-MS, an aqueous ammonium chloride solution was slowly added dropwise to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (PE/EA = 6/1) to obtain compound **B1-7** (3.6 g, yield: 81%) as a yellow solid. LCMS (ESI) m/z = 338.1 [M+H]⁺

### 5) Synthesis of intermediate B1-8:

Compound **B1-7** (3.6 g, 10.7 mmol) was added to dichloromethane (10 mL), and trifluoroacetic acid (10 mL) was then added. The mixture was warmed to 40 °C and stirred for 2 h. After the starting materials were consumed completely as detected by LC-MS, a saturated aqueous sodium bicarbonate solution was slowly added dropwise to the reaction liquid until the pH was equal to 7, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (PE/EA = 4/1) to obtain compound **B1-8** (1.7 g, yield: 85%) as a yellow solid. LCMS (ESI) m/z = 188.3 [M+H]⁺

### 6) Synthesis of intermediate B1-9:

Compound **B1-8** (1.0 g, 5.3 mmol) was added to dichloromethane (20 mL), and the mixture was cooled to 0 °C. Propionyl chloride (0.99 g, 10.6 mmol) and pyridine (2.09 g, 26.5 mmol) were slowly added dropwise, and the reaction liquid was stirred at 0 °C for 3 h. After the starting materials were consumed completely as detected by LC-MS, water was added to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (PE/EA = 3/1) to obtain compound **B1-9** (0.98 g, yield: 75%) as a yellow solid. LCMS (ESI) m/z = 244.1 [M+H]⁺

### 7) Synthesis of intermediate B1-10:

Compound **B1-9** (950 mg, 3.9 mmol) was added to tert-butanol (50 mL), and potassium tert-butoxide (2190 mg, 19.54 mmol) was slowly added. The reaction liquid was purged with nitrogen three times, heated to 95 °C, and stirred for 2 h under nitrogen atmosphere. After the starting materials were consumed completely as detected by LC-MS, the reaction liquid was cooled to room temperature, HCl (1 M) was added dropwise to the reaction liquid to adjust the pH to 7, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (DCM/MeOH = 50/1) to obtain compound **B1-10** (450 mg, yield: 51%) as a yellow solid. LCMS (ESI) m/z = 226.3 [M+H]⁺

### 8) Synthesis of intermediate B1-12:

Compound **B1-11** (3.0 g, 15.34 mmol) was dissolved in dichloromethane (20 mL), and triethylamine (3.10 g, 15.34 mmol) and bromoacetyl bromide (3.0 g, 15.34 mmol) were added at 0 °C. The mixture was stirred at room temperature for 5 h. After the starting materials were consumed completely as detected by LC-MS, an aqueous solution was slowly added dropwise to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (PE/EA = 50/1) to obtain compound **B1-12** (4.0 g, yield: 83%) as an off-white solid. LCMS (ESI) m/z = 315.9 [M+H]⁺.

### 9) Synthesis of intermediate B1-13:

Compound **B1-12** (950. mg, 3.02 mmol) and compound **B1-10** (678.6 mg, 3.02 mmol) were added to acetonitrile (10 mL), and DIEA (1168.7 mg, 9.06 mmol) was slowly added dropwise. The reaction liquid was purged with nitrogen three times, heated to 80 °C, and stirred overnight. After the starting materials were consumed completely as detected by LC-MS, the reaction liquid was concentrated under reduced pressure, and the residue was purified by column chromatography (PE/EA = 6/4) to obtain compound **B1-13** (850 mg, yield: 61.27%) as a white solid. LCMS (ESI) m/z = 461.0 [M+H]⁺

### 10) Synthesis of intermediate B1-14:

Compound **B1-13** (600 mg, 1.30 mmol) was added to dichloromethane (20 mL), and NBS (232.17 mg, 1.3 mmol) was added at 0 °C. The mixture was stirred at room temperature for 1 h. After the starting materials were consumed completely as detected by LC-MS, an aqueous solution was slowly added dropwise to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (PE/EA = 2/1) to obtain compound **B1-14** (550 mg, yield: 78%) as an off-white solid. LCMS (ESI) m/z = 538.9 [M+H]⁺.

### 11) Synthesis of intermediate B1-15:

Compound **B1-14** (180 mg, 0.33 mmol), piperazine (567.6 mg, 6.6 mmol), and silver tetrafluoroborate (130 mg, 0.66 mmol) were added to dimethyl sulfoxide (4 mL), and the reaction liquid was heated to 120 °C and allowed to react. After the starting materials were consumed completely as detected by LC-MS, an aqueous solution was added dropwise to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (DCM/MeOH = 10/1) to obtain compound **B1-15** (25 mg, yield: 14%) as an off-white solid. LCMS (ESI) m/z = 545.1 [M+H]⁺

### 12) Synthesis of product B1:

Compound **B1-16** (8.87 mg, 0.059 mmol), HOBT (5.94 mg, 0.044 mmol), and EDCI (11.52 mg, 0.06 mmol) were added to DMF (4 mL), and the mixture was purged with nitrogen three times and stirred for 2 h under nitrogen atmosphere. Compound **B1-15** (23 mg, 0.04 mmol) and DIEA (15.48 mg, 0.12 mmol) were added to the reaction liquid, and the mixture was then stirred at room temperature for another 2 h. After the starting materials were consumed completely as detected by LC-MS, an aqueous solution was added dropwise to quench the reaction, and the resulting mixture was extracted with dichloromethane. The organic phase was then washed with saturated brine, dried over anhydrous sodium sulfate, and filtered. The filtrate was concentrated under reduced pressure, and the residue was purified by preparative high-performance liquid chromatography to obtain compound **B1** (5.4 mg, yield: 19%). LCMS (ESI) m/z = 680.9 [M+H]⁺. ¹H NMR (400 MHz, CD₃OD): δ 8.53 (s, 1H), 8.32 (d, *J=* 7.6 Hz, 1H), 8.11-8.07 (m, 2H), 7.99-7.95 (m, 1H), 7.90-7.86 (m, 1H), 7.81 (s, 1H), 7.58 (dd, *J₁ =* 1.2 Hz, *J₂ =* 8.8 Hz, 1H), 5.70 (s, 2H), 4.74-4.70 (m, 1H), 4.07-3.91 (m, 3H), 3.52-3.40 (m, 3 H), 3.22-3.13 (m, 1 H), 2.99-2.96 (m, 1 H), 2.84-2.81 (m, 1 H), 2.53 (s, 3 H), 1.43 (t, *J* = 7.6 Hz, 3H).

### Example 2: Synthesis of Compound B6

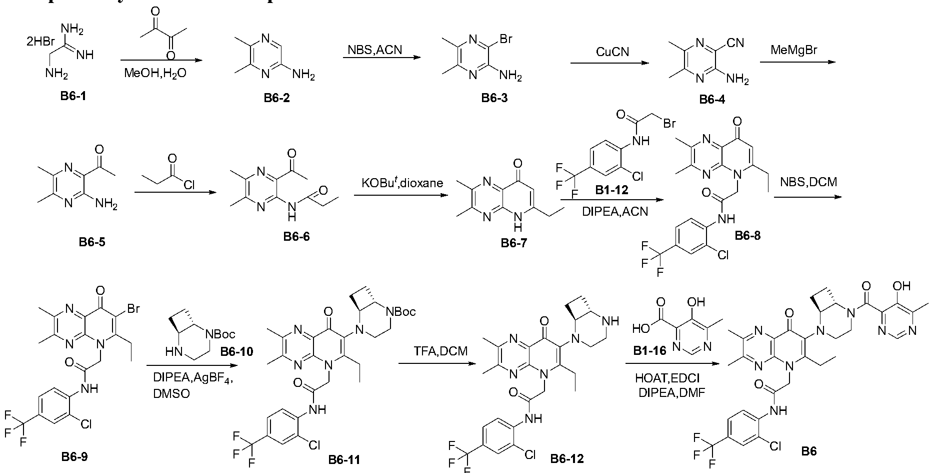

### 1) Synthesis of intermediate B6-2:

Compound **B6-1** (9 g, 38.31 mmol) was dissolved in methanol (100 mL), and the resulting solution was cooled to -30 °C and stirred for 10 min. Subsequently, an aqueous solution (100 mL) of 2,3-butanedione (3.3 g, 38.31 mmol) and sodium acetate (10.18 g, 122.59 mmol) was added, and after the dropwise addition was completed, a 40 mL (3.6 mol/L) sodium hydroxide solution was added. The mixture was then allowed to react at 0 °C for 30 min, and transferred to room temperature environment and allowed to react overnight. After the reaction was completed as detected by LCMS, the resulting mixture was extracted with water (200 mL) and ethyl acetate (200 mL × 3), and the organic phases were combined and then washed with saturated brine (20 mL × 3). The organic phase was dried over anhydrous sodium sulfate and concentrated to obtain a crude product of compound **B6-2** (3.85 g, yield: 81.6%). LCMS (ESI) m/z = 124.1 [M+H]⁺

### 2) Synthesis of intermediate B6-3:

Compound **B6-2** (3.8 g, 24.99 mmol) was dissolved in acetonitrile (40 mL), and the resulting solution was cooled to 0 °C. NBS (4.45 g, 24.99 mmol) was then added, and the reaction liquid was stirred at 25 °C for 0.5 h. After the reaction was completed as detected by LCMS, the resulting mixture was extracted with water (100 mL) and ethyl acetate (100 mL × 3), and the organic phases were combined and then washed with saturated brine (20 mL × 3). The organic phase was dried over anhydrous sodium sulfate, concentrated, and purified by column chromatography to obtain **B6-3** (3.9 g, yield: 77.23%). LCMS (ESI) m/z = 202.0 [M+H]⁺

### 3) Synthesis of intermediate B6-4:

Compound **B6-3** (3.9 g, 19.3 mmol) and cuprous cyanide (4.13 g, 46.13 mmol) were dissolved in DMF (40 mL), and the resulting solution was then allowed to react at 120 °C under microwave irradiation for 2 h. After the reaction was completed as detected by LCMS, the resulting mixture was extracted with water (100 mL) and ethyl acetate (100 mL × 3), and the organic phases were combined and then washed with saturated brine (20 mL × 3). The organic phase was dried over anhydrous sodium sulfate, concentrated, and purified by column chromatography to obtain **B6-4** (0.74 g, yield: 25.85%). LCMS (ESI) m/z = 149.2 [M+H]⁺

### 4) Synthesis of intermediate B6-5:

Compound **B6-4** (0.57 g, 3.85 mmol) was dissolved in tetrahydrofuran (6 mL), and the resulting solution was cooled to 0 °C. Subsequently, methylmagnesium bromide (3 M, 13 mL, 38.5 mmol) was added, and the mixture was then allowed to react at 50 °C overnight. After the reaction was completed as detected by LCMS, the resulting mixture was extracted with water (100 mL) and ethyl acetate (100 mL × 3), and the organic phases were combined and then washed with saturated brine (20 mL × 3). The organic phase was dried over anhydrous sodium sulfate, concentrated, and purified by column chromatography to obtain **B6-5** (0.5 g, yield: 78.68%). LCMS (ESI) m/z = 166.5 [M+H]⁺

### 5) Synthesis of intermediate B6-6:

Compound **B6-5** (0.7 g, 4.24 mmol) was added to dichloromethane (20 mL), and the mixture was cooled to 0 °C. Propionyl chloride (0.78 g, 8.48 mmol) and pyridine (1.68 g, 21.20 mmol) were slowly added dropwise, and the reaction liquid was stirred at 0 °C for 3 h. After the starting materials were consumed completely as detected by LC-MS, water was added to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography to obtain compound **B6-6** (0.6 g, yield: 63.99%) as a yellow solid. LCMS (ESI) m/z = 222.2 [M+H]⁺

### 6) Synthesis of intermediate B6-7:

Compound **B6-6** (0.35 g, 1.58 mmol) was added to 1,4-dioxane (5 mL), and potassium tert-butoxide (0.27 g, 2.37 mmol) was slowly added. The reaction liquid was purged with nitrogen three times, heated to 110 °C, and stirred for 1 h under nitrogen atmosphere. After the starting materials were consumed completely as detected by LC-MS, the reaction liquid was cooled to room temperature, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography to obtain compound **B6-7** (0.3 g, yield: 93.31 %) as a yellow solid. LCMS (ESI) m/z = 204.2 [M+H]⁺

### 7) Synthesis of intermediate B6-8:

Compound **B6-7** (0.28 g, 1.38 mmol) and compound **B1-12** (0.48 g, 1.52 mmol) were added to acetonitrile (3 mL), and DIPEA (0.89 g, 6.9 mmol) was slowly added dropwise. The reaction liquid was purged with nitrogen three times, heated to 45 °C, and stirred for 4 h. After the starting materials were consumed completely as detected by LC-MS, the reaction liquid was concentrated under reduced pressure, and the residue was purified by column chromatography to obtain compound **B6-8** (0.5 g, yield: 82.7%) as a white solid. LCMS (ESI) m/z = 439.2 [M+H]⁺

### 8) Synthesis of intermediate B6-9:

Compound **B6-8** (0.45 g, 1.03 mmol) was added to dichloromethane (10 mL), and NBS (0.32 g, 1.77 mmol) was added at 0 °C. The mixture was stirred at room temperature for 1 h. After the starting materials were consumed completely as detected by LC-MS, an aqueous solution was slowly added dropwise to quench the reaction, and the resulting mixture was extracted with ethyl acetate. The organic phase was then washed with saturated brine, dried over anhydrous sodium sulfate, and filtered. The filtrate was concentrated under reduced pressure. The residue was purified by column chromatography to obtain compound **B6-9** (0.2 g, yield: 37.67%) as an off-white solid. LCMS (ESI) m/z = 517.2 [M+H]⁺

### 9) Synthesis of intermediate B6-11:

Compound **B6-9** (0.15 g, 0.29 mmol), compound **B6-10** (0.45 g, 2.12 mmol), silver tetrafluoroborate (0.3 g, 1.54 mmol), and DIPEA (0.39 g, 2.9 mmol) were added to dimethyl sulfoxide (4 mL), and the reaction liquid was heated to 140 °C. After the starting materials were consumed completely as detected by LC-MS, an aqueous solution was added dropwise to quench the reaction, and the resulting mixture was extracted with ethyl acetate. The organic phase was then washed with saturated brine, dried over anhydrous sodium sulfate, and filtered. The filtrate was concentrated under reduced pressure, and the residue was purified by column chromatography (DCM/MeOH = 10/1) to obtain compound **B6-11** (20 mg, yield: 10.63%) as an off-white solid. LCMS (ESI) m/z = 649.2 [M+H]⁺

### 10) Synthesis of intermediate B6-12:

Compound **B6-11** (20 mg, 0.031 mmol) was dissolved in dichloromethane (0.5 mL), and trifluoroacetic acid (0.5 mL) was added dropwise. The reaction liquid was stirred at 25 °C for 1.5 h. After the reaction was completed as detected by LCMS, the reaction liquid was concentrated to obtain a trifluoroacetate of **B6-12** (60 mg, crude product) as a yellow oil. LCMS (ESI) m/z = 549.2 [M+H]⁺

### 11) Synthesis of product B6:

Compound **B1-16** (7.2 mg, 0.059 mmol), HOAT (6.7 mg, 0.046 mmol), and EDCI (11.52 mg, 0.056 mmol) were added to DMF (0.5 mL), and the mixture was purged with nitrogen three times and stirred for 1 h under nitrogen atmosphere. The trifluoroacetate of compound **B6-12** (60 mg, crude product) and DIPEA (12 mg, 0.093 mmol) were added to the reaction liquid, and the reaction liquid was then stirred at room temperature for another 0.5 h. After the starting materials were consumed completely as detected by LC-MS, the resulting mixture was purified by preparative reversed-phase C18 column chromatography (0.5% aqueous formic acid solution:acetonitrile = 9:1 to 1:9) to obtain compound **B6** (3.5 mg, two-step yield: 16.5%). LCMS (ESI) m/z = 685.2 [M+H]⁺. ¹H NMR (400 MHz, DMSO-d₆) δ 10.37 (s, 1H), 8.50 (s, 1H), 8.01 - 7.94 (m, 2H), 7.69 (dd, J = 8.8, 2.1 Hz, 1H), 5.44 (s, 2H), 4.48 (d, J = 13.0 Hz, 1H), 3.88 (d, J = 8.9 Hz, 1H), 3.60 - 3.11(m, 6H), 2.60 (s, 3H), 2.59 (s, 3H), 2.41 (s, 3H), 1.99 (dt, J = 13.4, 7.0 Hz, 1H), 1.65-1.45 (m, 2H), 1.34 - 1.27 (m, 3H), 1.06 (t, J = 8.2 Hz, 1H).

### Example 3: Synthesis of Compound B18

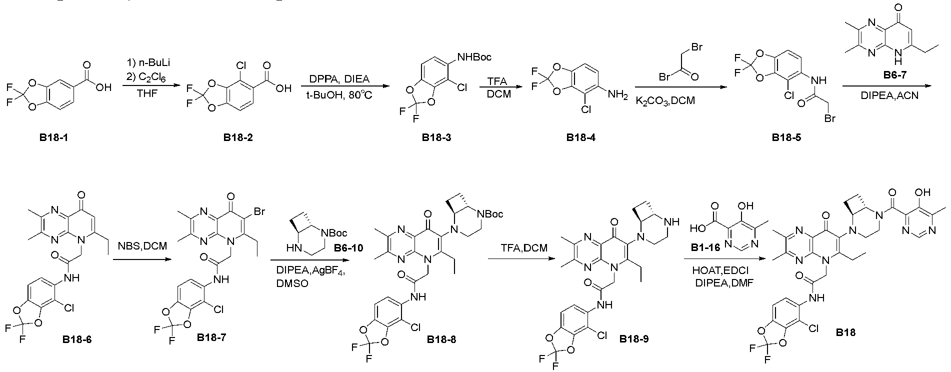

### 1) Synthesis of intermediate B18-2:

**B18-1** (9.0 g, 44.53 mmol) and anhydrous tetrahydrofuran (225 mL) were sequentially added to a 1000 mL three-necked flask, and the mixture was stirred at -70 °C to -75 °C under nitrogen atmosphere. A solution of n-butyllithium in n-hexane (2.5 mol/L, 48 mL, 120 mmol) was slowly added, and the mixture was stirred at -70 °C to -75 °C for 20 min, then slowly warmed to -5 °C to 0 °C, stirred at -5 °C to 0 °C for 70 min, and cooled to -70 °C to -75 °C. Hexachloroethane (31.6 g, 133.6 mmol) was then added at -70 °C to -75 °C, and the mixture was stirred at -70 °C to -75 °C for 40 min, then slowly warmed to room temperature, and stirred for another 40 min. The reaction was quenched with a 10% w/w aqueous citric acid solution, and the resulting mixture was extracted twice with ethyl acetate. The organic phases were combined, dried over anhydrous sodium sulfate, concentrated under reduced pressure, and subjected to column chromatography to obtain **B18-2** (3.5 g, 33%).

### 2) Synthesis of intermediate B18-3:

To a solution of **B18-2** (3 g, 12.68 mmol) in anhydrous tert-butanol (30 mL) was added diphenylphosphoryl azide (4.08 g, 14.84 mmol). After the addition, N,N-diisopropylethylamine (1.92 g, 19.02 mmol) was added to the reaction liquid. The reaction liquid was warmed to 80 °C and stirred overnight. The reaction liquid was concentrated under reduced pressure, and a saturated sodium bicarbonate solution was added to the residue. The resulting mixture was extracted with ethyl acetate (3 × 50 mL). The organic phase was washed with brine (30 mL), dried over anhydrous sodium sulfate, filtered, and concentrated under reduced pressure to obtain a crude product. The crude product was purified by column chromatography to obtain compound **B18-3** (2.8 g, 71%) as a white solid.

### 3) Synthesis of intermediate B18-4:

**B18-3** (450 mg, 1.46 mmol) was dissolved in a dichloromethane (3 mL) solution, and trifluoroacetic acid (1 mL) was added thereto. The reaction liquid was stirred at 25 °C for 2 h. After the reaction was completed as detected by LCMS, the reaction liquid was concentrated to obtain **B18-4** (300 mg, mixture, yield: 98.8%) as a brown oil. ESI m/z [M+H]⁺ = 208.1

### 4) Synthesis of intermediate B18-5:

Compound **B18-4** (0.3 g, 1.45 mmol) and potassium carbonate (0.6 g, 4.35 mmol) were dissolved in a dichloromethane (2 mL) solution, and bromoacetyl bromide (0.44 g, 2.17 mmol) was added dropwise under stirring at 0 °C. The mixture was stirred at 25 °C for 1 h. After the reaction was completed as detected by LCMS, the reaction liquid was poured into water, extracted with dichloromethane (50 mL × 2), and concentrated to obtain **B18-5** (0.3 g, 63%) as a yellow solid.

### 5) Synthesis of product B18:

Referring to the synthetic method for compound **B6** in Example 2, compound **B18** was obtained by replacing **B1-12** with compound **B18-5.** LCMS (ESI) m/z = 697.2 [M+H]⁺. ¹H NMR (400 MHz, DMSO-d₆) δ 10.37 (s, 1H), 8.57 (s, 1H), 7.41 (m, 2H), 5.39 (s, 2H), 4.57 - 4.41 (m, 1H), 3.91 (d, *J =* 8.6 Hz, 1H), 3.75 - 3.06 (m, 6H), 2.64 (s, 3H), 2.63 (s, 3H), 2.46 (s, 3H), 1.62-1.50 (m, 1H), 1.40-1.28 (m, 2H), 1.28 - 1.22 (m, 3H), 1.20-1.14 (m, 1H).

### Example 4: Synthesis of Compound B113

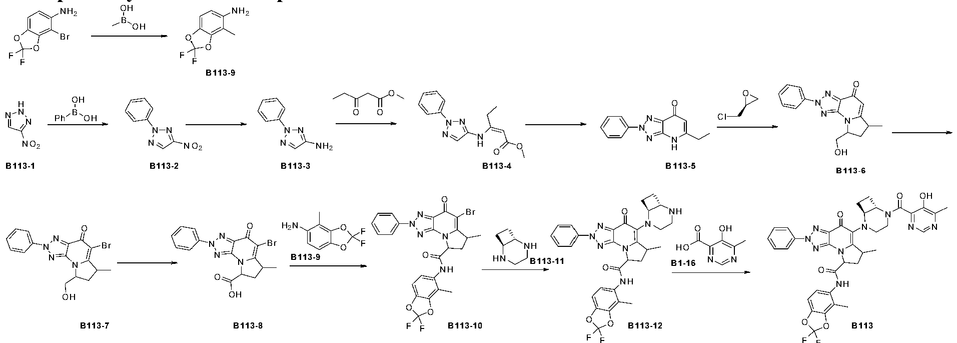

### 1) Synthesis of intermediate B113-9:

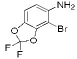

(0.6 g, 2.38 mmol, prepared according to the synthetic method disclosed in the patent application WO2008082484) and methylboronic acid (1.42 g, 23.8 mmol) were dissolved in a mixed solution of 1,4-dioxane (5 mL) and water (0.5 mL), and potassium carbonate (0.99 g, 7.14 mmol) was added under stirring. After the mixture was purged with a gas three times, [1,1'-bis(diphenylphosphino)ferrocene]palladium(II) dichloride (0.17 g, 0.24 mmol) was added, and the mixture was stirred at 80 °C for 16 h. After the reaction was completed as detected by LCMS, the reaction liquid was concentrated and purified by column chromatography (ethyl acetate/petroleum ether) to obtain **B113-9** (0.4 g, yield: 89.8%) as a white solid. m/z [M+H]⁺ = 188.1

### 2) Synthesis of intermediate B113-2:

4-Nitro-2H-1,2,3-triazole (1.0 g, 8.76 mmol, 1.0 eq), phenylboronic acid (2.14 g, 17.50 mmol, 2.0 eq.), and cupric acetate monohydrate (524 mg, 2.63 mmol, 0.3 eq.) were dissolved in DMSO (15 mL). The reaction liquid was purged with oxygen 3 times and stirred at 110 °C overnight. After the reaction was completed, the reaction liquid was diluted with water (150 mL) and filtered, and the filtrate was extracted with ethyl acetate (3 × 60 mL). The organic phases were combined, then washed with saturated brine, dried over anhydrous sodium sulfate, and concentrated. The crude product was separated and purified by silica gel column chromatography (eluent: ethyl acetate:petroleum ether = 1:10) to obtain **B113-2** (210 mg, 12%). LCMS: m/z = 191.1 [M+H]⁺; ¹H NMR (400 MHz, CDCl₃) δ 8.37 (s, 1H), 8.17 - 8.12 (m, 2H), 7.57 - 7.48 (m, 3H).

### 3) Synthesis of intermediate B113-3:

**B113-2** (9.0 g, 47.33 mmol, 1.0 eq), ammonium chloride (7.6 g, 141.98 mmol, 3.0 eq), and iron powder (7.9 g, 141.98 mmol, 3.0 eq) were mixed in ethanol (90 mL) and water (90 mL). The reaction liquid was stirred at 70 °C under nitrogen atmosphere for 2 h. After the reaction was completed, the reaction liquid was filtered through celite, and the filtrate was extracted with ethyl acetate (200 mL × 3). The organic phases were combined, then washed with saturated brine (200 mL), dried over anhydrous sodium sulfate, and filtered. The filtrate was concentrated under reduced pressure to obtain **B113-3** (8.5 g, 95%) as a yellow solid. LCMS *m*/*z =* 161.1 [M+H]⁺. ¹H NMR (400 MHz, DMSO-*d₆*) δ 7.82 - 7.78 (m, 2H), 7.48 - 7.44 (m, 2H), 7.26 (s, 1H), 6.76 - 6.73 (m, 1H), 5.50 (s, 2H).

### 4) Synthesis of intermediate B113-4:

**B113-3** (2.0 g, 9.79 mmol, 1.0 eq), methyl 3-oxopentanoate (10.2 g, 78.34 mmol, 8.0 eq), and anhydrous magnesium sulfate (2 g) were dissolved in toluene (10 mL). The reaction liquid was stirred at 110 °C overnight. After the reaction was completed, the reaction liquid was diluted with dichloromethane (50 mL) and filtered through celite, and the filtrate was concentrated under reduced pressure to obtain a crude product. The crude product was separated and purified by silica gel column chromatography (eluent: ethyl acetate:petroleum ether = 1:2) to obtain **B113-4** (2.9 g, yield: 93%). LCMS m/z = 273.2 [M+H]⁺

### 5) Synthesis of intermediate B113-5:

The solid **B113-4** (310 mg, 1.1 mmol, 1.0 eq) was directly poured into diphenyl ether (10 mL) at 235 °C. The reaction liquid was stirred at 235 °C for 1 h. After the reaction was completed, the reaction liquid was cooled to room temperature, diluted with petroleum ether (20 mL), and stirred for 20 min. The reaction mixture was filtered and washed with petroleum ether, and the filter cake was dried under reduced pressure to obtain a crude product. The crude product was separated and purified by silica gel column chromatography (eluent: dichloromethane:methanol = 40:1) to obtain **B113-5** (80 mg, yield: 30%). LCMS m/z = 241.1 [M+H]⁺. ¹H NMR (400 MHz, DMSO-d₆) δ 12.40 (s, 1H), 8.14 (d, *J =* 8.0 Hz, 2H), 7.64 (t, *J =* 8.0 Hz, 2H), 7.58 - 7.49 (m, 1H), 5.93 (s, 1H), 2.63 (q, *J =* 7.6 Hz, 2H), 1.25 (t, *J =* 7.6 Hz, 3H).

### 6) Synthesis of intermediate B113-6:

Under nitrogen atmosphere, diisopropylamine (303 mg, 3 mmol, 1.0 eq.) was dissolved in anhydrous tetrahydrofuran (1.3 mL), and the resulting solution was cooled to -78 °C. n-Butyllithium (2.5 M in hexane, 1.2 mL, 3 mmol, 1.0 eq) was slowly added dropwise to the reaction system, and the reaction liquid was allowed to react at -78 °C for 30 min.

Under nitrogen atmosphere, **B113-5** (29 mg, 0.12 mmol, 1.0 eq) was dissolved in anhydrous tetrahydrofuran (2 mL) and anhydrous DMSO (0.2 mL), and the reaction liquid was cooled to 0 °C. The above prepared LDA solution (0.6 mL, 0.6 mmol, 5.0 eq) was slowly added to the reaction liquid, and the reaction liquid was allowed to react at 0 °C for 30 min. (S)-chloroepoxypropane (22 mg, 0.24 mmol, 2.0 eq) was dissolved in anhydrous tetrahydrofuran (0.4 mL), and the resulting solution was slowly added dropwise to the reaction liquid. The mixture was stirred at room temperature overnight. After the reaction was completed, the reaction liquid was quenched with saturated ammonium chloride (10 mL) and extracted with ethyl acetate (3 × 5 mL). The organic phases were combined, then washed with saturated brine, dried over anhydrous sodium sulfate, and concentrated. The crude product was separated and purified by preparative thin-layer chromatography (eluent: dichloromethane:methanol = 10:1) to obtain **B113-6** (10 mg, 28%) as a pale yellow solid. LCMS m/z = 297.1 [M+H]⁺

### 7) Synthesis of intermediate B113-7:

Under nitrogen atmosphere, **B113-6** (298 mg, 1.0 mmol, 1.0 eq) was dissolved in anhydrous dichloromethane (12 mL), and the reaction liquid was cooled to 0 °C. N-bromosuccinimide (268 mg, 1.5 mmol, 1.5 eq) was slowly added to the reaction liquid. The reaction liquid was slowly warmed to room temperature and stirred at room temperature overnight. After the reaction was completed, the reaction liquid was quenched with water (20 mL) and extracted with dichloromethane (3 × 10 mL). The organic phases were combined, then washed with saturated brine, dried over anhydrous sodium sulfate, and concentrated under reduced pressure to obtain **B113-7** (375 mg, 100% crude product) as a red solid. LCMS m/z = 375.1 [M+H]⁺

### 8) Synthesis of intermediate B113-8:

**B113-7** (375 mg, 1.0 mmol, 1.0 eq), sodium bicarbonate (840 mg, 10 mmol, 10.0 eq), potassium bromide (12 mg, 0.1 mmol, 0.1 eq), TOMAC (21 mg, 0.05 mmol, 0.05 eq), and TEMPO (8 mg, 0.05 mmol, 0.05 eq) were dissolved in dichloromethane (12 mL) and water (9 mL), and the reaction liquid was cooled to 0 °C. A 5% aqueous sodium hypochlorite solution (7.5 g, 5.0 mmol, 5.0 eq) was slowly added dropwise to the reaction liquid. The reaction liquid was slowly warmed to room temperature and stirred at room temperature for 2.5 h. After the reaction was completed, a sodium bicarbonate solution was added to the reaction liquid to quench the reaction, and the resulting mixture was extracted with dichloromethane (3 × 20 mL). The resulting aqueous phase was adjusted to pH = 3 with a 3 M hydrochloric acid solution and then extracted with ethyl acetate (3 × 20 mL). The organic phases were combined, then washed with saturated brine, dried over anhydrous sodium sulfate, and concentrated under reduced pressure to obtain **B113-8** (120 mg, 31%) as a pale yellow solid. LCMS m/z = 389.1 [M+H]⁺

### 9) Synthesis of intermediate B113-10:

Under nitrogen atmosphere, compounds **B113-8** (120 mg, 0.31 mmol, 1.0 eq), **B113-9** (70 mg, 0.37 mmol, 1.2 eq), and N,N-diisopropylethylamine (160 mg, 1.23 mmol, 4.0 eq) were dissolved in anhydrous dichloromethane (2.5 mL), and CMPI (119 mg, 0.46 mmol, 1.5 eq) was then added. The reaction liquid was stirred at room temperature for 1.5 h. After the reaction was completed, water (20 mL) was added to the reaction liquid to quench the reaction, and the resulting mixture was extracted with ethyl acetate (3 × 20 mL). The organic phases were combined, then washed with saturated brine, dried over anhydrous sodium sulfate, and concentrated under reduced pressure. The crude product was separated and purified by silica gel column chromatography (eluent: dichloromethane:methanol = 150:1) to obtain **B113-10** (85 mg, yield: 46%) as a pale yellow solid. LCMS m/z = 558.1 [M+H]⁺. ¹H NMR (400 MHz, CDCl₃) δ 10.09 (s, 1H), 8.12 - 8.03 (m, 1H), 7.86 - 7.77 (m, 1H), 7.52 - 7.41 (m, 1H), 7.38 - 7.28 (m, 3H), 6.88 (d, *J =* 8.5 Hz, 1H), 5.43 - 5.32 (m, 1H), 3.31 - 3.09 (m, 1H), 2.79 - 2.63 (m, 1H), 2.41 - 2.36 (m, 3H), 2.10 - 1.90 (m, 1H), 1.41 (d, *J = 7.1* Hz, 3H).

### 10) Synthesis of intermediate B113-12:

Under nitrogen atmosphere, **B113-10** (53 mg, 0.09 mmol, 1.0 eq), **B113-11** (160 mg, 1.42 mmol, 15.0 eq), N,N-diisopropylethylamine (48 mg, 0.47 mmol, 5.0 eq), and silver tetrafluoroborate (28 mg, 0.14 mmol, 1.5 eq) were dissolved in dry DMSO (0.8 mL), and the reaction liquid was stirred at 125 °C for 3.5 h. After the reaction was completed, the reaction liquid was diluted with methanol (2 mL) and filtered, and the filtrate was directly separated and purified by C18 column chromatography (H₂O containing 10-60% ACN (0.1% formic acid)) to obtain **B113-12** (30 mg, crude product, 50% purity) as a yellow solid. LCMS m/z = 590.30 [M+H]⁺.

### 11) Synthesis of product B113:

Under nitrogen atmosphere, **B1-16** (9.8 mg, 0.063 mmol) and HOAt (8.8 mg, 0.065 mmol) were dissolved in acetonitrile (2 mL), and EDCI (16.1 mg, 0.084 mmol) was added to the reaction liquid. The mixture was stirred at room temperature for 1 h. Subsequently, **B113-12** (25 mg, crude product, 50% purity) and N,N-diisopropylethylamine (18.9 mg, 0.14 mmol, 3.5 eq) were added to the reaction liquid, and the reaction liquid was stirred at room temperature for another 0.5 h. After the reaction was completed, water (20 mL) was added to the reaction liquid to quench the reaction, and the resulting mixture was extracted with ethyl acetate (3 × 15 mL). The organic phases were combined, then washed with saturated brine, dried over anhydrous sodium sulfate, and concentrated under reduced pressure. The crude product was separated and purified by preparative thin-layer chromatography (eluent: dichloromethane:methanol = 10:1) to obtain **B113** (1 mg). LCMS m/z = 726.2 [M+H]⁺. ¹H NMR (400 MHz, CD₃OD) δ 8.56 - 8.52 (m, 1H), 8.28 - 8.15 (m, 2H), 7.63 - 7.57 (m, 2H), 7.55 - 7.49 (m, 1H), 7.13 - 6.96 (m, 2H), 5.50 - 5.42 (m, 1H), 4.79 - 4.66(m, 1H), 4.00 - 3.88 (m, 3H), 3.78 - 3.69 (m, 2H), 3.57 - 3.48 (m, 1H), 3.18 - 3.13 (m, 1H), 2.65 - 2.54 (m, 1H), 2.52 - 2.50 (m, 3H), 2.31 - 2.22 (m, 4H), 1.82 - 1.67 (m, 1H), 1.61 (d, J = 7.3 Hz, 3H), 1.55 - 1.44 (m, 2H).

### Biological Assay Section

### Experimental Example 1: Biological Activity Assay

### ATPase activity assay of WRN helicase

The ADP content generated by ATP hydrolysis catalyzed by WRN helicase was measured using a commercial ADP-Glo assay kit (Promega, #V9102), and the measured ADP level could reflect the ATPase activity of WRN helicase.

The 45 nt oligonucleotide single-stranded DNA FLAP26 (TTTTTTTTTTTTTTTTTTTTTTCCAAGTAAAACGACGGCCAGTGC) was synthesized by Azenta. Reference was made, for example, to Brosh RM Jr et al., J Biol Chem, 2002 Jun;277(26):23236-45. A reaction buffer (30 mM Tris pH 7.5, 2 mM MgCl₂, 0.02% BSA, 50 mM NaCl, 0.1% pluronic F127) was prepared. To a 384-well clear plate were sequentially added 5 µL of 3× test compound (which was diluted in the reaction buffer to contain 0.5% DMSO, with a final initial concentration of 10 µM and subjected to 1:3 serial dilution to obtain a total of 9 concentration gradients), 5 µL of 3× WRN recombinant protein, and 3× ATP substrate solution (diluted in the reaction buffer, with final concentrations of WRN and ATP being 10 nM and 300 µM, respectively). The resulting mixture was mixed well by shaking and incubated at 37 °C for 3 h. Subsequently, a 3× FLAP26 solution was prepared using the reaction buffer, and 5 µL of the solution (with a final FLAP26 concentration of 0.4 nM) was added to the 384-well clear plate. The mixture was mixed well by shaking to initiate the enzymatic reaction, and then incubated at room temperature for 30 min.

5 µL of the above mixture was transferred to a 384-well white plate, and 5 µL ofADP-Glo reagent was added. The mixture was mixed well by shaking, and incubated at room temperature in the dark for 40 min. 10 µL of kinase detection reagent was added to the above solution. The mixture was mixed well by shaking and incubated at room temperature in the dark for 30 min, and then chemiluminescence readings were recorded. The inhibition rate of the compound on the enzyme activity was calculated, and the inhibition rate value and the logarithm value of the compound concentration were fitted using non-linear regression (dose response-variable slope) to obtain the IC₅₀ value of the test compound.

**Table 1. WRN ATPase activity data of compounds in the examples of the present application**

| **Compound** | **WRN ATPase activity inhibition IC₅₀ (µM)** |
|---|---|
| B1 | 0.009 |
| B6 | 0.009 |
| B18 | 0.009 |
| B113 | 0.005 |

As can be seen from Table 1, the compounds of the present application exhibited good inhibitory activity against the ATPase activity of WRN protein.

### Inhibitory activity assay for tumor cell proliferation

A DLD1-WRN-KO cell line was constructed by stably knocking out the WRN gene in DLD1 cells using CRISPR/Cas9 technology, and the constructed cell line was used to evaluate potential off-target effects of the compounds.

After treatment of SW48 cell lines with microsatellite instability and the control DLD1-WRN-KO cell line using the test compounds for 4 days, ATP levels were measured using a CellCounting-Lite kit from Vazyme to evaluate inhibitory effects of the test compounds on the growth of tumor cell lines.

In the present application, the SW48 cell line and the DLD1-WRN-KO cell line were seeded into a 96-well cell culture plate at an appropriate cell density. After 24 h, the cells were treated with the test compounds at the highest concentration of 10 µM, followed by 1:3 serial dilution to obtain 9 concentration gradients, and a DMSO treatment group was set up separately. The cells were cultured in an incubator at 37 °C with 5% CO₂ for 4 days. To evaluate inhibitory effects of the test compounds on tumor cell proliferation, the cells were equilibrated at room temperature for 30 min, and then 100 µL of a cell proliferation detection reagent, CellCounting-Lite (CCL), was added to each well. The plate was shaken for 5 min and then incubated in the dark for 10 min. Chemiluminescence values were read using a Thermo Varioskan LUX-3020 multifunctional microplate reader for conversion to proliferation indices to calculate the inhibition rate of the compound on tumor cell proliferation, and the inhibition rate value and the logarithm value of the compound concentration were fitted using non-linear regression (dose response-variable slope) to obtain the IC₅₀ value of the compound.

**Table 2. Inhibitory activity data of compounds in the examples of the present application against tumor cell proliferation**

| **Compound** | **SW48 Proliferation inhibition IC₅₀ (µM)** | **DLD1-WRN-KO Proliferation inhibition IC₅₀ (µM)** |
|---|---|---|
| B1 | 0.014 | >10 |
| B6 | 0.013 | >10 |
| B18 | 0.013 | >10 |
| B113 | 0.010 | >10 |

As can be seen from Table 2, the compounds of the present application exhibited good proliferation inhibition activity on SW48 cells with microsatellite instability, while no significant proliferation inhibition activity was observed against WRN knockout DLD1 cells, indicating good selectivity.

### Experimental Example 2: Stability Assay in Liver Microsomes

Phase I metabolic stability of the test compounds in liver microsomes of CD-1 mice, Sprague-Dawley rats, beagle dogs, cynomolgus monkeys, and humans was assessed.

### Experimental system:

The animal and human liver microsomes used in the assay system were purchased from Xenotech, Corning, or other qualified suppliers and stored in a freezer at a temperature below -60 °C prior to use.

### Brief introduction of the experiment:

The test samples and control compounds were separately incubated with animal and human liver microsomes at 37+1 °C for a period of incubation time up to 60 min. The samples were removed at the indicated time points, and the reaction was stopped with acetonitrile or another organic solvent containing an internal standard. After centrifugation, the resulting supernatants were assayed by the liquid chromatography-tandem mass spectrometry (LC-MS/MS) method.

### Experimental method:

### 1. Preparation of buffer

73.21 g of dipotassium hydrogen phosphate trihydrate and 10.78 g of potassium dihydrogen phosphate were dissolved in 4000 mL of ultrapure water. The solution was adjusted to pH 7.40+0.10 with 10% phosphoric acid or 1 M potassium hydroxide to make a final concentration of 100 mM.

### 2. Preparation of working solutions

The test sample powder was prepared into a stock solution at a certain concentration with DMSO or another organic solvent, which was then further diluted with a suitable organic solvent.

The reference compounds testosterone, diclofenac, and propafenone were each prepared into a 10 mM stock solution with DMSO, which was then further diluted with a suitable organic solvent.

### 3. Preparation of liver microsome solution

Microsomes of each species were diluted into a 2× working solution with a 100 mM potassium phosphate buffer. The final concentration of the microsomes in the reaction system was 0.5 mg/mL.

### 4. Preparation of reduced nicotinamide adenine dinucleotide phosphate (NADPH) regeneration system

An appropriate amount of nicotinamide adenine dinucleotide phosphate (NADP) and isocitric acid (ISO) powder was weighed out, dissolved in a magnesium chloride solution, and mixed well by shaking. An appropriate amount of isocitrate dehydrogenase (IDH) was added, and the mixture was mixed well by gently turning upside down. The final concentrations in the reaction system were: 1 mM NADP, 1 mM magnesium chloride, 6 mM ISO, and 1 unit/mL IDH.

### 5. Preparation of stop solution

The stop solution was prepared with acetonitrile or another organic solvent containing an internal standard (tolbutamide or another suitable compound). The prepared stop solution was stored in a freezer at 2-8 °C.

### 6. Incubation process

Incubation was performed in a 96-well plate. Eight incubation plates were prepared and designated T0, T5, T15, T30, T45, T60, Blank60, and NCF60, respectively. The first 6 plates corresponded to reaction time points of 0 min, 5 min, 15 min, 30 min, 45 min, and 60 min, respectively. No test sample or reference compound was added to the Blank60 plate, and samples were taken after 60 min of incubation. In the NCF60 plate, incubation was performed for 60 min using the potassium phosphate buffer instead of the NADPH regeneration system solution. Three replicates were made for samples in all conditions.

The microsomes and the test sample or reference compound were mixed, and then the incubation plates Blank60, T5, T15, T30, T45, and T60 except for T0 and NCF60 were placed in a 37 °C water bath for pre-incubation for about 10 min. In the incubation plate T0, a stop solution was first added, followed by the addition of the NADPH regeneration system working solution, and 98 µL of the potassium phosphate buffer was added to each sample well of the incubation plate NCF60 to initiate the reaction. After the pre-incubation of the incubation plates Blank60, T5, T15, T30, T45, and T60 was completed, 98 µL of the NADPH regeneration system working solution was added to each sample well to initiate the reaction. The reaction temperature was 37±1 °C, the final volume of the reaction was 200 µL, and the reaction system included 0.5 mg/mL microsomes, 1.0 µM substrate, 1 mM NADP, 6 mM ISO, and 1 unit/mL IDH.

At 5 min, 15 min, 30 min, 45 min, and 60 min, a cold stop solution containing an internal standard was added to the reaction plate to stop the reaction.

All the reaction plates after the reaction was stopped were shaken well and centrifuged at 3220× g at 4 °C for 20 min. After the supernatant was diluted at a certain ratio, LC-MS/MS analysis was performed.

### Sample analysis

Sample analysis was performed by the liquid chromatography-tandem mass spectrometry (LC-MS/MS) method without standard curves and quality control samples. Semi-quantitative determination was performed according to the ratio of the analyte peak area to the internal standard peak area. The retention times of the analyte and internal standard, the chromatogram acquisition, and chromatogram integration were processed using the Analyst software (Sciex, Framingham, Massachusetts, USA).

The CV for the internal standard peak area in each matrix in each analysis batch should be within 20%.

### Data analysis

The *in vitro* elimination rate constant ke of the compound was obtained by converting the ratio of the peak area of the compound to the internal standard peak area in the following formula into the remaining rate: $Remaining rate \left(%\right) = \frac{Peak area ratio of compound to internal standard at any time point}{Peak area ratio of compound to internal standard at 0 min} \times 100$ ${C}_{t} = {C}_{0} \cdot {e}^{- {k}_{e} \cdot t}$ *when* ${C}_{t} = \frac{1}{2} {C}_{0} ,$ ${T}_{1 / 2} = \frac{Ln 2}{{k}_{e}} = \frac{0.693}{{k}_{e}}$
CL_{int (mic)} = 0.693/T_{1/2}/microsome protein content (microsome concentration during incubation in mg/mL)
CL_{int (liver)} = CL_{int (mic)} × microsome protein amount in liver (mg/g) × liver-to-body weight ratio

According to the well stir model, the hepatic intrinsic clearance and hepatic clearance can be converted through the following formula. ${CL}_{\left(liver\right)} = \left({CL}_{int \left(liver\right)}\times {Q}_{h}\right) / \left({CL}_{int \left(liver\right)}\times {Q}_{h}\right)$

Parameters in the formula are shown in the table below.

Parameters in the data analysis formula

| **Species** | **Liver-to-body weight ratio (g/kg body weight)** | **Liver blood flow volume (Qₕ) (mL/min/kg)** | **Microsome protein amount (mg/g liver weight)** |
|---|---|---|---|
| Mouse | 88 | 90.0 | |
| Rat | 40 | 55.2 | |
| Dog | 32 | 30.9 | 45 |
| Monkey | 30 | 43.6 | |
| Human | 20 | 20.7 | |

### Experimental Example 3: Metabolic Stability Assay in Hepatocytes

This experimental example was used to assay the metabolic stability of the compounds in hepatocytes.

A suspension of hepatocytes was prepared at 0.5 × 10⁶/mL with a pre-heated medium, then 198 µL of the pre-heated cell suspension was added to a 96-well plate. To each well of the 96-well plate, 2 µL of the test compound was added so that the final concentration was 1 µM, and 2 replicate wells were set. For the T = 0 min sample, the compound was mixed well with the cells for 1 min, then 25 µL of the sample was immediately added to 125 µL of a stop solution (an acetonitrile solution containing 200 ng/mL tolbutamide and 200 ng/mL labetalol) in an ice bath, and the mixture was mixed well. At the same time, all the plates were placed in an incubator at 37 °C with 5% CO₂, with the shaker set at 600 rpm. The sample was mixed well at 15, 30, 60, and 90 min of incubation, separately. 25 µL of the sample was added to 125 µL of a stop solution (an acetonitrile solution containing 200 ng/mL tolbutamide and 200 ng/mL labetalol) in an ice bath, and the mixture was mixed well and shaken at 500 rpm for 10 min. Subsequently, the plate was centrifuged for 20 min at 3220× g at 4 °C. After the centrifugation was completed, 80 µL of the supernatant from each well was transferred to another 96-well plate containing 240 µL of ultrapure water. Subsequently, analysis was carried out using LC-MS/MS, and intrinsic clearance (CLint) and half-life (T1/2) were calculated.

### Experimental Example 4: Pharmacokinetic Study in Rats

In this experimental example, the pharmacokinetic profile of the compounds after administration by intravenous injection (IV) and oral gavage (PO) in SD rats was evaluated.

On the day of administration, rats were weighed for actual body weight, and the administration volume was calculated. Each compound was tested in two groups with 3 rats in each group; one group was subjected to administration by single intravenous injection, and the other group was subjected to administration by single oral gavage. Whole blood samples were collected via the jugular vein at prescribed time points (0.25 h, 0.5 h, 1 h, 2 h, 4 h, 8 h, and 24 h after administration). After the blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis was performed.

Plasma concentrations were determined by the LC-MS/MS method. Plasma drug concentration data for the compounds were processed using a non-compartmental model in the WinNonlin Version 6.3 (Pharsight, Mountain View, CA) pharmacokinetic software. The relevant pharmacokinetic parameters were calculated by a linear-log trapezoidal method.

### Experimental Example 5: Pharmacokinetic Study in Mice

In this experimental example, the pharmacokinetic profile of the compounds after administration by intravenous injection (IV) and oral gavage (PO) in BALB/c mice was evaluated.

On the day of administration, mice were weighed for actual body weight, and the administration volume was calculated. Each compound was tested in two groups with 9 mice in each group; one group was subjected to administration by single intravenous injection, and the other group of mice was subjected to administration by single oral gavage. Whole blood samples were collected via the orbit at prescribed time points (0.25 h, 0.5 h, 1 h, 2 h, 4 h, 8 h, and 24 h after administration). After the blood sample collection, the samples were immediately transferred to labeled commercial sample tubes containing K2-EDTA (0.85-1.15 mg), followed by centrifugation (3200× g, 4 °C, 10 min) and plasma collection. The plasma was transferred to a pre-cooled centrifuge tube, frozen in dry ice, and then stored in an ultra-low temperature freezer at -60 °C or lower until the LC-MS/MS analysis was performed.

Plasma concentrations were determined by the LC-MS/MS method. Plasma drug concentration data for the compounds were processed using a non-compartmental model in the WinNonlin Version 6.3 (Pharsight, Mountain View, CA) pharmacokinetic software. The relevant pharmacokinetic parameters were calculated by a linear-log trapezoidal method.

### Experimental Example 6: hERG Inhibition Assay

HEK293 cells were cultured in a DMEM medium containing 10% fetal bovine serum and 0.8 mg/mL G418 at 37 °C with 5% CO₂. The cells were digested by TrypLE^{™} Express and then centrifuged. The cell density was adjusted to 2 × 10⁶ cells/mL. The cells were then gently mixed for 15 to 20 min using a shaker equilibrated at room temperature, and subjected to a patch clamp assay on a machine. The medium of the prepared cells was replaced with extracellular fluid. The intracellular fluid and the extracellular fluid were taken from the fluid pool and added to the intracellular fluid pool and the cell and test substance pool of the QPlate chip, respectively. The voltage stimulation of the whole-cell hERG potassium current was recorded by the whole-cell patch clamp, and the experimental data were collected and stored by Qpatch. The compound was subjected to a 3-fold dilution starting from 30 µM to obtain 6 concentration points, each for two administrations over a period of at least 5 min. The current detected in compound-free extracellular fluid for each cell served as its own control group, and the detection was repeated twice independently using at least two cells per concentration. All electrophysiological experiments were performed at room temperature.

For data analysis, the current after the action of each drug concentration and the current of the blank control were standardized ( $\frac{Peak tail {current}_{compound}}{Peak tail {current}_{vehicle}}$), and then the inhibition rate corresponding to each drug concentration was calculated ( $1 - \frac{Peak tail {current}_{compound}}{Peak tail {current}_{vehicle}}$). The mean and standard error were calculated for each concentration, and the half maximal inhibitory concentration of each compound was calculated: $Y = Bottom + \frac{Top - Bottom}{1 + {10}^{∧} \left(\left({LogIC}_{50}-C\right)\times Hillslope\right)}$. The dose-dependent effect was fitted non-linearly using the above equation, where Y represents the inhibition rate, C represents the concentration of the test substance, IC₅₀ is the half maximal inhibitory concentration, and HillSlope represents the Hill coefficient. Curve fitting and calculation of IC₅₀ were performed using Graphpad software.

### Experimental Example 7: Cytochrome Oxidase P450 Inhibition Assay

### 1) Preparation of buffer:

100 mM K-Buffer: 9.5 mL of stock solution A was mixed with 40.5 mL of stock solution B, the total volume was adjusted to 500 mL with ultrapure water, and the buffer was titrated to pH 7.4 with KOH or H₃PO₄. Starting material A (1 M potassium dihydrogen phosphate): 136.5 g of potassium dihydrogen phosphate in 1 L of water;
stock B (1 M potassium dihydrogen phosphate): 174.2 g of potassium dihydrogen phosphate in 1 L of water.

### 2) Preparation of test substance

The test substance powder was prepared into a stock solution at a certain concentration with DMSO or another organic solvent, which was then further diluted with a suitable organic solvent.

### 3) In vitro incubation

The liver microsomal *in vitro* incubation system for the CYP450 enzyme metabolism phenotype study was a biochemical reaction of the prepared liver microsomes supplemented with a redox coenzyme and an enzyme-specific selective inhibitor under simulated physiological temperature and physiological environment conditions.

### 4) Detection of parent drug or metabolite

The concentration of the parent drug or a metabolite thereof in the incubation solution was determined by LC-MS/MS.

### Experimental Example 8: Pharmacodynamic Model of Mouse Tumor

In this experimental example, the *in vivo* efficacy of the compound in a mouse xenograft tumor model after administration by oral gavage (PO) was studied.

Human colonic adenocarcinoma SW48 cells were cultured in monolayer *in vitro* under the following conditions: DMEM medium containing 10% fetal bovine serum and 1% penicillin and streptomycin, at 37 °C with 5% CO₂. The cells were digested with trypsin twice a week for passaging. When the saturation degree of the cells was 80% to 90%, the cells were collected, counted, and inoculated. 0.1 mL (10⁷ cells) of SW48 cells were subcutaneously inoculated on the right back of each mouse. On day 14 after inoculation of the cells, the mean tumor volume reached about 200 mm³. The mice were randomly grouped for administration and intragastrically dosed once daily. Changes in body weight and tumor volume were recorded. After a certain number of days of administration, the experiment was ended. Tumor volume changes and mouse body weight changes were counted and analyzed.

Various modifications of the present disclosure in addition to those described herein will be apparent to those skilled in the art on the basis of the aforementioned description. Such modifications are also intended to fall within the scope of the appended claims. References (including all patents, patent applications, journal articles, books, and any other publications) cited in the present application are all incorporated herein by reference in their entirety.

## Claims

1. A compound, or a pharmaceutically acceptable salt, an ester, a stereoisomer, an atropisomer, a tautomer, a polymorph, a solvate, a metabolite, an isotopically labeled compound, or a prodrug thereof, wherein the compound has a structure of formula (I): 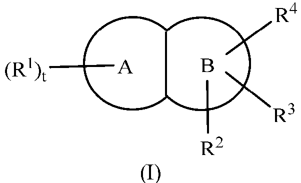 wherein: 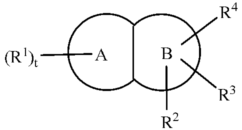 is selected from 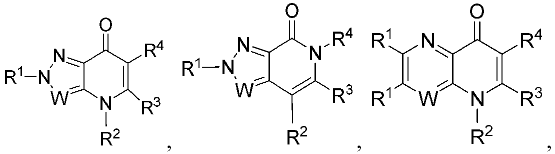 and 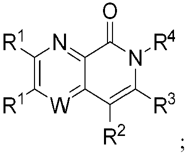
W is C-R¹ or N;
R¹, at each occurrence, is each independently selected from H, halogen, -OH, -NH₂, -CN, -NO₂, -SF₅, C₁₋₆ alkyl, deuterated C₁₋₆ alkyl, C₁₋₆ haloalkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, -C(=O)R^{a}, -OC(=O)R^{a}, - C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a_}C(=O)R^{b}, -NR^{a}-C(=O)OR^{b}, -NR^{a}-S(=O)₂-R^{b}, -NR^{a}-C(=O)-NR^{a}R^{b}, -P(=O)R^{a}R^{b}, -C₁₋₆ alkylene-R^{a}, -C₁₋₆ alkylene-OR^{a}, -C₁₋₆ alkylene-NR^{a}R^{b}, -O-C₁₋₆ alkylene-NR^{a}R^{b}, (-C₃₋₆ cyclohydrocarbylene)-CN, and (-C₃₋₆ cyclohydrocarbylene)-C₁₋₆ haloalkyl;
or, two R¹ at adjacent positions, together with the group to which they are attached, optionally form a C₃₋₆ hydrocarbon ring, a 3- to 10-membered heterocyclic ring, a C₆₋₁₀ aromatic ring, or a 5- to 14-membered heteroaromatic ring;
R² is 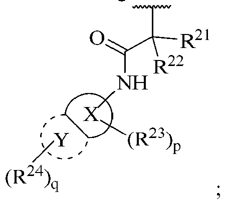
R³, R²¹, R²², R²³, and R²⁴, at each occurrence, are each independently selected from H, halogen, -OH, -NH₂, -CN, -NO₂, -SF₅, C₁₋₆ alkyl, deuterated C₁₋₆ alkyl, C₁₋₆ haloalkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, - C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -S(=O)(-NR^{a})R^{b}, - NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -P(=O)R^{a}R^{b}, -C₁₋₆ alkylene-R^{a}, -C₁₋₆ alkylene-OR^{a}, -C₁₋₆ alkylene-NR^{a}R^{b}, -O-C₁₋₆ alkylene-NR^{a}R^{b}, (-C₃₋₆ cyclohydrocarbylene)-CN, and (-C₃₋₆ cyclohydrocarbylene)-C₁₋₆ haloalkyl;
or, R³ and R²¹ or R²², together with the groups to which they are attached, optionally form a C₃₋₆ hydrocarbon ring, a 3- to 10-membered heterocyclic ring, a C₆₋₁₀ aromatic ring, or a 5- to 14-membered heteroaromatic ring;
R⁴ is 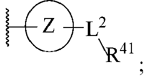 R⁴¹ is selected from a C₃₋₆ hydrocarbon ring, a 3- to 10-membered heterocyclic ring, a C₆₋₁₀ aromatic ring, and a 5- to 14-membered heteroaromatic ring;
ring X and ring Z are each independently selected from a C₃₋₆ hydrocarbon ring, a 3- to 10-membered heterocyclic ring, a C₆₋₁₀ aromatic ring, and a 5- to 14-membered heteroaromatic ring;
ring Y is absent or selected from a C₃₋₆ hydrocarbon ring, a 3- to 10-membered heterocyclic ring, a C₆₋₁₀ aromatic ring, and a 5- to 14-membered heteroaromatic ring; when ring Y is absent, R²⁴ is also absent;
L² is selected from -O-, -C(=O)-, -NRC(=O)-, -S-, -S(=O)-, -S(=O)₂-, C₁₋₆ alkylene, and -O-(C₁₋₆ alkylene)-; R, R^{a}, and R^{b}, at each occurrence, are each independently selected from H, C₁₋₆ alkyl, C₃₋₁₀ cyclohydrocarbyl, 3- to 10-membered heterocyclyl, C₆₋₁₀ aryl, 5- to 14-membered heteroaryl, and C₆₋₁₂ aralkyl;
the alkylene, the alkyl, the alkenyl, the alkynyl, the cyclohydrocarbylene, the cyclohydrocarbyl, the hydrocarbon ring, the heterocyclyl, the heterocyclic ring, the aryl, the aromatic ring, the heteroaryl, the heteroaromatic ring, and the aralkyl described above, at each occurrence, are each optionally substituted with one or more substituents independently selected from the following: a deuterium atom, halogen, -OH, =O, - NH₂, -CN, -NO₂, =CH₂, C₁₋₆ alkyl, deuterated C₁₋₆ alkyl, C₂₋₆ alkenyl, C₂₋₆ alkynyl, C₃₋₆ cyclohydrocarbyl, 3- to 10-membered heterocyclyl, C₆₋₁₀ aryl, 5- to 14-membered heteroaryl, C₆₋₁₂ aralkyl, -C(=O)R^{c}, - OC(=O)R^{c}, -C(=O)OR^{c}, -OR^{c}, -SR^{c}, -S(=O)R^{c}, -S(=O)₂R^{c}, -S(=O)₂NR^{c}R^{d}, -NR^{c}R^{d}, -C(=O)NR^{c}R^{d}, -NR^{c}-C(=O)R^{d}, -NR^{c}-C(=O)OR^{d}, -NR^{c}-S(=O)₂-R^{d}, -NR^{c}-C(=O)-NR^{c}R^{d}, -C₁₋₆ alkylene-OR^{c}, -C₁₋₆ alkylene-NR^{c}R^{d}, and -O-C₁₋₆ alkylene-NR^{c}R^{d}, and when a same ring atom or adjacent ring atoms of the cyclohydrocarbylene, the cyclohydrocarbyl, the hydrocarbon ring, the heterocyclyl, the heterocyclic ring, the aryl, the aromatic ring, the heteroaryl, and the heteroaromatic ring are substituted with two substituents, the two substituents, together with groups to which they are attached, optionally form a C₃₋₆ hydrocarbon ring, a 3- to 10-membered heterocyclic ring, a C₆₋₁₀ aromatic ring, or a 5- to 14-membered heteroaromatic ring; the alkylene, the alkyl, the alkenyl, =CH₂, the alkynyl, the cyclohydrocarbyl, the hydrocarbon ring, the heterocyclyl, the heterocyclic ring, the aryl, the aromatic ring, the heteroaryl, the heteroaromatic ring, and the aralkyl are each further optionally substituted with one or more substituents independently selected from the following: halogen, -OH, =O, -C(=O)O-tert-butyl, -NH₂, -CN, -NO₂, C₁₋₆ alkyl, C₁₋₆ haloalkyl, C₃₋₆ cyclohydrocarbyl, 3- to 10-membered heterocyclyl, C₆₋₁₀ aryl, 5- to 14-membered heteroaryl, C₆₋₁₂ aralkyl, -C₁₋₆ alkylene-C₃₋₆ cyclohydrocarbyl, -O-C₁₋₆ alkyl, and -C₁₋₆ alkylene-O-C₁₋₆ alkyl;
R^{c} and R^{d}, at each occurrence, are each independently selected from: H, C₁₋₆ alkyl, C₃₋₁₀ cyclohydrocarbyl, 3- to 10-membered heterocyclyl, C₆₋₁₀ aryl, 5- to 14-membered heteroaryl, and C₆₋₁₂ aralkyl, wherein the alkyl, the cyclohydrocarbyl, the heterocyclyl, the aryl, the heteroaryl, and the aralkyl are further optionally substituted with one or more substituents independently selected from the following: halogen, -OH, =O, - C(=O)O-tert-butyl, -NH₂, -CN, -NO₂, C₁₋₆ alkyl, C₁₋₆ haloalkyl, C₃₋₆ cyclohydrocarbyl, 3- to 10-membered heterocyclyl, C₆₋₁₀ aryl, 5- to 14-membered heteroaryl, C₆₋₁₂ aralkyl, and -C₁₋₆ alkylene-O-C₁₋₆ alkyl; and
p, q, and t are each independently an integer selected from 1, 2, or 3.

2. The compound, or the pharmaceutically acceptable salt, the ester, the stereoisomer, the atropisomer, the tautomer, the polymorph, the solvate, the metabolite, the isotopically labeled compound, or the prodrug thereof according to claim 1, wherein the compound has structures of the following formulas: 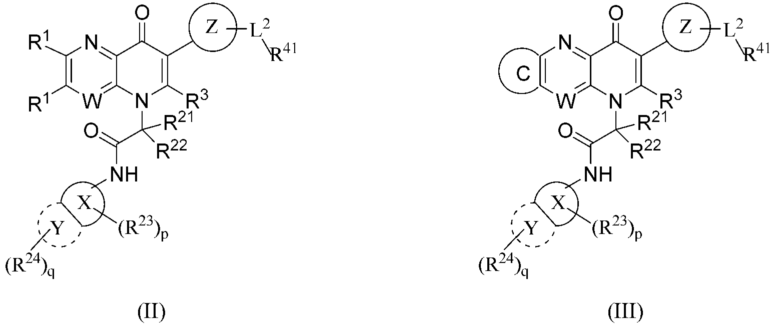 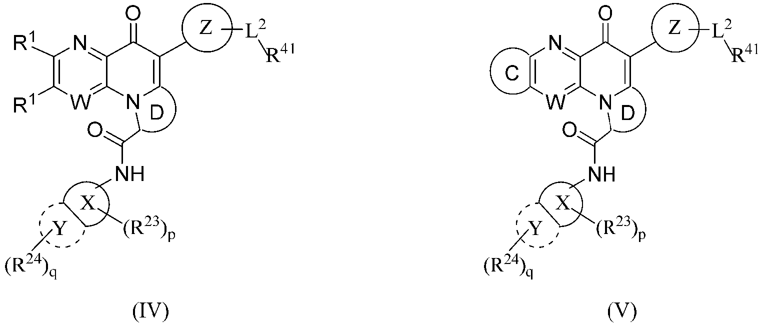 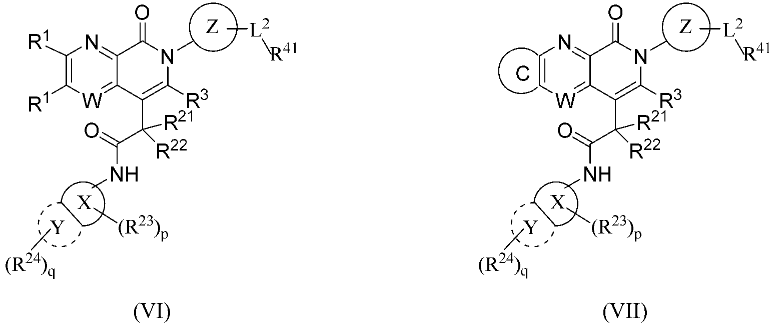 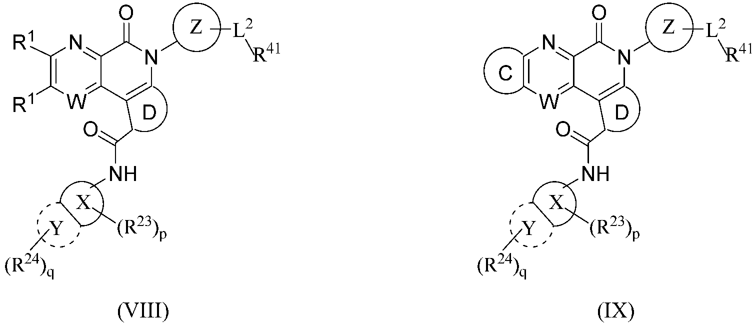 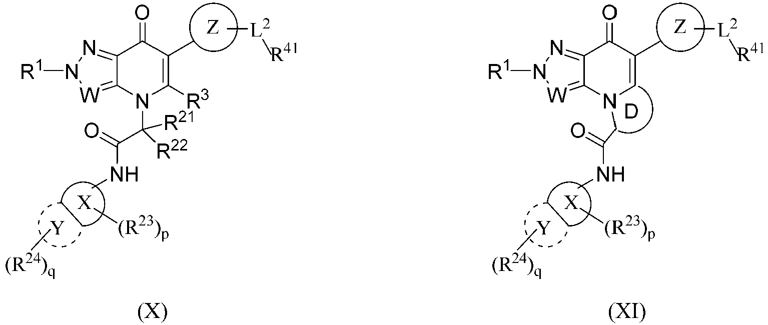 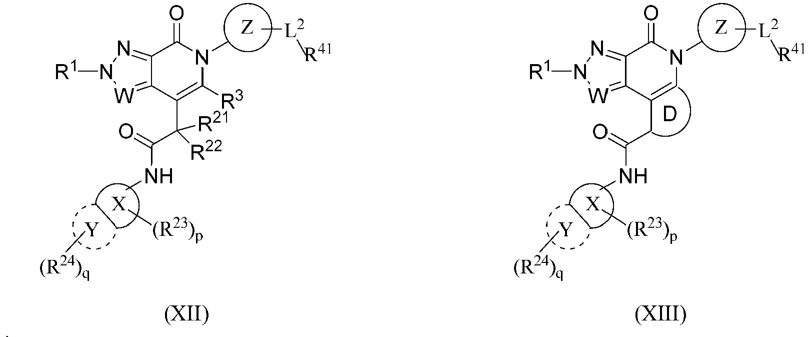 wherein:
ring C and ring D are each independently a C₃₋₆ hydrocarbon ring, a 3- to 10-membered heterocyclic ring, a C₆₋₁₀ aromatic ring, or a 5- to 14-membered heteroaromatic ring, wherein the hydrocarbon ring, the heterocyclic ring, the aromatic ring, and the heteroaromatic ring are each optionally substituted with one or more substituents independently selected from the following: a deuterium atom, halogen, -OH, C₁₋₆ alkyl, C₁₋₆ haloalkyl, and -O-C₁₋₆ alkyl, and when a same ring atom or adjacent ring atoms of the hydrocarbon ring, the heterocyclic ring, the aromatic ring, and the heteroaromatic ring are substituted with two substituents, the two substituents, together with groups to which they are attached, optionally form a C₃₋₆ hydrocarbon ring, a 3- to 10-membered heterocyclic ring, a C₆₋₁₀ aromatic ring, or a 5- to 14-membered heteroaromatic ring;
preferably, ring C is 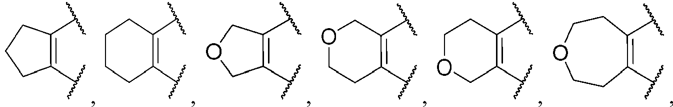 a benzene ring, or a pyridine ring, each being optionally substituted with one or more substituents independently selected from the following: halogen, C₁₋₆ alkyl, and C₁₋₆ haloalkyl; and/or
ring D is a cyclopentene ring, a cyclohexene ring, a pyrrolidine ring, a piperidine ring, or a morpholine ring, each being optionally substituted with one or more substituents independently selected from the following: a deuterium atom, halogen, -OH, C₁₋₆ alkyl, C₁₋₆ haloalkyl, and -O-C₁₋₆ alkyl; when a same ring atom or adjacent ring atoms of ring D are substituted with two substituents, the two substituents, together with groups to which they are attached, optionally form a C₃₋₆ hydrocarbon ring or a 3- to 10-membered heterocyclic ring; the other groups are as defined in claim 1.

3. The compound, or the pharmaceutically acceptable salt, the ester, the stereoisomer, the atropisomer, the tautomer, the polymorph, the solvate, the metabolite, the isotopically labeled compound, or the prodrug thereof according to claim 1 or 2, wherein:
R¹, at each occurrence, is each independently selected from H, halogen, C₁₋₆ alkyl, C₃₋₆ cyclohydrocarbyl, 3-to 10-membered heterocyclyl, C₆₋₁₀ aryl, 5- to 14-membered heteroaryl, -OR^{a}, and -NR^{a}R^{b}, and preferably, R¹, at each occurrence, is each independently selected from H, halogen, C₁₋₆ alkyl, C₃₋₆ cyclohydrocarbyl, and -OR^{a}, wherein the alkyl, the cyclohydrocarbyl, the heterocyclyl, the aryl, and the heteroaryl are each optionally substituted with one or more substituents independently selected from the following: halogen, C₁₋₆ alkyl, C₂₋₆ alkenyl, =CH₂, C₃₋₆ cyclohydrocarbyl, 3- to 10-membered heterocyclyl, C₆₋₁₀ aryl, and 5- to 14-membered heteroaryl; the alkyl, the alkenyl, =CH₂, the cyclohydrocarbyl, the heterocyclyl, the aryl, and the heteroaryl are each further optionally substituted with one or more substituents independently selected from the following: halogen, C₁₋₆ alkyl, C₃₋₆ cyclohydrocarbyl, 3- to 10-membered heterocyclyl, and -C₁₋₆ alkylene-C₃₋₆ cyclohydrocarbyl;
preferably, R¹ is H, methyl, halogen, methoxy, 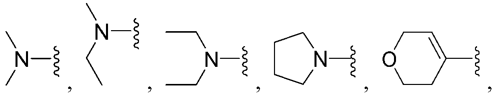 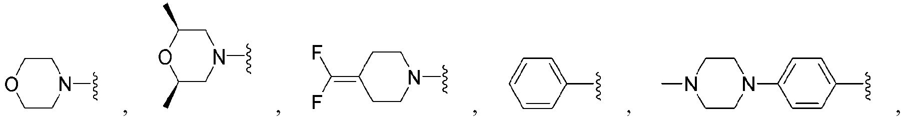 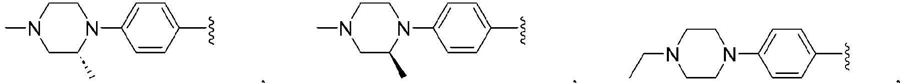 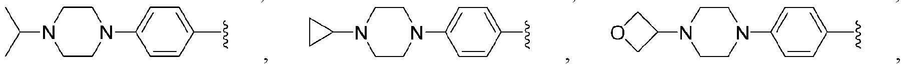 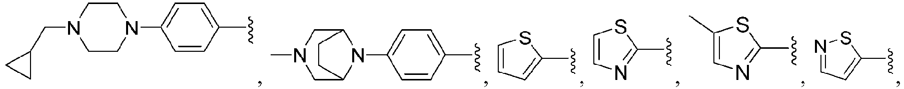 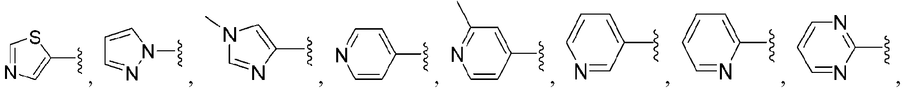 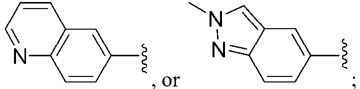
or, two R¹ at adjacent positions, together with the group to which they are attached, optionally form a C₃₋₆ hydrocarbon ring, a 3- to 10-membered heterocyclic ring, a C₆₋₁₀ aromatic ring, or a 5- to 14-membered heteroaromatic ring, and the hydrocarbon ring, the heterocyclic ring, the aromatic ring, and the heteroaromatic ring are each optionally substituted with one or more substituents independently selected from the following: halogen, C₁₋₆ alkyl, and C₁₋₆ haloalkyl;
preferably, two R¹ at adjacent positions, together with the group to which they are attached, optionally form 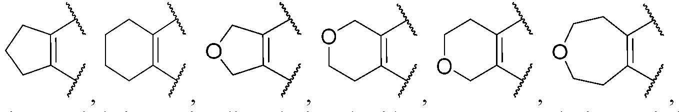 a benzene ring, or a pyridine ring, each being optionally substituted with one or more substituents independently selected from the following: halogen, C₁₋₆ alkyl, and C₁₋₆ haloalkyl.

4. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 3, wherein
ring X is a benzene ring, a 5- to 6-membered heterocyclic ring, or a 5- to 6-membered heteroaromatic ring (preferably a thiophene ring, a thiazole ring, or a pyridine ring), and ring Y is absent; or
ring X is a benzene ring or a 5- to 6-membered heteroaromatic ring, and ring Y is a C₃₋₆ hydrocarbon ring, a 5- to 6-membered heterocyclic ring, or a 5- to 6-membered heteroaromatic ring;
preferably, 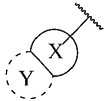 is 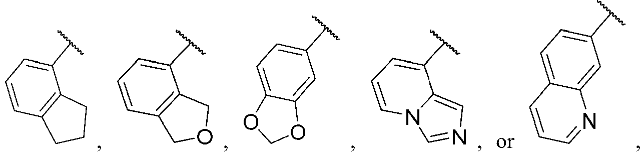 more preferably

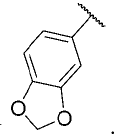

5. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 4, wherein R²¹, R²², R²³, and R²⁴, at each occurrence, are each independently selected from H, halogen, -SF₅, C₁₋₆ alkyl, C₃₋₆ cyclohydrocarbyl, 3- to 10-membered heterocyclyl, -O-(C₁₋₆ alkyl), -S(=O)₂-(C₁₋₆ alkyl), -S(=O)₂-(C₃₋₆ cyclohydrocarbyl), -P(=O)(C₁₋₆ alkyl)₂, (-C₃₋₆ cyclohydrocarbylene)-CN, and (-C₃₋₆ cyclohydrocarbylene)-C₁₋₆ alkyl, and the alkyl, the cyclohydrocarbylene, the cyclohydrocarbyl, and the heterocyclyl are each optionally substituted with one or more substituents independently selected from the following: halogen, C₁₋₆ alkyl, and C₁₋₆ haloalkyl;
preferably, R²¹, R²², R²³, and R²⁴, at each occurrence, are each independently selected from H, halogen, -SF₅, C₁₋₆ alkyl, C₃₋₆ cyclohydrocarbyl, 3- to 10-membered heterocyclyl, and -O-(C₁₋₆ alkyl), and the alkyl, the cyclohydrocarbyl, and the heterocyclyl are each optionally substituted with one or more substituents independently selected from the following: halogen, C₁₋₆ alkyl, and C₁₋₆ haloalkyl.

6. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 5, wherein 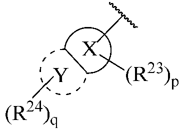 is selected from:

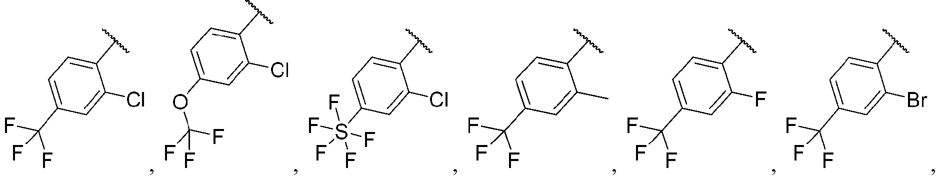

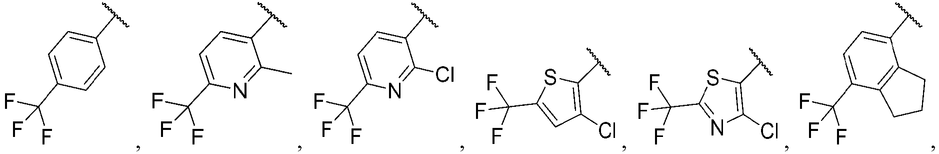

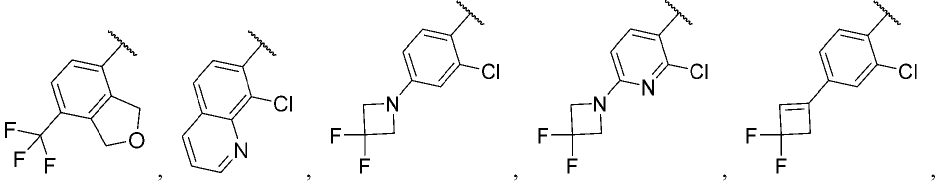

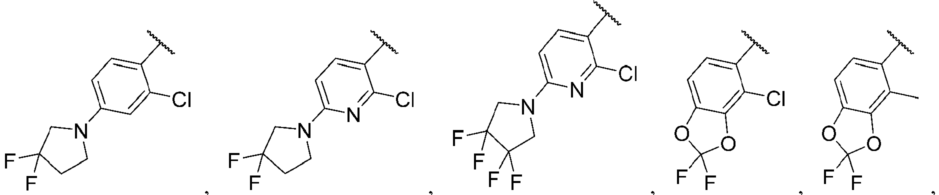

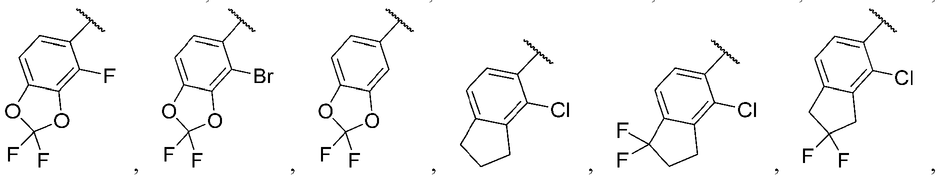

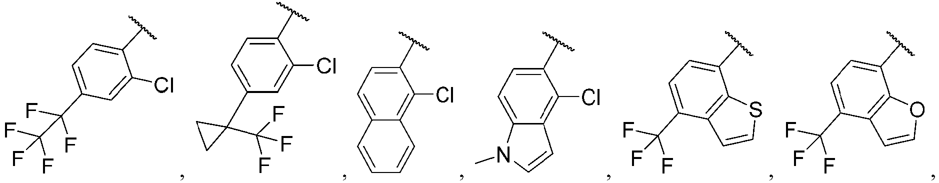

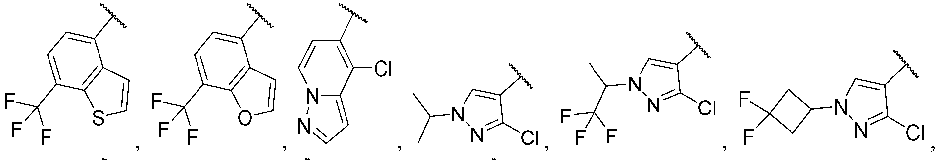

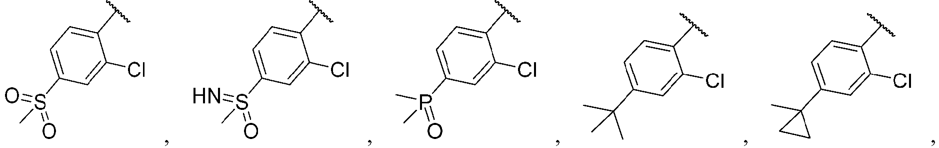

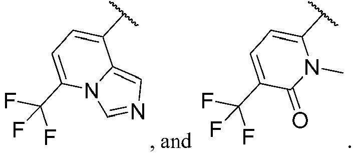

7. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 6, wherein R³ is H, C₁₋₆ alkyl, -OR^{a}, or -SR^{a}; preferably, R³ is H, ethyl, -O-CH₃, or -S-CH₃;
or, R³ and R²¹ or R²², together with the groups to which they are attached, optionally form a C₃₋₆ hydrocarbon ring or a 3- to 10-membered heterocyclic ring, wherein the hydrocarbon ring and the heterocyclic ring are optionally substituted with one or more substituents selected from a deuterium atom, halogen, -OH, C₁₋₆ alkyl, C₁₋₆ haloalkyl, and -O-C₁₋₆ alkyl, and when a same ring atom or adjacent ring atoms of the hydrocarbon ring and the heterocyclic ring are substituted with two substituents, the two substituents, together with groups to which they are attached, optionally form a C₃₋₆ hydrocarbon ring or a 3- to 10-membered heterocyclic ring; preferably, R³ and R²¹ or R²², together with the groups to which they are attached, optionally form a cyclopentene ring, a cyclohexene ring, a pyrrolidine ring, a piperidine ring, or a morpholine ring, wherein the cyclopentene ring, the cyclohexene ring, the pyrrolidine ring, the piperidine ring, and the morpholine ring are optionally substituted with one or more substituents selected from a deuterium atom, halogen, -OH, C₁₋₆ alkyl, C₁₋₆ haloalkyl, and -O-C₁₋₆ alkyl, and when a same ring atom of the cyclopentene ring, the cyclohexene ring, the pyrrolidine ring, the piperidine ring, and the morpholine ring is substituted with two substituents, the two substituents, together with the groups to which they are attached, optionally form a C₃₋₆ hydrocarbon ring.

8. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 7, wherein ring Z is a 3- to 10-membered heterocyclic ring or a benzene ring, preferably a 5- to 10-membered heterocyclic ring, and more preferably a 5- to 6-membered heterocyclic ring; and
the heterocyclic ring and the benzene ring, at each occurrence, are each optionally substituted with one or more substituents independently selected from the following: halogen, C₁₋₆ alkyl, and C₁₋₆ haloalkyl;
preferably, ring Z is

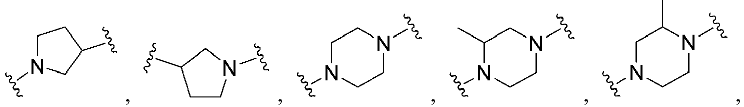

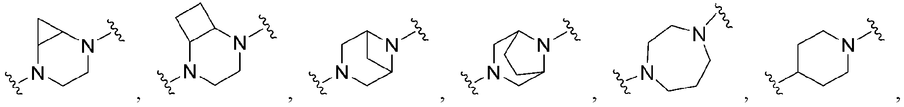

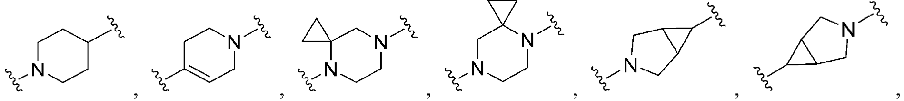

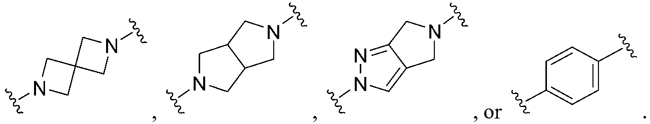

9. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 8, wherein L² is -C(=O)- or -NRC(=O)-, R being H or C₁₋₆ alkyl; preferably, L² is -C(=O)-.

10. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 9, wherein R⁴¹ is selected from a 3- to 10-membered heterocyclic ring, a C₆₋₁₀ aromatic ring, and a 5- to 14-membered heteroaromatic ring, and the heterocyclic ring, the aromatic ring, and the heteroaromatic ring are each optionally substituted with one or more substituents independently selected from the following: halogen, -OH, C₁₋₆ alkyl, -O-C₁₋₆ alkyl, and -S-C₁₋₆ alkyl, preferably, the heterocyclic ring, the aromatic ring, and the heteroaromatic ring at least being substituted with -OH or -O-C₁₋₆ alkyl;
preferably, -L²-R⁴¹ is

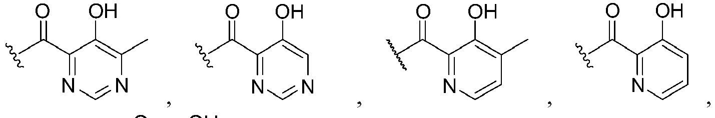

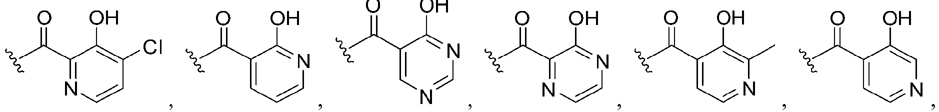

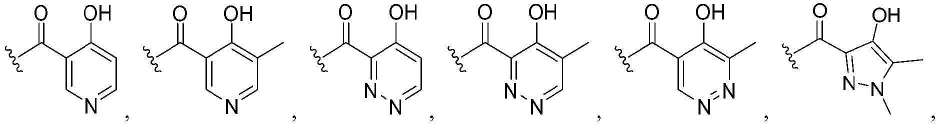

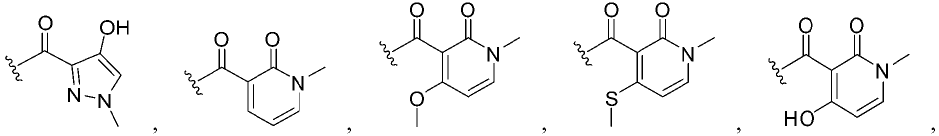

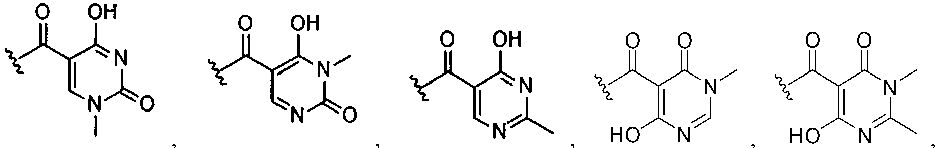

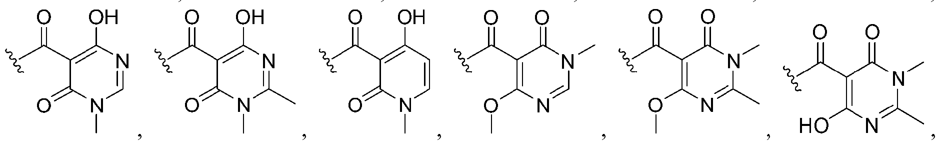

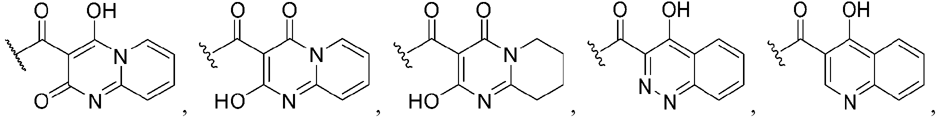

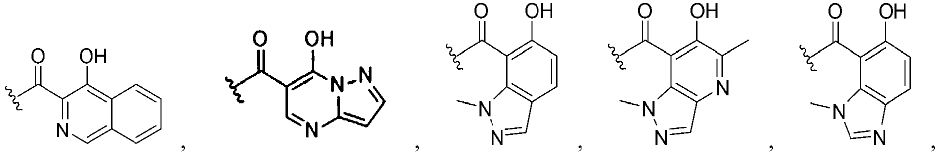

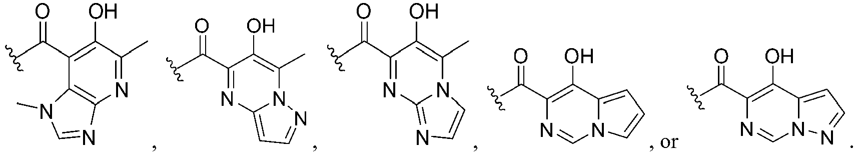

11. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 10, wherein R⁴¹ is a 5- to 6-membered heteroaromatic ring, preferably a 6-membered heteroaromatic ring, and more preferably a pyridine ring or a pyrimidine ring, being at least substituted with one -OH;
preferably, -L²-R⁴¹ is

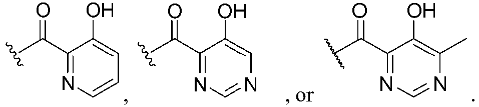

12. The compound, or the pharmaceutically acceptable salt, the ester, the stereoisomer, the atropisomer, the tautomer, the polymorph, the solvate, the metabolite, the isotopically labeled compound, or the prodrug thereof according to any one of claims 1 to 11, wherein the compound is selected from:
| **Compound No.** | **Structure** | **Compound No.** | **Structure** |
|---|---|---|---|
| **B1** | | **B2** | |
| **B3** | | **B4** | |
| **B5** | 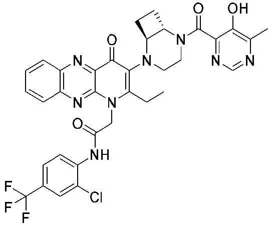 | **B6** | 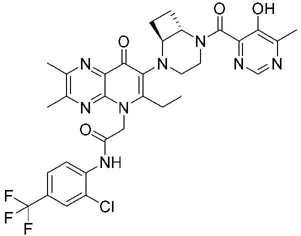 |
| **B7** | 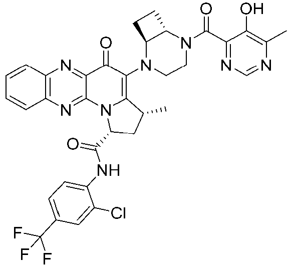 | **B8** | 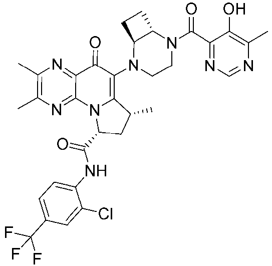 |
| **B9** | 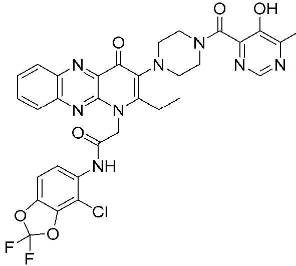 | **B10** | 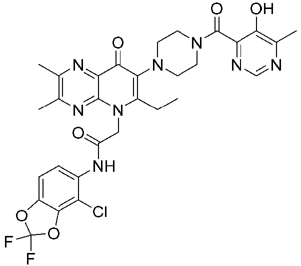 |
| **B11** | 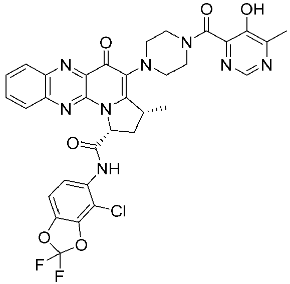 | **B12** | 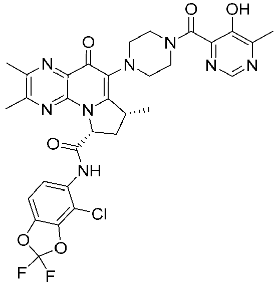 |
| **B17** | 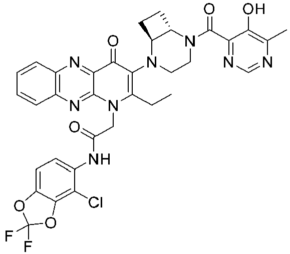 | **B18** | 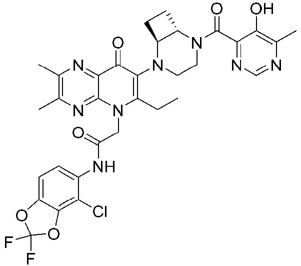 |
| **B19** | 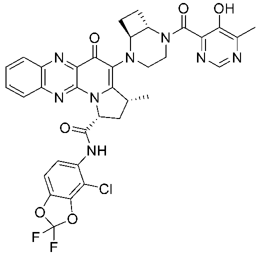 | **B20** | 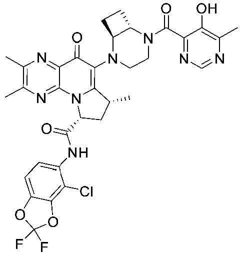 |
| **B21** | 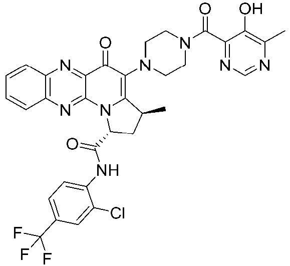 | **B22** | 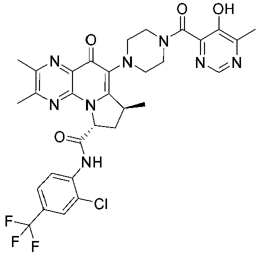 |
| **B23** | 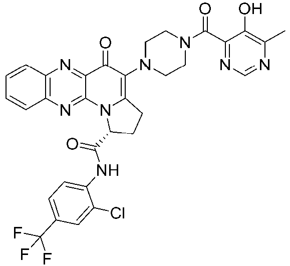 | **B24** | 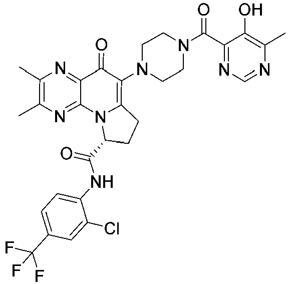 |
| **B25** | 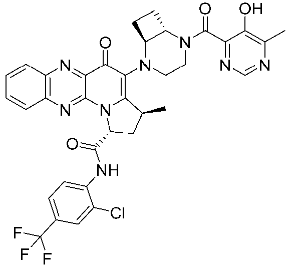 | **B26** | 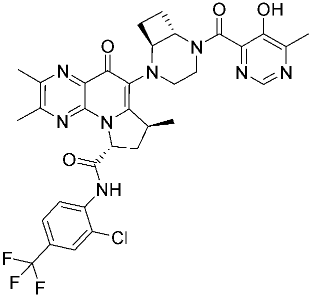 |
| **B27** | 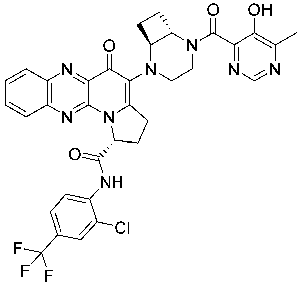 | **B28** | 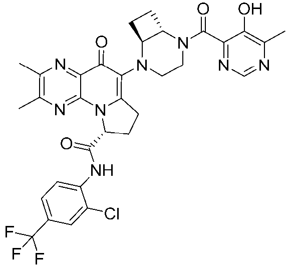 |
| **B29** | 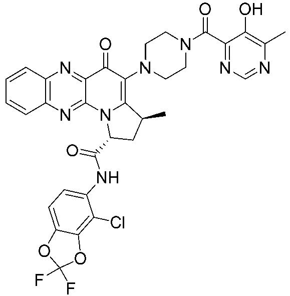 | **B30** | 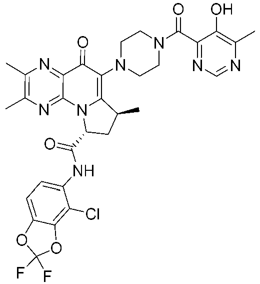 |
| **B31** | 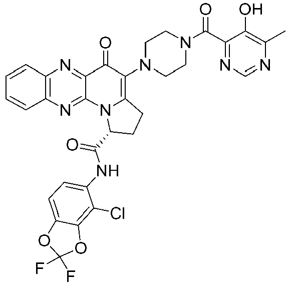 | **B32** | 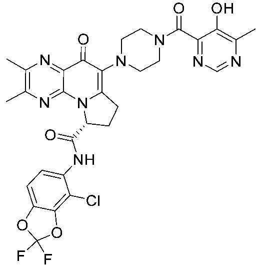 |
| **B33** | 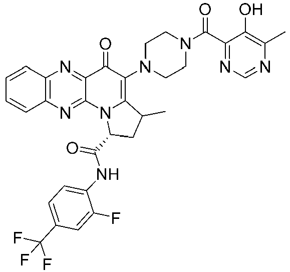 | **B34** | 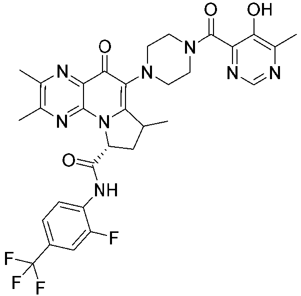 |
| **B35** | 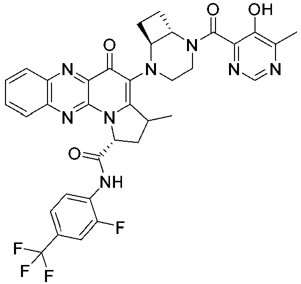 | **B36** | 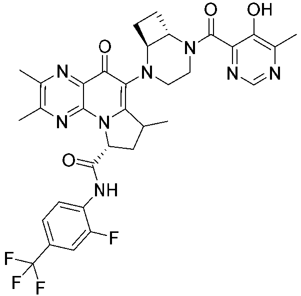 |
| **B37** | 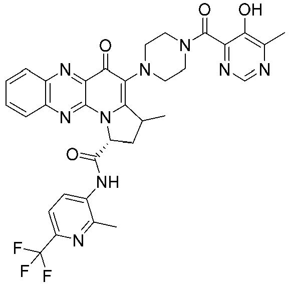 | **B38** | 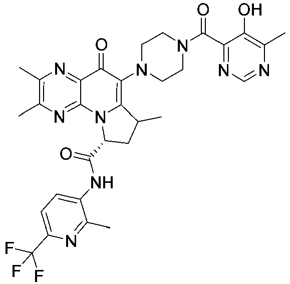 |
| **B39** | 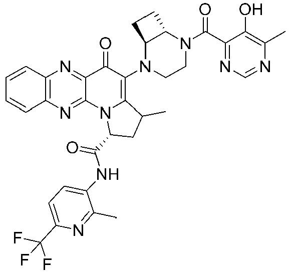 | **B40** | 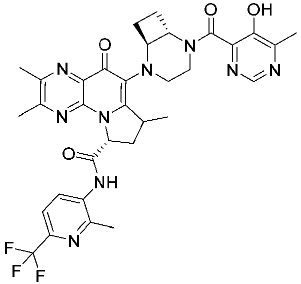 |
| **B41** | 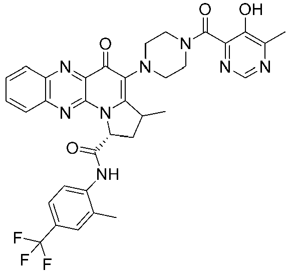 | **B42** | 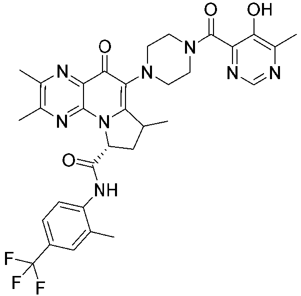 |
| **B43** | 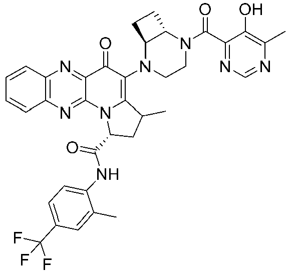 | **B44** | 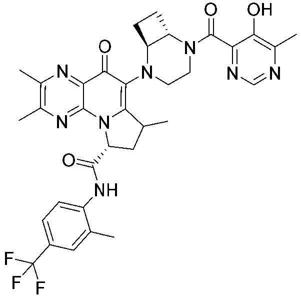 |
| **B45** | 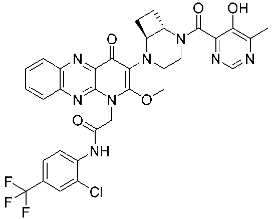 | **B46** | 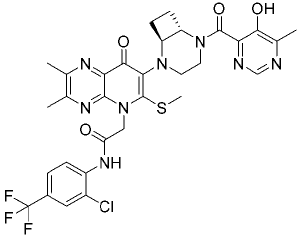 |
| **B47** | 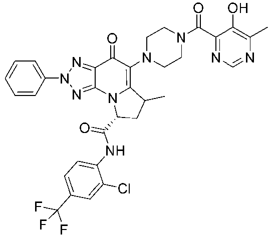 | **B48** | 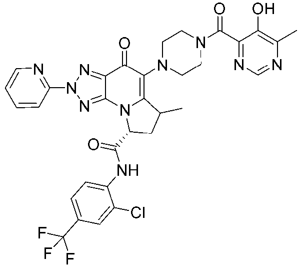 |
| **B49** | 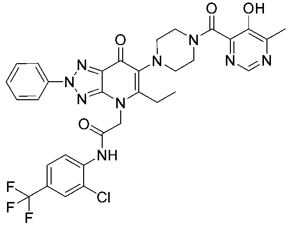 | **B50** | 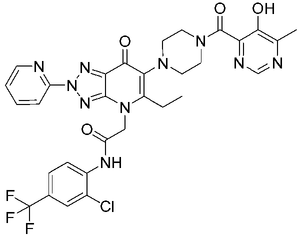 |
| **B51** | 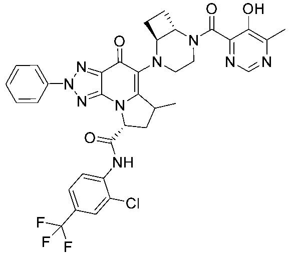 | **B52** | 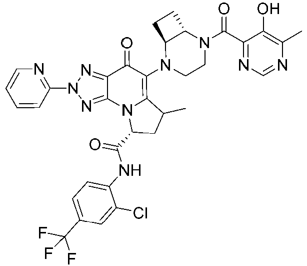 |
| **B53** | 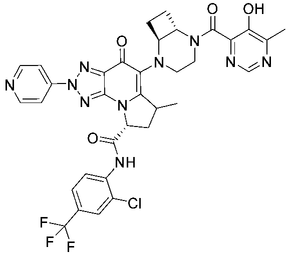 | **B54** | 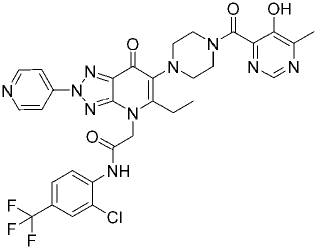 |
| **B55** | 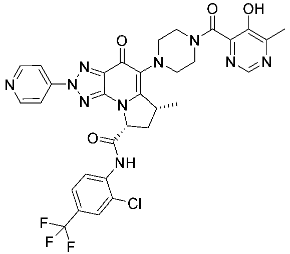 | **B56** | 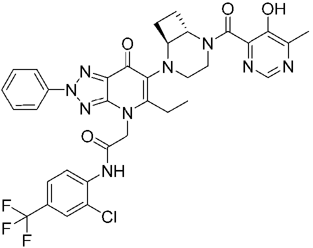 |
| **B57** | 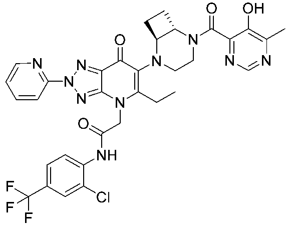 | **B58** | 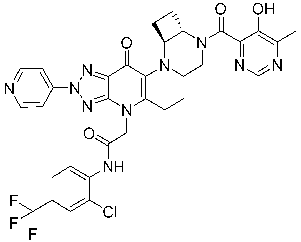 |
| **B59** | 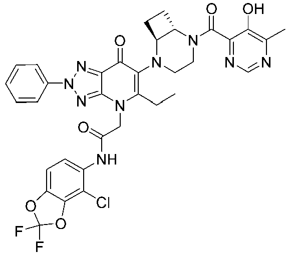 | **B60** | 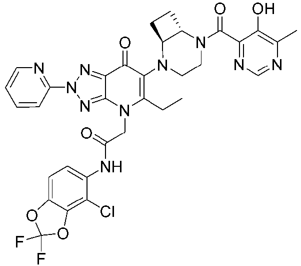 |
| **B61** | 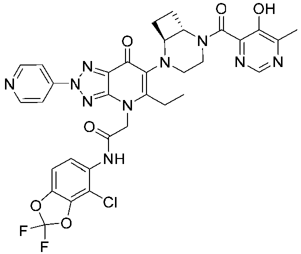 | **B62** | 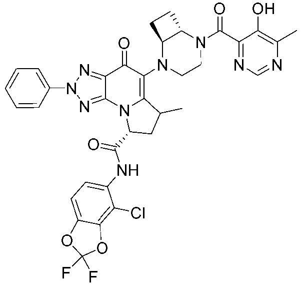 |
| **B63** | 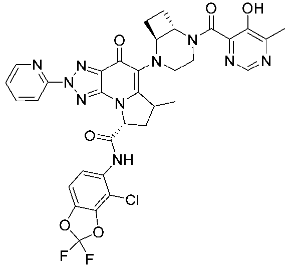 | **B64** | 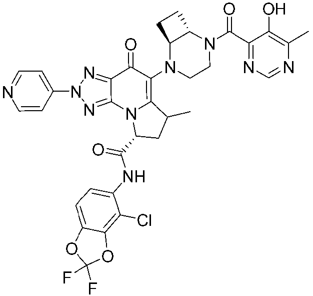 |
| **B65** | 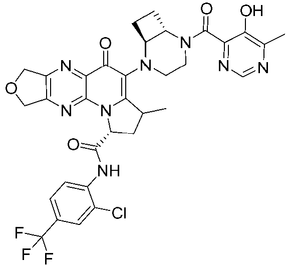 | **B66** | 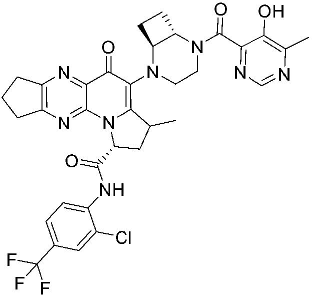 |
| **B67** | 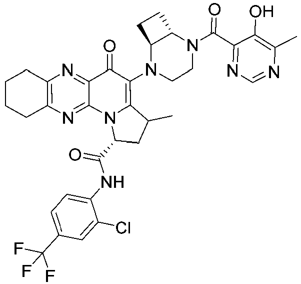 | **B68** | 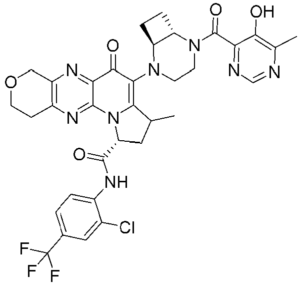 |
| **B69** | 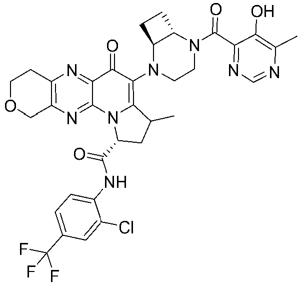 | **B70** | 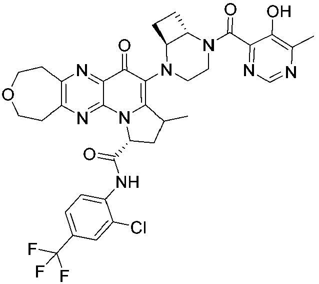 |
| **B71** | 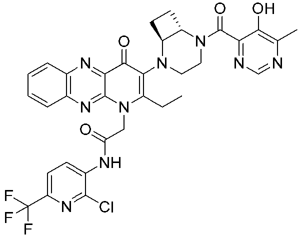 | **B72** | 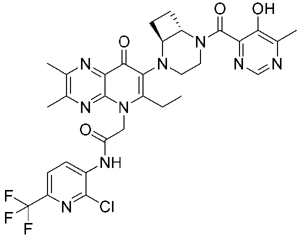 |
| **B73** | 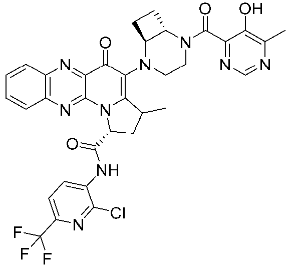 | **B74** | 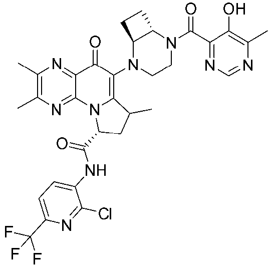 |
| **B75** | 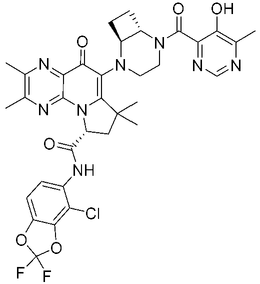 | **B76** | 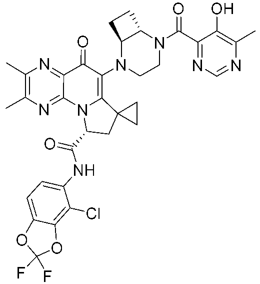 |
| **B77** | 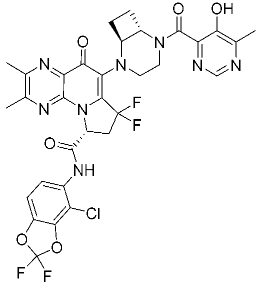 | **B78** | 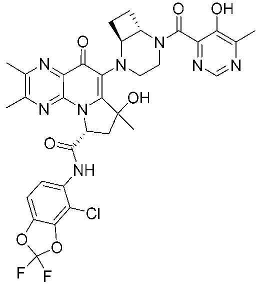 |
| **B79** | 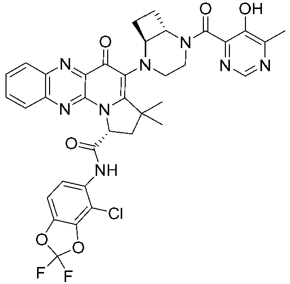 | **B80** | 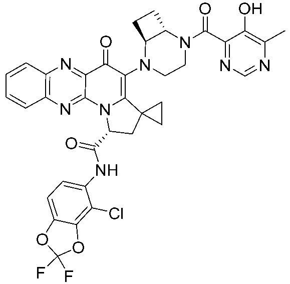 |
| **B81** | 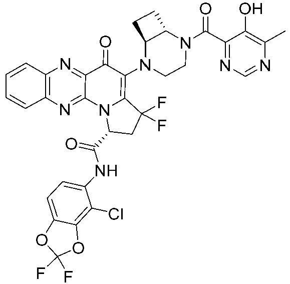 | **B82** | 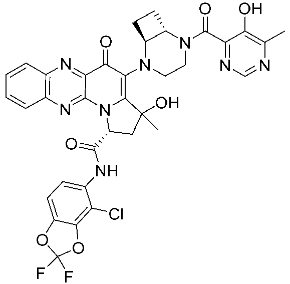 |
| **B83** | 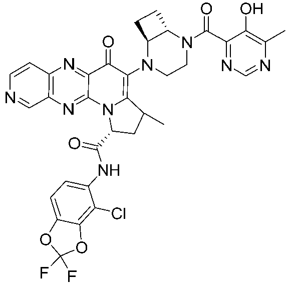 | **B84** | 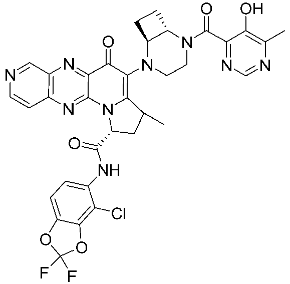 |
| **B85** | 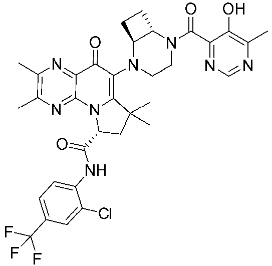 | **B86** | 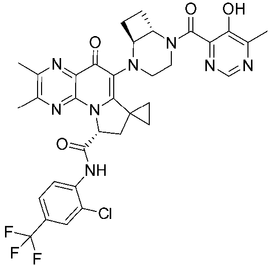 |
| **B87** | 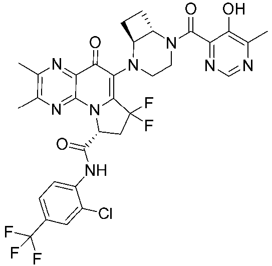 | **B88** | 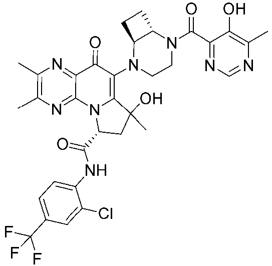 |
| **B89** | 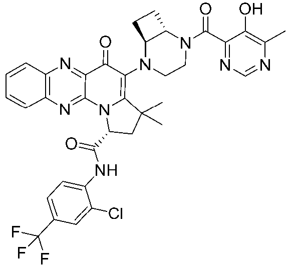 | **B90** | 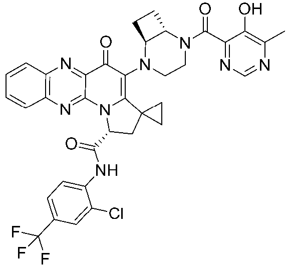 |
| **B91** | 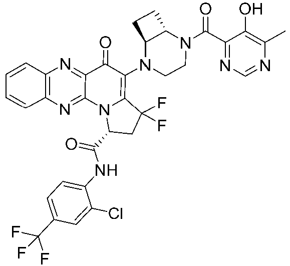 | **B92** | 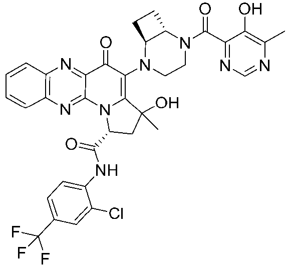 |
| **B93** | 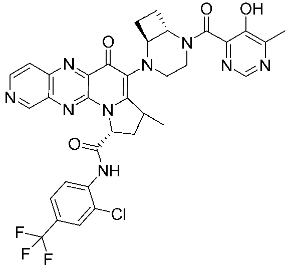 | **B94** | 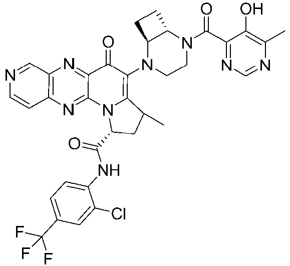 |
| **B95** | 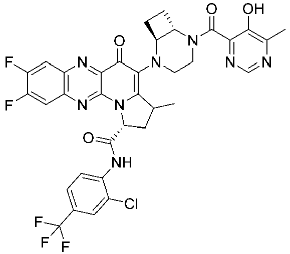 | **B96** | 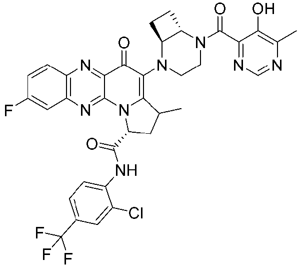 |
| **B97** | 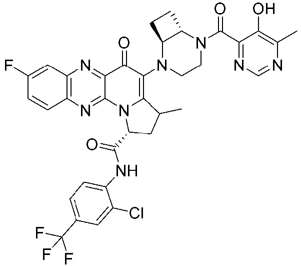 | **B98** | 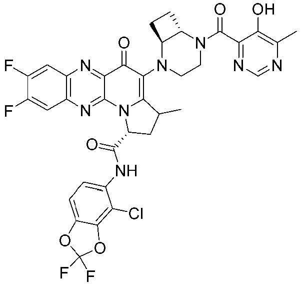 |
| **B99** | 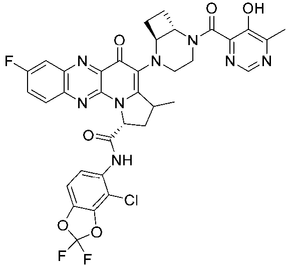 | **B100** | 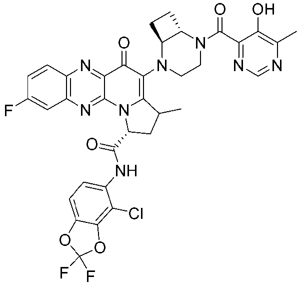 |
| **B101** | 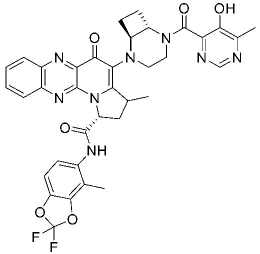 | **B102** | 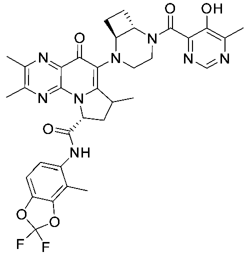 |
| **B103** | 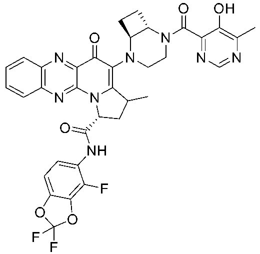 | **B104** | 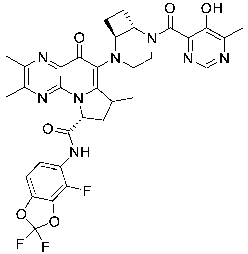 |
| **B105** | 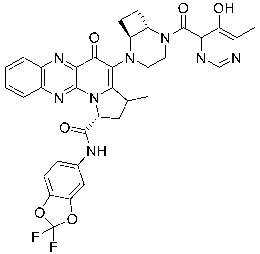 | **B106** | 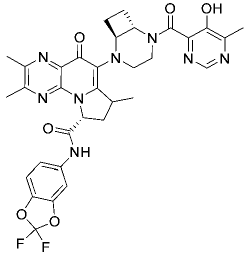 |
| **B107** | 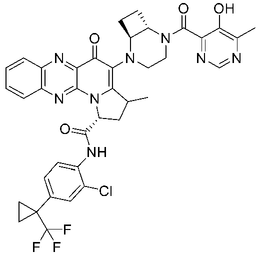 | **B108** | 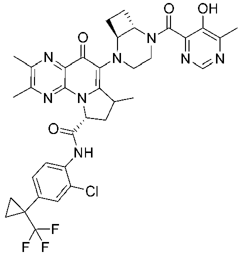 |
| **B109** | 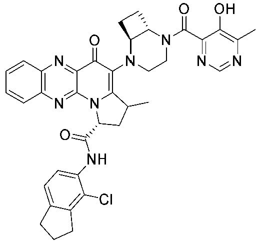 | **B110** | 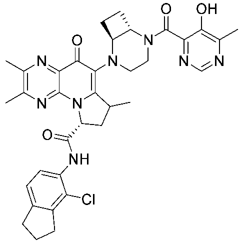 |
| **B111** | 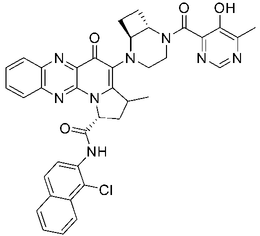 | **B112** | 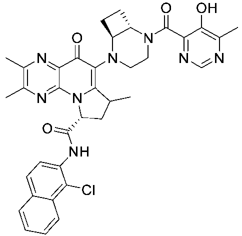 |
| **B113** | 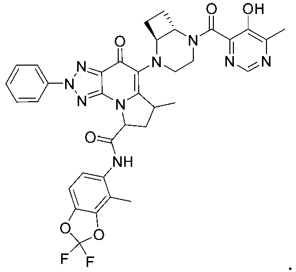 | | |

13. A pharmaceutical composition, comprising a prophylactically or therapeutically effective amount of the compound, or the pharmaceutically acceptable salt, the ester, the stereoisomer, the tautomer, the polymorph, the solvate, the metabolite, the isotopically labeled compound, or the prodrug thereof according to any one of claims 1 to 12, and a pharmaceutically acceptable carrier.

14. Use of the compound, or the pharmaceutically acceptable salt, the ester, the stereoisomer, the tautomer, the polymorph, the solvate, the metabolite, the isotopically labeled compound, or the prodrug thereof according to any one of claims 1 to 12, or the pharmaceutical composition according to claim 13 in preparing a medicament as a WRN inhibitor, wherein preferably, the medicament is used for preventing or treating a cancer (preferably, the cancer is **characterized by** microsatellite instability-high (MSI-H) or mismatch repair deficiency (dMMR)); preferably, the cancer is selected from colorectal cancer, gastric cancer, endometrial cancer, uterine cancer, adrenocortical carcinoma, cervical cancer, esophageal cancer, breast cancer, renal cancer, prostate cancer, and ovarian cancer.
